# EUROPEAN PATENT SPECIFICATION

(11) **EP 2 216 033 B1**
(45) Date of publication and mention of the grant of the patent: **06.11.2013**
(21) Application number: 10154555.6
(22) Date of filing: 04.03.2004
(51) Int. Cl.: A61K 35/12, A61K 35/39, A61K 35/407

(54) **Methods of treating disease by transplantation of allogeneic or xenogeneic organs or tissues**
Behandlungsmethode einer Erkrankung durch Transplantation eines allogenen oder xenogenen Organs oder Gewebe
Méthode de traitement d'une maladie par transplantation d'organes ou de tissus allogenes or xenogenes

(30) Priority: 06.03.2003 US 379725; 20.01.2004 US 759033
(43) Date of publication of application: 11.08.2010
(62) Divisional of application: 04717207.7
(73) Proprietor: Yeda Research and Development Co. Ltd., 76100 Rehovot (IL)
(72) Inventor: Reisner, Yair, 68 037, Tel Aviv (IL); Dekel, Benjamin, 62599, Tel Aviv (IL)
(74) Representative: Dennemeyer & Associates S.A.

(56) References cited:
- WO-A1-96/37602
- US-A1- 2003 032 184
- YOON K-H ET AL: "DIFFERENTIATION AND EXPANSION OF BETA CELL MASS IN PORCINE NEONATAL PANCREATIC CELL CLUSTERS TRANSPLANTED INTO NUDE MICE" CELL TRANSPLANTATION, ELSEVIER SCIENCE, US, vol. 8, no. 6, 1 November 1999 (1999-11-01), pages 673-689, XP008005474 ISSN: 0963-6897
- OTONKOSKI T ET AL: "DIFFERENTIATION AND MATURATION OF PORCINE FETAL ISLET CELLS IN VITRO AND AFTER TRANSPLANTATION" TRANSPLANTATION, WILLIAMS AND WILKINS, BALTIMORE US LNKD- DOI:10.1097/00007890-199912150-00010, vol. 68, no. 11, 15 December 1999 (1999-12-15), pages 1674-1683, XP009007988 ISSN: 0041-1337
- CASTAING M ET AL: "Blood glucose normalization upon transplantation of human embryonic pancreas into beta-cell-deficient SCID mice" DIABETOLOGIA, SPRINGER, BERLIN, DE LNKD- DOI:10.1007/S001250100012, vol. 44, no. 11, 1 November 2001 (2001-11-01), pages 2066-2076, XP002235309 ISSN: 0012-186X
- LENSCHOW D J ET AL: "LONG-TERM SURVIVAL OF XENOGENEIC PANCREATIC ISLET GRAFTS INDUCED BY CTLA4LG" SCIENCE, AMERICAN ASSOCIATION FOR THE ADVANCEMENT OF SCIENCE, WASHINGTON, DC; US LNKD- DOI:10.1126/SCIENCE.1323143, vol. 257, no. 5071, 7 August 1992 (1992-08-07), pages 789-792, XP002021689 ISSN: 0036-8075
- STEURER W ET AL: "EX VIVO COATING OF ISLET CELL ALLOGRAFTS WITH MURINE CTLA4/FC PROMOTES GRAFT TOLERANCE" JOURNAL OF IMMUNOLOGY, AMERICAN ASSOCIATION OF IMMUNOLOGISTS, US, vol. 155, no. 3, 1 August 1995 (1995-08-01), pages 1165-1174, XP002031135 ISSN: 0022-1767
- HORI YUICHI ET AL: "Growth inhibitors promote differentiation of insulin-producing tissue from embryonic stem cells" PROCEEDINGS OF THE NATIONAL ACADEMY OF SCIENCES OF THE UNITED STATES (PNAS), NATIONAL ACADEMY OF SCIENCE, US LNKD- DOI:10.1073/PNAS.252618999, vol. 99, no. 25, 10 December 2002 (2002-12-10), pages 16105-16110, XP002392968 ISSN: 0027-8424
- EVENTOV-FRIEDMAN S ET AL: "Embryonic pig pancreatic tissue transplantation for the treatment of diabetes" PLOS MEDICINE, PUBLIC LIBRARY OF SCIENCE, US LNKD- DOI:10.1371/JOURNAL.PMED.0030215, vol. 3, no. 7, 1 July 2006 (2006-07-01), pages 1165-1177, XP002544044 ISSN: 1549-1676
- HECHT GIL ET AL: "Embryonic pig pancreatic tissue for the treatment of diabetes in a nonhuman primate model" PROCEEDINGS OF THE NATIONAL ACADEMY OF SCIENCES OF THE UNITED STATES (PNAS), NATIONAL ACADEMY OF SCIENCE, US LNKD- DOI:10.1073/PNAS.0812253106, vol. 106, no. 21, 26 May 2009 (2009-05-26), pages 8659-8664, XP009122169 ISSN: 0027-8424
- EVENTOV-FRIEDMAN SMADAR ET AL: "Embryonic pig liver, pancreas, and lung as a source for transplantation: Optimal organogenesis without teratoma depends on distinct time windows" PROCEEDINGS OF THE NATIONAL ACADEMY OF SCIENCES OF THE UNITED STATES OF AMERICA, vol. 102, no. 8, 22 February 2005 (2005-02-22), pages 2928-2933, XP002527680 ISSN: 0027-8424
- SANDHU JASWINDERPAL S ET AL: "Stem cell properties and repopulation of the rat liver by fetal liver epithelial progenitor cells" AMERICAN JOURNAL OF PATHOLOGY, vol. 159, no. 4, October 2001 (2001-10), pages 1323-1334, XP002605655 ISSN: 0002-9440
- SIERRA ELENA ET AL: "Liver gene expression and increase in albumin synthesis by fetal hepatocytes transplanted into analbuminemic rats" LIFE SCIENCES, vol. 67, no. 20, 6 October 2000 (2000-10-06), pages 2417-2432, XP002605656 ISSN: 0024-3205
- BATANOV A N ET AL: "Effect of fetal tissue transplantation on reparative processes in experimental liver cirrhosis." BULLETIN OF EXPERIMENTAL BIOLOGY AND MEDICINE AUG 2000 LNKD- PUBMED:11177248, vol. 130, no. 8, August 2000 (2000-08), pages 798-801, XP002605657 ISSN: 0007-4888
- THOMAS DAVID BRYNMOR: "The infusion of human fetal liver cells" STEM CELLS (DAYTON), vol. 11, no. SUPPL. 1, 1993, pages 66-71, XP002605658 & CONFERENCE CELEBRATING THE 40TH ANNIVERSARY OF THE INSTITUTE OF HEMATOLOGY AND BLOOD TRANSFUSION; PRAGUE, CZECH REPUBLIC; OCTOBER 23-24, 1992 ISSN: 1066-5099

## Description

The present invention relates to a eastational stage porcine pancreatic organ or tissue graft for use in the treatment of a pancreatic disorder associated with a pathological organ or tissue physiology or morphology according to claim 1.

Disclosed are methods of treating diseases by transplantation of developing, non syngeneic organs/tissues. More particularly, disclosed are relates to methods of treating disease via transplantation of 20- to 42 day gestational stage porcine organs/tissues, respectively.

Transplantation of fully differentiated organs/tissues is a widely practiced, life-saving, medical procedure of choice for treatment of numerous highly debilitating or lethal diseases, including kidney, heart, pancreas, lung, hematological, genetic, and liver diseases. For example, the number of human kidney transplants has increased rapidly in recent years, but the demand greatly exceeds organ availability. In the case of kidney failure, permanent hemodialysis can be used to prolong life, however, this is a highly debilitating, cumbersome and expensive procedure with limited effectiveness which carries a significant risk of opportunistic infection. In the case of diabetes, a disease of tremendous medical and economic impact, daily injection of insulin, the standard prior art therapy, does not satisfactorily prevent the debilitating or lethal consequences of this disease. World-wide, diabetes occurs in nearly 5 percent of the population ranging in age from 20 to 79 years, and hence affects 150 million people. In the United States alone, an estimated 17 million people-over 6 percent of the population-have diabetes mellitus, and each year about 1 million Americans aged 20 or older are diagnosed with the disease. In 1999, about 450,000 deaths occurred among adults with diabetes in the United States. Heart disease is the predominant cause of disability and death in all industrialized nations, and, in addition, the incidence of heart failure is increasing in the United States, with more than half a million Americans dying of this disease yearly (Braunwald E., 1997. N Eng J Med. 337:1360; Eriksson H., 1995. J Inter Med. 237:135). In addition, in the United States, cardiac disease accounts for about 335 deaths per 100,000 individuals (approximately 40 % of the total mortality) overshadowing cancer, which follows with 183 deaths per 100,000 individuals. Liver damage occurs in a number of acute and chronic clinical conditions, including drug-induced hepatotoxicity, viral infections, vascular injury, autoimmune disease and blunt trauma. In addition, patients subject to inborn errors of metabolism may be at risk for developing liver damage. Symptoms of liver damage occurring as a result of these clinical conditions include, for example, fulminant hepatic failure with cholestasis, hepatic lesions, and liver tissue necrosis, and in many instances, the restoration of normal liver function is vital to the survival of patients.

Therapeutic transplantation in humans is normally performed by transplanting fully differentiated organs/tissues between suitably haplotype matched allogeneic donors and recipients. Such a treatment modality, however, suffers from considerable disadvantages. Allogeneic transplantation of differentiated organs/tissues is impossible to implement in a great many cases due to the unavailability of suitably immunologically and morphologically matched transplant donors. Furthermore, use of human donors to provide organs/tissues for transplantation requires subjecting live donors to major surgery, for example in the case of kidney transplantation. Alternately, the use of cadaveric organs/tissues also often presents ethical dilemmas. In the case of diabetes, transplantation of adult cadaveric donor pancreatic islets has been shown to be technically feasible, however, this approach cannot be routinely practiced due to the insufficient numbers of immunologically matching allogeneic donor pancreases from which to isolate the sufficient numbers of islets required.

Thus, large numbers of patients who would otherwise benefit from therapeutic transplantation succumb to diseases associated with kidney, heart, liver, pancreatic, pulmonary or hematological failure, while awaiting matched transplant donors. Moreover, even when suitably haplotype matched transplant donors are found, permanent and harmful immunosuppressive treatments, such as daily administration of toxic drugs such as cyclosporin A, are generally required to prevent graft rejection. Use of drugs such as cyclosporin A is highly undesirable since these cause severe side-effects such as carcinogenicity, nephrotoxicity and increased susceptibility to opportunistic infections. Such immunosuppressive treatments contribute to the drawbacks of allogeneic transplantation since these are often unsuccessful in preventing rejection in the short term, and are generally incapable of indefinitely preventing rejection in the long term. Acute rejection of cardiac or hepatic grafts is often fatal. In the case of kidney transplantation, the inability of current immunosuppressive regimens to prevent acute graft rejection often necessitates emergency surgical intervention to remove the graft followed by the necessity to be placed on kidney dialysis pending availability of another compatible organ for transplantation.

An alternative to allograft transplantation which has been proposed involves xenograft transplantation, i.e., transplantation of animal derived grafts, in particular porcine grafts which are well established as the potential animal alternative of choice to human grafts. The great advantages of using xenografts for transplantation would be their availability on demand to all patients in need of transplantation, as well as avoidance of the medical and ethical burden of harvesting grafts from live or cadaveric human donors. However, to date, xenogeneic organ/tissue grafts have been ruled out for human transplantation due to their heretofore insurmountable immunological incompatibility with human recipients.

Thus, the ability to generate organs/tissues, such as pancreatic, renal, hepatic, cardiac or lymphoid organs/tissues, suitable for therapeutic transplantation in sufficient quantities and optimally tolerated in immunocompetent humans without or with minimal immunosuppression is a highly desired goal. One strategy which has been proposed to fulfill this aim involves using organs/tissues at early developmental stages for transplantation. Such an approach is promising since it has been shown that immunological tolerance to grafts derived from developing tissue is better than that to grafts derived from adult stage tissues (Dekel B. et al., 1997. Transplantation 64, 1550; Dekel B. et al., 1997. Transplantation 64, 1541; Dekel B. et al., 1999. Int Immunol. 11, 1673; Hammerman MR., 2000. Pediatr Nephrol. 14, 513). Furthermore, the enhanced growth and differentiation potential of developing organs/tissues is highly desirable for generating optimally functional, host integrated grafts. For example, developing human renal tissue derived grafts were shown to display reduced tissue apoptosis and destruction as well as a sustained growth phase (Dekel B. et al., 1997. Transplantation 64, 550; Dekel B. et al., 2000. Transplantation 69, 1470). In the developing human kidney, fresh stem cells are induced into the nephrogenic pathway to form nephrons until 34 weeks of gestation. Such nephrogenic differentiation pathway involves invasion of a specialized region of intermediate mesoderm by an epithelial source (ureteric bud), which grows and branches to form a collecting duct system, and induces disorganized metarenal mesenchymal stem cells to group and differentiate into nephrons [Woolf, A.S. in: Pediatric Nephrology, 4th ed. Barratt, T.M., Avner, A. and Harmon, W. (eds.), Williams & Wilkins, Baltimore, Maryland. pp. 1-19 (1999)]. Thus, transplants of gestational stage renal tissue may be a potential source of regenerating kidney cells, and a promising solution for the current shortage of organs for kidney transplantation. In the case of pancreatic tissues, pancreatic islet cells, such as insulin producing beta cells, display enhanced cell growth and differentiation relative to differentiated islet beta cells. For example, it has been shown that human fetal islets including the earliest insulin secreting cells, transplanted into nude mice and rats, which are immunodeficient hosts, display continued growth and development, including production of the other pancreatic hormones; glucagon, somatostatin, and pancreatic polypeptide (Usadel et al., 1980. Diabetes 29 Suppl 1:74-9). Similarly, it has been shown that human embryonic pancreas-derived grafts transplanted into NOD/SCID mice, which are also immunodeficient hosts, generated graft-derived insulin producing human beta cells (Castaing M. et al., 2001. Diabetologia 44:2066). It has also been shown that gestational stage porcine islet transplants in mice may display a similar differentiation program, with similar timing, as the normal non transplanted tissues.

Various mechanisms have been suggested to explain the reduced immunogenicity of developing tissue grafts. It has been suggested that such developing tissue derived grafts induce attenuated host anti graft immune responses compared to adult stage tissue derived grafts due to the former being predominantly vascularized by host derived vasculature, as opposed to the predominantly graft derived graft vascularization observed in the latter (Hyink D. P. et al., 1996. Am J Physiol. 270, F886). It has further been suggested that the low levels of major histocompatibility (MHC) and adhesion molecule expression, and of antigen presenting cells in gestational stage tissue grafts decreases the capacity of such grafts to activate host immune responses.

Approaches involving utilization of developing human organs/tissues, however, are hampered by the practical and ethical obstacles involved in obtaining sufficient numbers of human embryos/fetuses, as well as the ethical problems involved with the use of human embryonic tissue. To circumvent such obstacles, the use of animal derived developing organs/tissues, in particular porcine developing organs/tissues has been suggested (Auchincloss, H. and Sachs, D.H., 1998. Annu. Rev. Immunol. 16, 433-470; Hammerman, M.R., 2002. Curr. Opin. Nephrol. Hypertens. 11, 11-16).

Various approaches for utilizing transplantation of developing, non syngeneic organs/tissues for treatment of diseases have been attempted in the prior art.

One approach involves transplanting gestational stage kidneys into allogeneic, non-immunosuppressed recipients in attempts to generate graft derived, functional, immunologically tolerated renal organs in such recipients, as shown using transplantation of grafts from embryonic day 15 rats under the renal capsule or into the omentum of non-immunosuppressed adult rat hosts, without (Rogers, S.A. et al., 1998. Kidney Int. 54, 27-37; Rogers, S.A. et al., 2001. Am. J. Physiol. Regul. Integr. Comp. Physiol. 280, R132-136), or with (Rogers, S.A. and Hammerman, M.R., 2001. Am. J. Physiol. Regul. Integr. Comp. Physiol. 281, R661-665) prior *in-vitro* preservation of grafts.

Another approach involves transplanting gestational stage kidneys into xenogeneic recipients treated with CTLA4-Ig blockade of costimulation in attempts to generate graft derived, functional, immunologically tolerated renal organs in such recipients, as shown using transplantation of grafts from embryonic day 15 rats into mouse hosts (Rogers, S.A. and Hammerman, M.R., 2001. Am. J. Physiol. Regul. Integr. Comp. Physiol. 280, R1865-1869).

Yet another approach involves transplanting gestational stage renal tissue into immunodeficient xenogeneic recipients reconstituted with human PBMCs in attempts to generate functional, immunologically tolerated, graft derived renal organs in such hosts, as shown by transplantation of 12- to 22-week gestational stage human tissue into SCID/Lewis and SCID/nude chimeric rats (Dekel B. et al., 1997. Transplantation 64, 1550), and transplantation of 70-day gestational stage organs/tissues into NOD/SCID mice (Dekel B. et al., 2001. J Am Soc Nephrol. 13, 977-90; Dekel B. et al., 2000. Transplantation 69, 1470).

Still another approach involves transplanting cultured gestational stage pancreatic grafts into xenogeneic immunodeficient recipients in attempts to generate functional, immunologically tolerated, graft derived pancreatic cells and tissues in such recipients, as attempted by transplantation of gestational stage porcine islet cells in nude mice (Otonkoski T. et al., 1999. Transplantation 68, 1674), of human fetal islets in nude mice and rats (Usadel et al., 1980. Diabetes 29 Suppl 1:74-9), and of human embryonic pancreases in NOD/SCID mice (Castaing M. et al., 2001. Diabetologia 44:2066).

A further approach involves transplanting porcine fetal islet cell clusters intraportally or under the renal capsule in diabetic human recipients in attempts to treat diabetes in such recipients (Groth CG. et al., 1999. J Mol Med. 77,153).

Yet a further approach involves striatal transplantation of allogeneic fetal ventral mesencephalic tissue into human recipients with Parkinson's disease in attempts to treat this disease (Subramanian, T., 2001. Semin Neurol. 21, 103; Schumacher JM. et al., 2000. Neurology 54, 1042).

However, all prior art approaches involving transplantation of developing, non syngeneic tissues suffer from some or all of the following drawbacks: (i) suboptimal tolerance by allogeneic/xenogeneic human lymphocytes; (ii) suboptimal structural and functional differentiation, for example with respect to urine production by renal grafts, or insulin production by pancreatic grafts; (iii) predominantly graft derived, as opposed to host derived vascularization; (iv) suboptimal growth; (v) inadequate availability of transplantable organs/tissues; and/or (vi) suboptimal safety for human administration, notably with respect to avoidance of generation of graft-derived teratomas.

Thus, all prior art approaches have failed to provide an adequate solution for using transplantation of developing, non syngeneic organs/tissues to treat human diseases amenable to therapeutic transplantation.

There is thus a widely recognized need for, and it would be highly advantageous to have, a method of treating human diseases amenable to therapeutic transplantation by transplantation of developing and/or non syngeneic organs/tissues devoid of the above limitation. The gestational stage porcine pancreatic organ or tissue graft for use in the treatment of a pancreatic disorder associated with a pathological organ or tissue physiology or morphology of the present invention is defined In claim 1.

According to one aspect there is provided a method of treating a disorder associated with pathological organ or tissue physiology or morphology, the method comprising transplanting into a subject in need thereof a therapeutically effective mammalian organ or tissue graft, the organ or tissue graft selected not substantially expressing or presenting at least one molecule capable of stimulating or enhancing an immune response in the subject, thereby treating the disorder in the subject.

According to further features in preferred embodiments described below, the organ or tissue graft is a porcine organ or tissue graft.

According to still another aspect of the present invention there is provided a method of treating a disorder associated with pathological organ or tissue physiology or morphology, the method comprising transplanting into a subject in need thereof a therapeutically effective porcine organ or tissue graft, the porcine organ or tissue graft selected at a stage of differentiation corresponding to 20 to 63 days of gestation, thereby treating the disorder in the subject.

According to further features in preferred embodiments of the invention described below, the stage of differentiation corresponds to 20 to 56 days of gestation.

According to still further features in the described preferred embodiments, the stage of differentiation corresponds to 20 to 42 days of gestation.

According to a further aspect of the present invention there is provided a method of evaluating the suitability of a graft for transplantation into a subject, the method comprising testing the graft for expression or presentation of at least one molecule capable of stimulating or enhancing an immune response in the subject, thereby evaluating the suitability of the graft for transplantation into the subject.

According to still further features in the described preferred embodiments, the mammalian graft is a porcine graft.

According to still further features in the described preferred embodiments, the testing is effected via RT-PCR analysis of the graft.

According to still further features in the described preferred embodiments, the method of treating the disorder further comprises treating the subject with an immunosuppressive regimen prior to, concomitantly with or following transplanting the organ or tissue graft into the subject, thereby promoting engraftment of the organ or tissue in the subject.

According to still further features in the described preferred embodiments, the treating the subject with an immunosuppressive regimen is effected by administering an immunosuppressant drug to the subject.

According to still further features in the described preferred embodiments, the immunosuppressant drug is capable of blocking binding of a lymphocyte coreceptor with a ligand of the lymphocyte coreceptor.

According to still further features in the described preferred embodiments, the immunosuppressant drug is CTLA4-Ig.

According to still further features in the described preferred embodiments, the administering an immunosuppressant drug to the subject is effected during a single time period selected from a range of 1 to 20 days.

According to still further features in the described preferred embodiments, the at least one molecule capable of stimulating or enhancing an immune response in the subject is a lymphocyte coreceptor or lymphocyte coreceptor ligand.

According to still further features in the described preferred embodiments, the lymphocyte coreceptor or lymphocyte coreceptor ligand is selected from the group consisting of B7-1, CD40, and CD40L.

According to still further features in the described preferred embodiments, selecting the organ or tissue graft is effected via RT-PCR analysis of the organ or tissue graft.

According to still further features of the disclosure, the organ or tissue graft is a renal organ or tissue graft, and transplanting the organ or tissue graft into the subject is effected by transplanting the organ or tissue graft into an anatomical location of the subject selected from the group consisting of the renal capsule, the kidney, the portal vein, the liver, the spleen, the testicular fat, the sub-cutis, the omentum and the intra-abdominal space.

According to still further features in the described preferred embodiments, the organ or tissue graft is a pancreatic organ or tissue graft, and transplanting the organ or tissue graft into the subject is effected by transplanting the organ or tissue graft into an anatomical location of the subject selected from the group consisting of the portal vein, the liver, the pancreas, the renal capsule, the testicular fat, the sub-cutis, the omentum and the intra-abdominal space.

According to still further features of the disclosure, the organ or tissue graft is a hepatic organ or tissue graft, and transplanting the organ or tissue graft into the subject is effected by transplanting the organ or tissue graft into an anatomical location of the subject selected from the group consisting of the portal vein, the liver, the renal capsule, the testicular fat, the sub-cutis, the omentum, the spleen and the intra-abdominal space.

According to still further features of the disclosure, the organ or tissue graft is a cardiac organ or tissue graft, and transplanting the organ or tissue graft into the subject is effected by transplanting the organ or tissue graft into an anatomical location of the subject selected from the group consisting of the the heart cavity, the heart, the myocardium, and the intra-abdominal space.

According to still further features of the disclosure, the organ or tissue graft is a lymphoid organ or tissue graft, and transplanting the organ or tissue graft into the subject is effected by transplanting the organ or tissue graft into an anatomical location of the subject selected from the group consisting of the portal vein, the liver, the renal capsule, the sub-cutis, the omentum and the intra-abdominal space.

According to still further features of the disclosure, the disorder is a kidney disorder, and the organ or tissue graft is a renal organ or tissue graft.

According to still further features in the described preferred embodiments, the disorder is a pancreatic disorder, and the organ or tissue graft is a pancreatic organ or tissue graft.

According to still further features in the described preferred embodiments, the pancreatic disorder is diabetes, and the pancreatic organ or tissue graft is a pancreatic islet organ or tissue graft.

According to still further features of the disclosure, the disorder is a hepatic disorder and/or metabolic disorder, and the organ or tissue graft is a hepatic organ or tissue graft.

According to still further features of the disclosure, the disorder is a cardiac disorder, and the organ or tissue graft is a cardiac organ or tissue graft.

According to still further features of the disclosure, the disorder is a hematological and/or genetic disorder, and the organ or tissue graft is a lymphoid organ or tissue graft.

According to still further features of the disclosure, the lymphoid organ or tissue graft is selected from the group consisting of a splenic graft, a lymph node derived graft, a Peyer's patch derived graft, a thymic graft and a bone marrow derived graft.

According to still further features in the described preferred embodiments, the subject is a mammal.

According to still further features in the described preferred embodiments, the mammal is a human.

According to a yet a further aspect of the present invention there is provided a method of evaluating the stage of differentiation of a mammalian organ or tissue most suitable for transplantation thereof into a mammalian subject, the method comprising evaluating a test transplant taken from the organ or tissue at a specific stage of differentiation for the presence of at least one molecule capable of stimulating or enhancing an immune response in the subject prior to and/or following a test transplantation of the test transplant into a mammalian test recipient, wherein an effective absence of the at least one molecule in the test transplant prior to and/or following the test transplantation indicates that the specific stage of differentiation is suitable for transplantation of the organ or tissue into the subject.

According to further features in preferred embodiments of the invention described below, the method of evaluating the developmental stage of an organ or tissue most suitable for transplantation thereof into a subject further comprises evaluating the test transplant for the presence of the at least one molecule capable of stimulating or enhancing an immune response in the subject prior to the test transplantation.

According to still further features in the described preferred embodiments, evaluating the test transplant for the presence of the at least one molecule is effected following a posttransplantation period of the test transplantation selected from the range of 1 second to 45 days.

According to still further features in the described preferred embodiments, the test recipient is a rodent and/or the subject.

According to still further features in the described preferred embodiments, the rodent is a mouse.

According to still further features in the described preferred embodiments, the test recipient bears functional human T lymphocytes.

According to still further features in the described preferred embodiments, the human T lymphocytes and the organ or tissue are non syngeneic.

According to still further features in the described preferred embodiments, the at least one molecule capable of stimulating or enhancing an immune response in the subject is a lymphocyte coreoeptor or lymphocyte coreceptor ligand.

According to still further features in the described preferred embodiments, the lymphocyte coreceptor or lymphocyte coreceptor ligand is selected from the group consisting of B7-1, CD40 and CD40L.

According to still further features in the described preferred embodiments, the testing is effected via RT-PCR analysis of the test transplant.

According to still further features in the described preferred embodiments, the organ or tissue is selected from the group consisting of an organ explant, a tissue explant, a cell explant, an organ culture, a tissue culture, and a cell culture.

According to still further features in the described preferred embodiments, the organ or tissue is a porcine organ or tissue.

According to still further features in the described preferred embodiments, the organ or tissue is a porcine organ or tissue and the specific stage of differentiation is selected corresponding to 20 to 63 days of gestation.

The present invention successfully addresses the shortcomings of the presently known configurations by providing a generally applicable and optimal method of treating essentially any disease amenable to therapeutic transplantation using transplantation of allogeneic/xenogeneic organ or tissue grafts by virtue of such grafts enabling generation of graft-derived organs/tissues which display optimal structural and functional lineage-specific differentiation, and are optimally tolerated by alloreactive/xenoreactive human lymphocytes in a recipient without or with minimal immunosuppression.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In case of conflict, the patent specification, including definitions, will control. In addition, the materials, methods, and examples are illustrative only and not intended to be limiting.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

All figures and examples relating to human embryonic tissue are only disclosed as reference but not part of the invention. Furthermore, the parts relating to other organs than those in the claims are not part of the invention.

In the drawings:

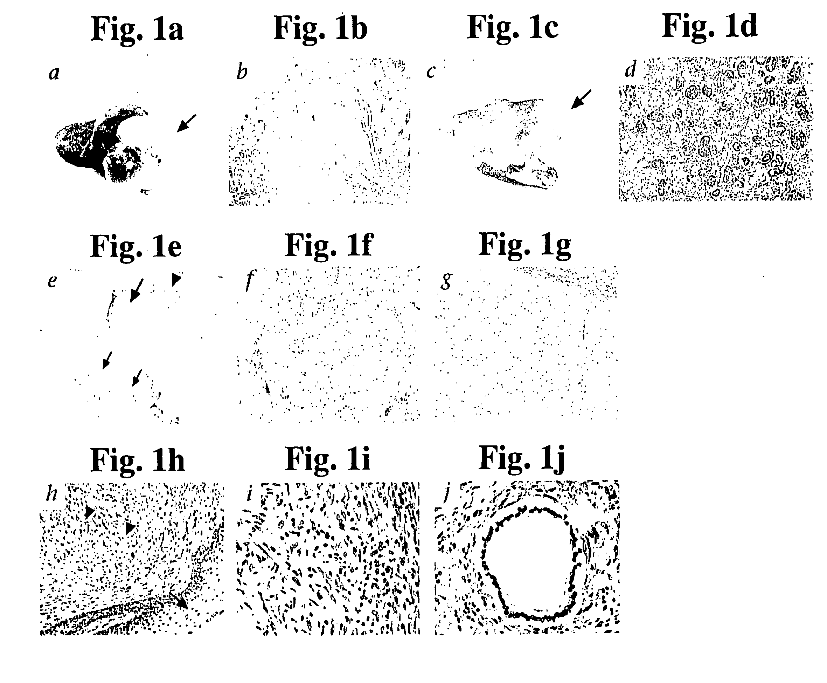

FIGs. 1a-j are photographs depicting growth and differentiation of early human and porcine kidney precursors after transplantation. Figures 1a-b, respectively, depict a macroscopic view and histology (Figure 1b; H&E; ×10 original magnification) of an 8-week gestational stage human renal tissue graft, 8 weeks after transplantation. Note massive growth and the formed shape of a kidney (arrow) and appearance of layers of glomeruli and tubuli. Figures 1c-d are a macroscopic view and histological analysis (H&B; ×10 original magnification), respectively, of a 4-week gestational stage porcine renal tissue graft, 8 weeks after transplantation. Note massive growth (arrow) and external vascular beds and numerous glomeruli and tubuli. Transplanted early embryonic kidney cells differentiate into other cell fates following transplantation of 20- to 21-day gestational stage (Figures 1e-g) and 24- to 25-day gestational stage (Figures 1h-j) porcine renal grafts. Figures le is a ×4 original magnification H&E histology photomicrograph showing blood vessels (arrowheads), cartilage (large arrow), and bone (small arrows). Figures 1f-g are ×40 original magnification H&E histology photomicrographs depicting bone and cartilage, respectively. Figure 1h is a ×10 original magnification H&E histology photomicrograph showing myofibroblasts (arrowheads) and cartilage (large arrow). Figures 1i-j are ×40 original magnification H&E histology photomicrographs depicting myofibroblasts and a representative glandular tissue-like structure, respectively.

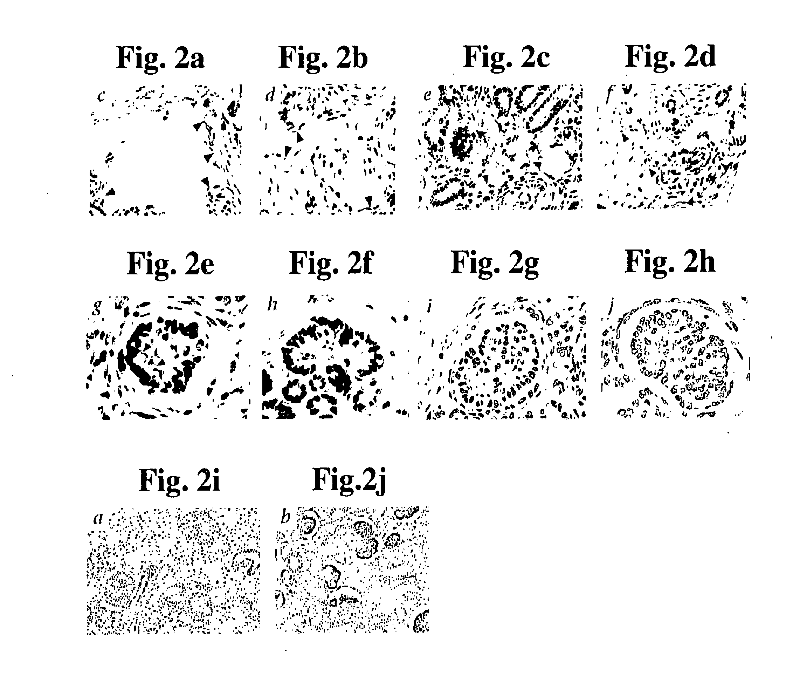

FIGs. 2a-j are photomicrographs depicting vascularization of early gestational stage renal tissue derived grafts by mouse recipient blood vessels. Immunostaining of 8-week gestational stage human (Figures 2a, 2c, and 2e) and 4-week gestational stage porcine (Figures 2b, 2d, and 2f) renal tissue grafts, 4 weeks after transplantation, with antibody against mouse CD31 (PECAM) is shown (×40 original magnification). Figures 2c-d depict positive staining (arrowheads) in larger vessels, Figures 2e-f depict medium and small-size capillaries, and Figures 2g-h depict developing glomeruli. Figures 2g-h depict lack of staining in glomeruli and small-size capillaries mature 16-week gestational stage human and 8-week gestational stage porcine fetal kidney tissue, 4 weeks after transplantation. Figures 2i-j show lack of host derived vessels in control vascularized human and porcine fetal kidneys, respectively (×20 original magnification).

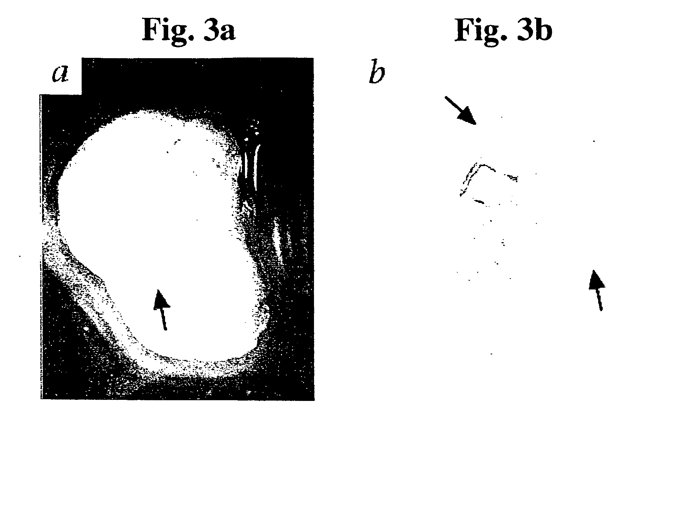

FIGs. 3a-b are whole-graft photographs depicting urine-like fluid filled cysts generated by transplants of early embryonic human and porcine kidney precursors. Figures 3a-b, respectively, depict macroscopic views of 8-week gestational stage human, and 4-week gestational stage porcine renal tissue derived intra-abdominal grafts containing large cysts (indicated by arrows), 8 weeks after transplantation. Analysis of cyst fluid identified it as dilute urine.

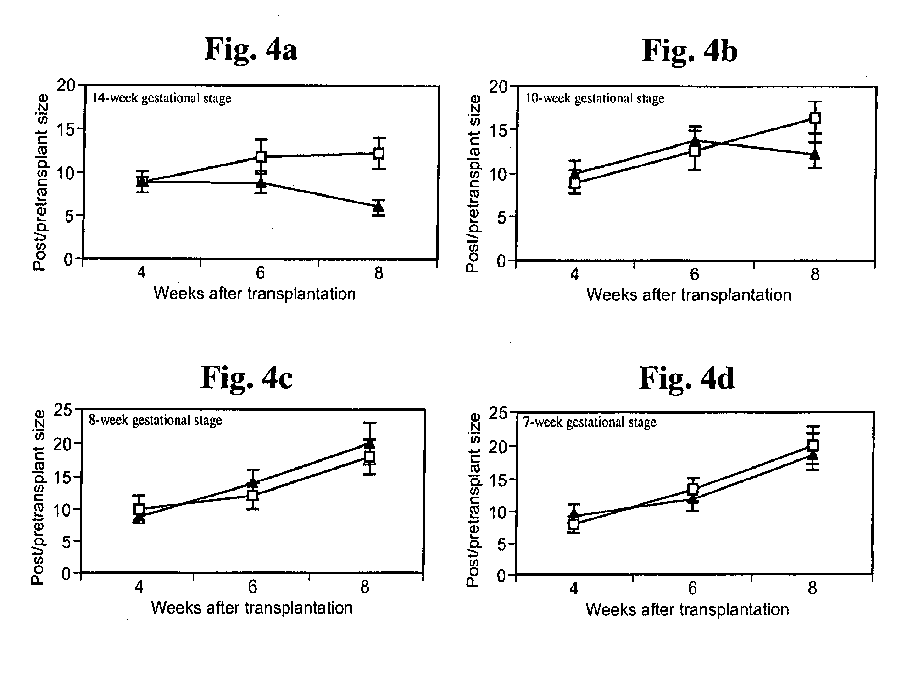

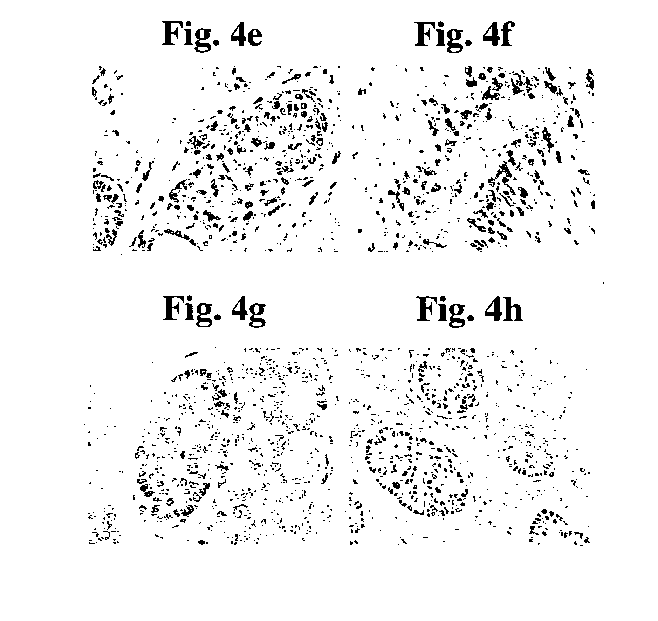

FIGs. 4a-d are data plots depicting growth curves of 14-, 10-, 8-, and 7-week gestational stage human renal tissue grafts, respectively, in the presence (▲, closed triangles) or absence (□, open squares) of alloreactive human PBMCs. In 14- or 10-week gestation stage renal tissue grafts, 8 weeks after transplantation, *P* < 0.01 and *P* < 0.05 compared with controls, respectively.
FIGs. 4e-f are photomicrographs depicting a transplant of a 14-week gestational stage human renal tissue graft immunostained with antibodies against human CD3 (×40 original magnification) demonstrating destruction of glomerulus (Figure 4e) and tubule (Figure 4f) by human T cells.
FIGs. 4g-h are photomicrographs depicting an 8-week gestational stage renal tissue derived transplant immunostained with antibodies specific for human CD3 (×40 original magnification). Note absence of T cell infiltration, and presence of intact glomeruli and tubuli (Figures 4g-h, respectively).

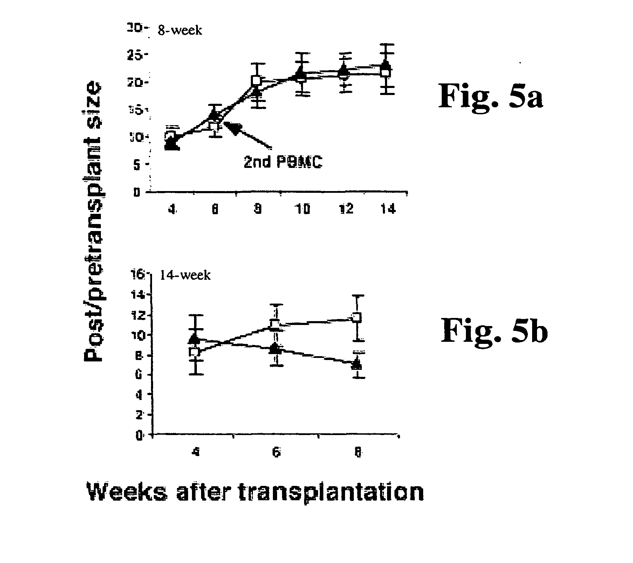

FIGs. 5a-b are data plots depicting similar growth curves of 8-week gestational stage human renal tissue derived grafts (Figure 5a) in recipients either receiving two independent infusions of alloreactive human PBMCs at the time of transplantation and 6 weeks post-transplant (▲, closed triangles), or in recipients not infused with PBMCs (□, open squares). The growth curve of transplants originating from 14 week old human fetuses demonstrates halted growth (Figure 5b; ▲, closed triangles) when the latter are transplanted in recipients concomitantly with the second dose of human PBMCs, as compared to those not subjected to PBMC infusion (□, open squares; *P* < 0.05, 8 weeks after transplantation).

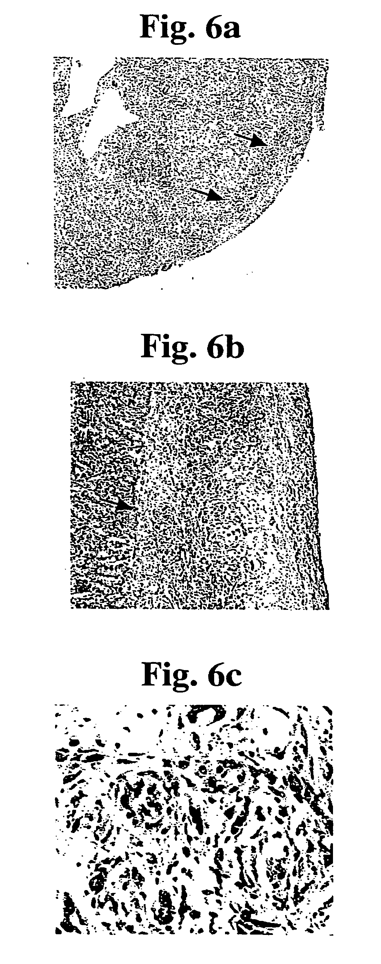

FIGs. 6a-c are photomicrographs depicting rejection of adult porcine renal tissue derived grafts by human leukocytes. Figures 6a-b are ×4 and ×20 magnification views, respectively, depicting hematoxylin and eosin (H&E) histological staining of subcapsular adult porcine kidney tissue derived grafts 4 weeks following intraperitoneal infusion of human PBMCs. Figure 6c depicts T cell infiltration in transplanted tissue, as determined using immunohistochemical detection of human CD3.

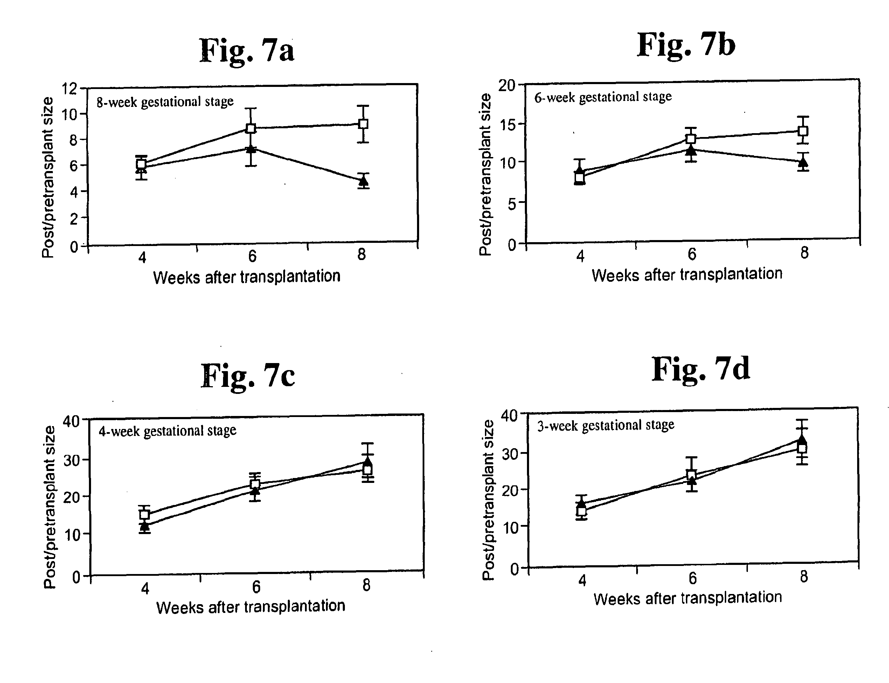

FIGs. 7a-d are data plots depicting growth curves of 8-, 6-, 4-, and 3-week gestational stage porcine renal tissue grafts (Figures 7a-d, respectively) in the presence (▲, closed triangles) or absence (□, open squares) of xenoreactive human PBMCs. In transplants originating from 8- or 6-week-old porcine fetuses, 8 weeks after transplantation, *P* < 0.01 and *P* < 0.05 compared with controls, respectively.

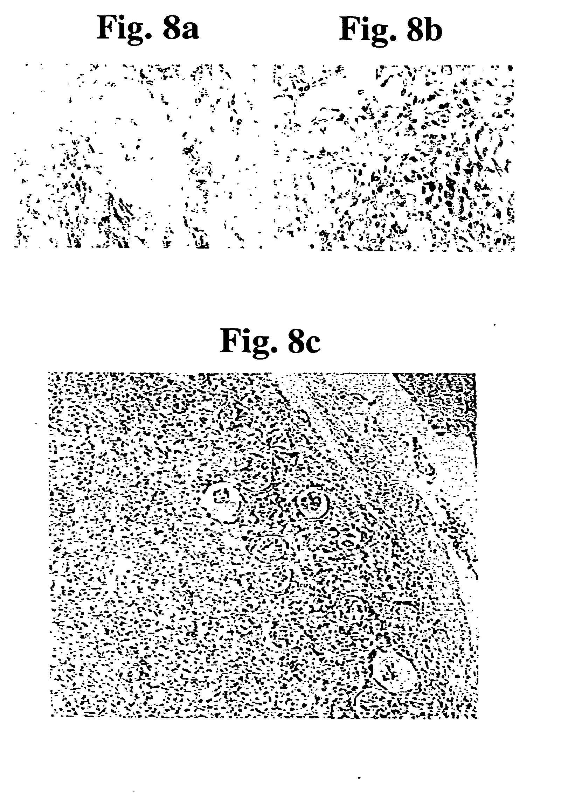

FIGs. 8a-c are photomicrographs depicting destruction of transplant tissue by invading human T cells in a transplant derived from 8-week gestational stage porcine renal tissue, 4 weeks posttransplantation. Figures 8a-b (×40 original magnification) depict immunostaining with antibodies against human CD3, and FIG. 8c depicts H&E histological staining (×10 original magnification).

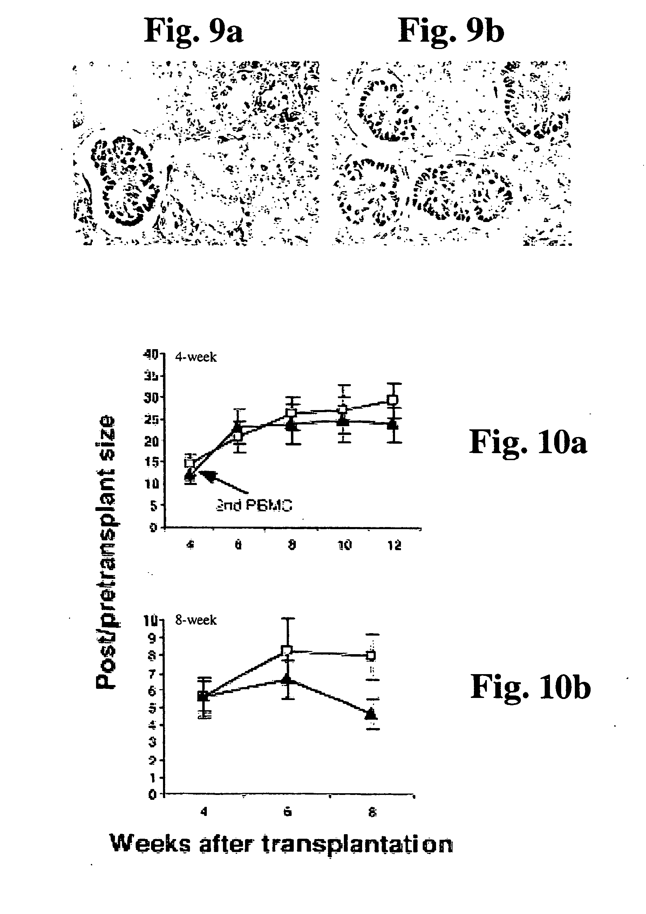

FIGs. 9a-b are photomicrographs of a 4-week gestational stage porcine renal tissue derived transplant demonstrating preserved glomeruli and tubuli with no CD3 positive infiltrate (×40 original magnification), 4 weeks posttransplantation.
FIGs. 10a-b are data plots depicting similar growth curves of 4-week gestational stage porcine renal tissue derived grafts (Figure 10a) in recipients either receiving 2 independent infusions of xenoreactive human PBMCs at the time of transplantation and 4 weeks post-transplant (▲, closed triangles), or in recipients not infused with PBMCs (□, open squares). The growth curve (Figure 10b) of transplants originating from 8-week-old porcine fetuses demonstrates arrested growth (▲, closed triangles) when the latter are transplanted in recipients concomitantly with the second dose of human PBMCs and compared to those not subjected to PBMC infusion (□, open squares) (P < 0.05, 8 weeks after transplantation).

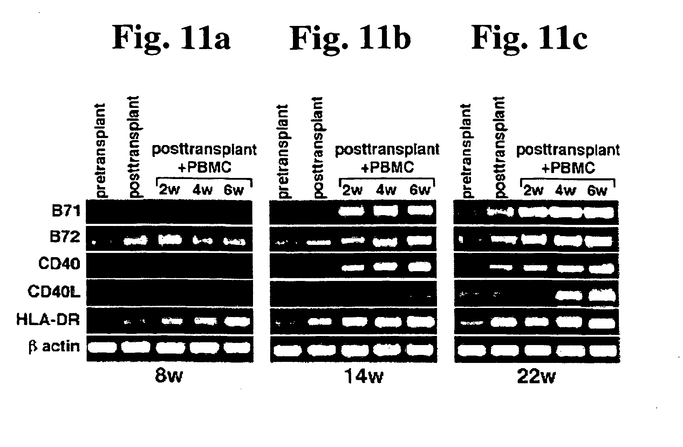

FIGs. 11a-c are agarose gel electrophoresis UV photographs depicting RT-PCR analysis of co-stimulatory molecule mRNA expression in normal human developing kidney tissue (pre-transplant), in transplanted developing human renal tissue immediately after transplantation, but prior to administration of alloreactive human PBMCs (post-transplant), and at 2, 4 and 6 weeks after recipient mice were reconstituted with human PBMCs. Transplants analyzed were derived from 8-, 14-and 22-week gestational stage human renal tissues (Figures 11a-c, respectively).

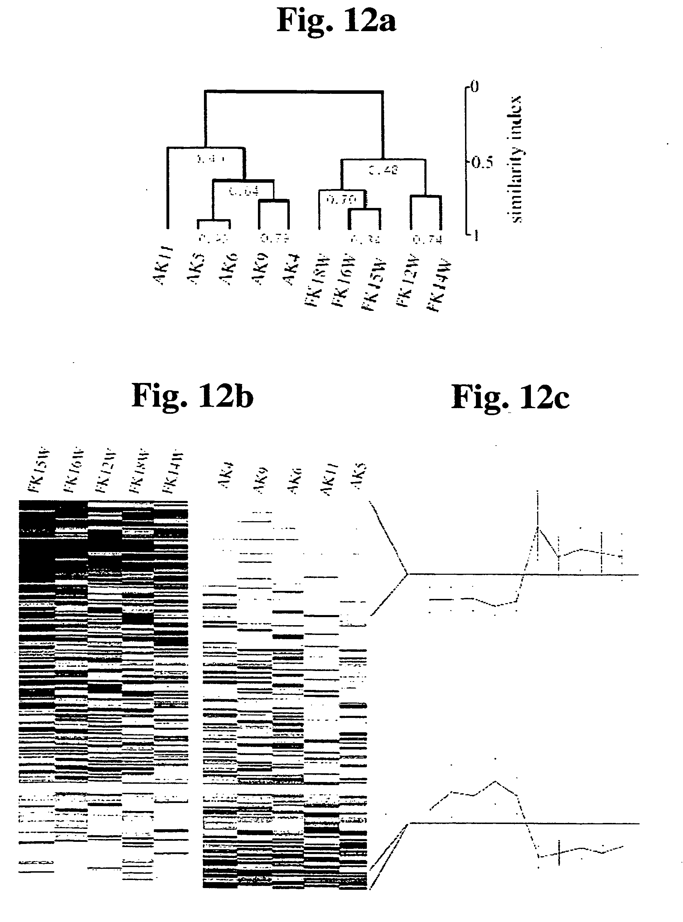

FIGs. 12a-c depict differential gene expression patterns of immunity related genes in normal adult versus gestational stage human renal tissues. Figure 12a is a hierarchical clustering dendrogram (Zuo, F. et al., 2002. Proc. Natl. Acad. Sci. USA 99, 6292-6297) of the experimental groups generated on the basis of the similarity of their expression profiles depicting that the adult and fetal expression patterns cluster separately. Figure 12b is a microarray analysis output diagram depicting gene expression patterns in the 231 immunity related genes analyzed showing that 122 of such genes scored a TNoM = 0 or 1 (Kaminski, N. and Friedman, N., 2002. Am. J. Respir. Cell Mol. Biol. 27, 125-132). Gene expression values were divided by a geometric mean of all samples, log transformed and then plotted using PLOTTOPGENE software (Kaminski, N. and Friedman, N., 2002. Am. J. Respir. Cell Mol. Biol. 27, 125-132). Yellow and purple represent maximal and minimal expression, respectively. Note that most of the immunity related genes were expressed at lower levels in gestational stage compared to adult renal tissue. Figure 12c is a data plot depicting gene expression of 68 genes having TNoM = 0 (P < 0.05). Plots are the mean expression values of all genes in the group. To eliminate outlier effect, genes were normalized to a range of [0,1], signifying that the maximum value for every gene was set to be 1, the minimum value to be zero, and the rest of the values were linearly fitted to this range. Note again that most statistically significant genes (57/68) were lower in gestational stage as compared to adult stage renal tissue.

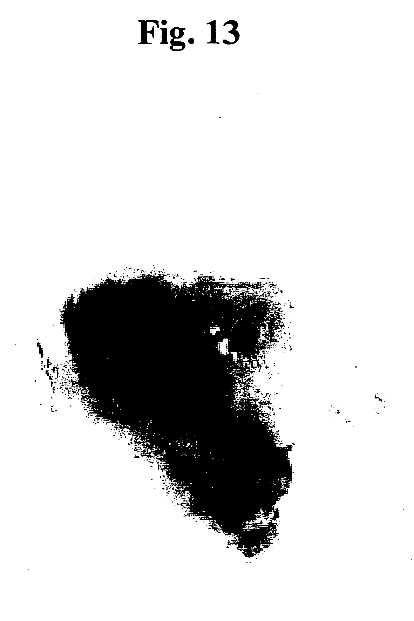

FIG. 13 is a whole graft photograph depicting a 12-week gestational stage human pancreatic tissue derived graft, 8 weeks posttransplantation, transplanted in an NOD/SCID mouse recipient bearing alloreactive human PBMCs. Note pronounced growth of the graft and the absence of any signs of graft rejection.

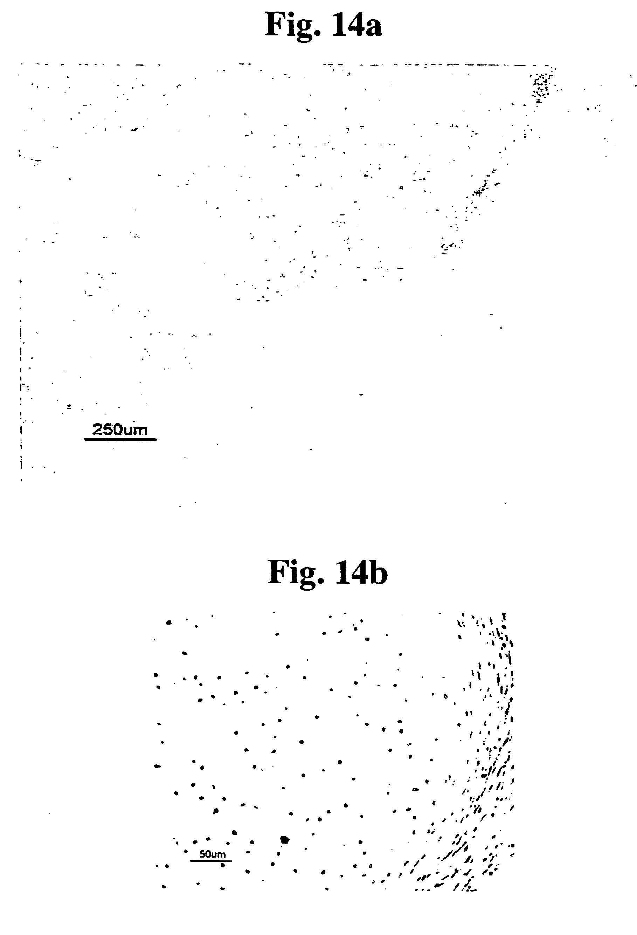

FIGs. 14a-b are photomicrographs depicting an H&E-stained 21-day gestational stage porcine liver-derived graft transplanted into an NOD/SCID mouse recipient, 7 weeks posttransplantation at ×4 and ×20 original magnification, respectively. Figure 14a shows clear teratoma development with extensive cartilage differentiation (Figure14b).

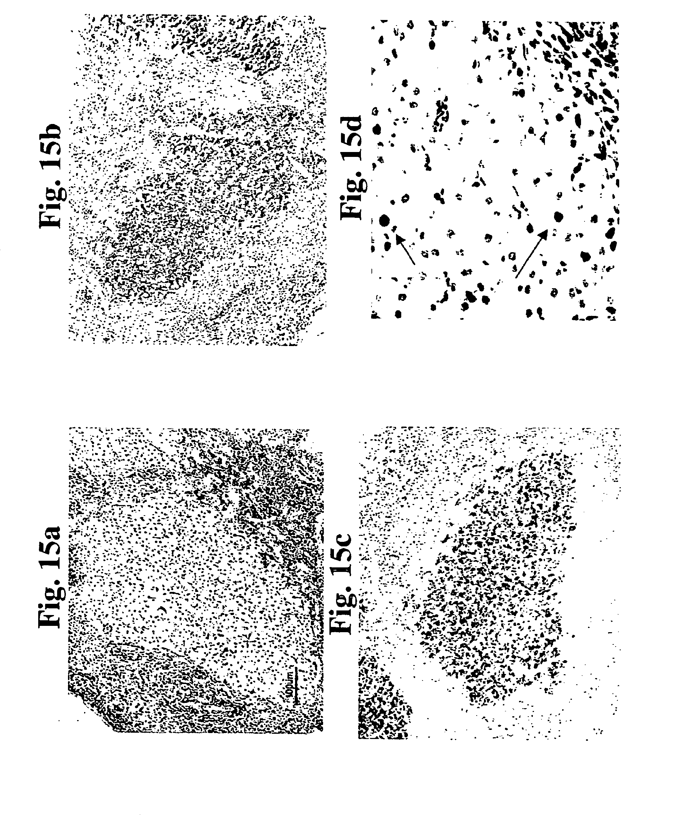

FIGs. 15a-d are photomicrographs depicting hepatic differentiation in histology sections of a 28-day gestational stage porcine liver-derived grafts transplanted into the spleen of an NOD/SCID mouse, 6 weeks posttransplantation, stained with H&E (Figure 15a), periodic acid-Schiff (PAS; Figure 15b), anti porcine albumin antibody (Figure 15c), and anti-Ki67 antibody (Figure 15d). Note lobular patterns of hepatocyte arrangement in Figures 15a-c. Functionality of growing liver is suggested by the staining for PAS and for albumin (Figures 15b and 15c, respectively). Original magnification of photomicrographs in Figures 15a-c: ×10. In Figure 15d, positive staining of hepatocyte nuclei (arrows) with anti Ki67 antibody demonstrates proliferation of graft-derived hepatocytes.

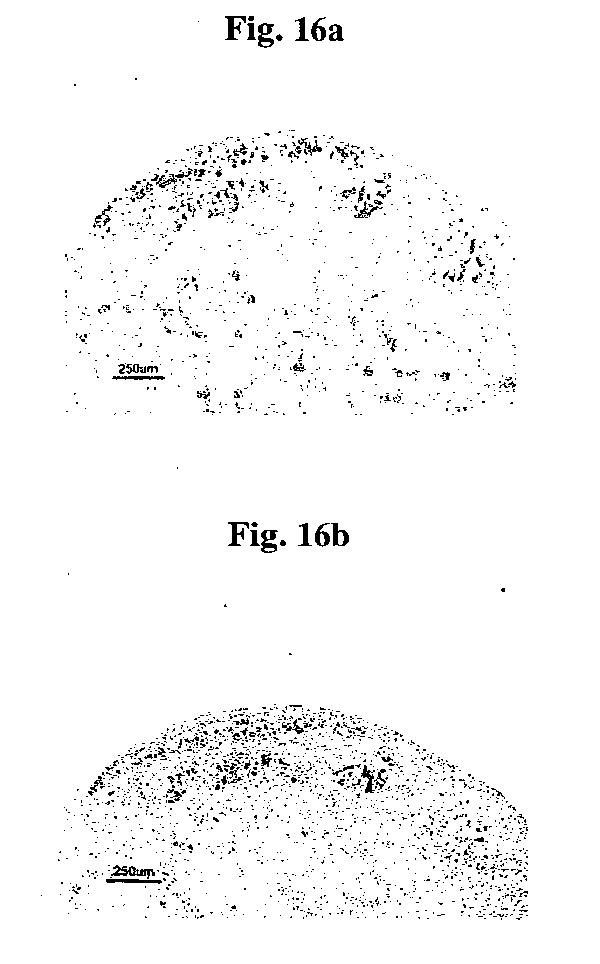

FIG. 16a-b are photomicrographs depicting hepatic differentiation in histology sections of a 28-day gestational stage porcine liver-derived graft transplanted under the renal capsule of an NOD/SCID mouse, 6 weeks posttransplantation. The sections were stained with PAS, and anti porcine albumin antibody (Figure 16a and 16b, respectively). Original magnification: ×4. Functional activity of transplanted liver is demonstrated by glycogen (PAS positivity) and albumin synthesis.

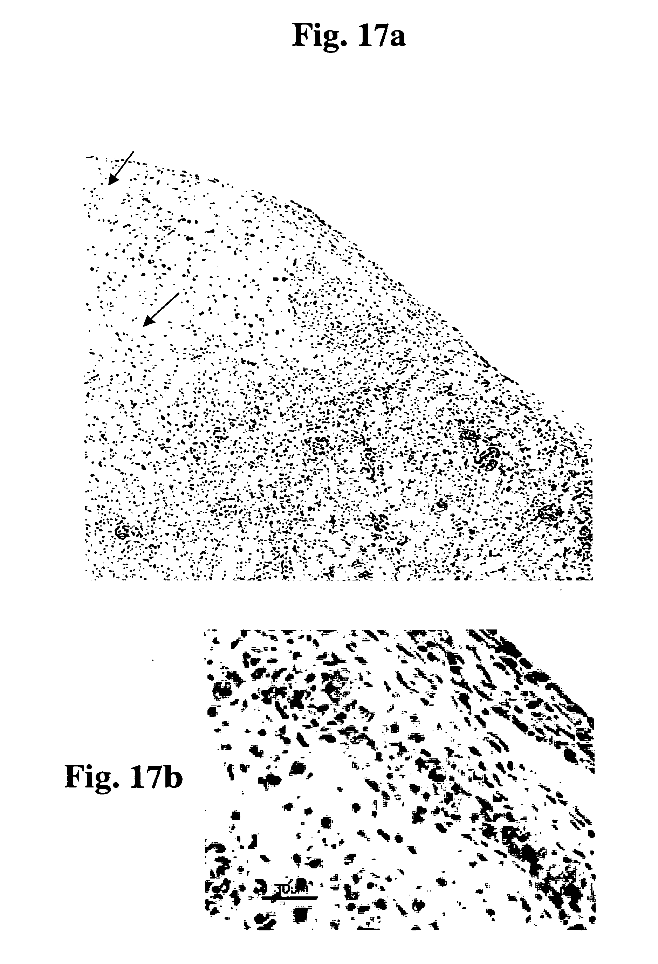

FIGS. 17a-b are photomicrographs depicting hepatic differentiation in a histology section of a 7-week gestational stage human liver-derived graft transplanted under the renal capsule of an NOD/SCID mouse, 6 weeks posttransplantation. Figure 17a depicts H&E staining, original magnification ×4, note bile duct differentiation (arrows). Figure 17b depicts PAS staining, original magnification ×40, note presence of differentiated, glycogen-stored hepatocytes.

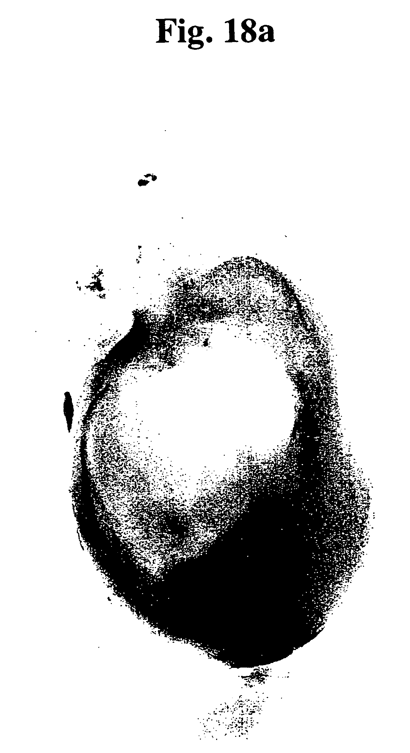

FIG. 18a is a stereomicrograph depicting pancreatic growth of a whole 28-day gestational stage porcine pancreas graft transplanted under the renal capsule of an NOD/SCID mouse, 5 weeks posttransplantation.

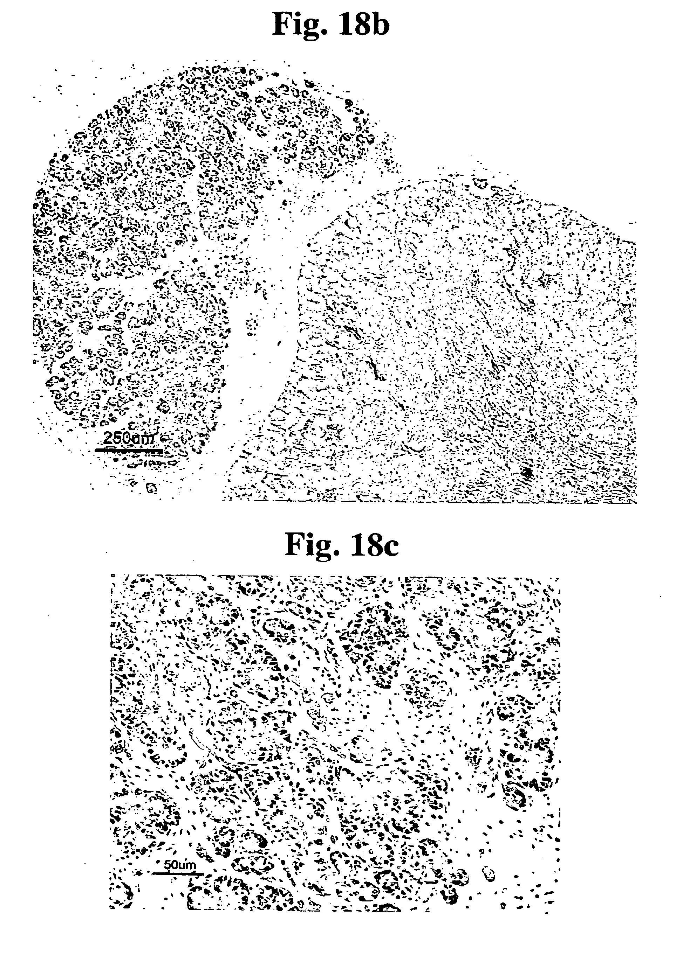

FIGs. 18b-c are photomicrographs depicting pancreatic tissue differentiation in a histology section of a 27-day gestational stage porcine pancreas-derived graft transplanted under the renal capsule of an NOD/SCID mouse, 6 weeks posttransplantation. Figures 18b and 18c depict photomicrographs of the section at low and high magnification, respectively. The section was stained with H&E, note differentiation of the pancreatic lobule with ductal and acinar pancreatic structures.

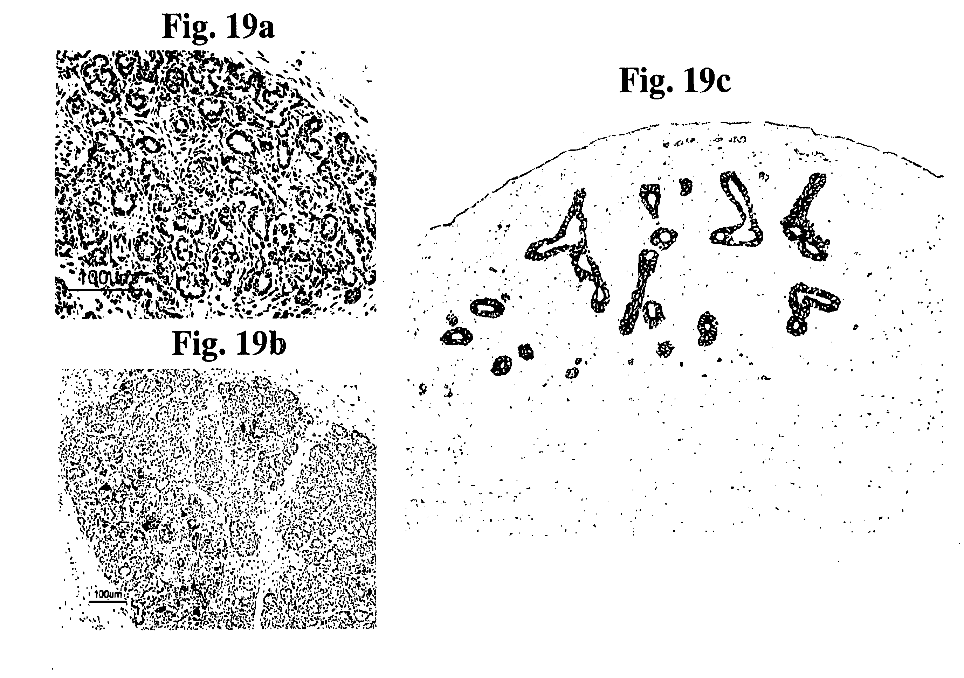

FIGs. 19a-c are photomicrographs depicting functional pancreatic differentiation in histology sections of gestational stage porcine pancreas-derived grafts transplanted under the renal capsule of NOD/SCID mice, 6 weeks posttransplantation. Sections stained with anti insulin antibody (Figure 19a), and anti pancreatic polypeptide (PP) antibody (Figure 19b) demonstrate insulin and PP synthesis, respectively, in a graft derived from 27-day gestational stage tissue. Figure 19c depicts a histology section of a 28-day gestational stage porcine pancreas-derived graft immunostained with anti cytokeratin antibody (the antibody is non-reactive with mouse epithelia). Note differentiation of graft derived pancreatic ductal epithelia.

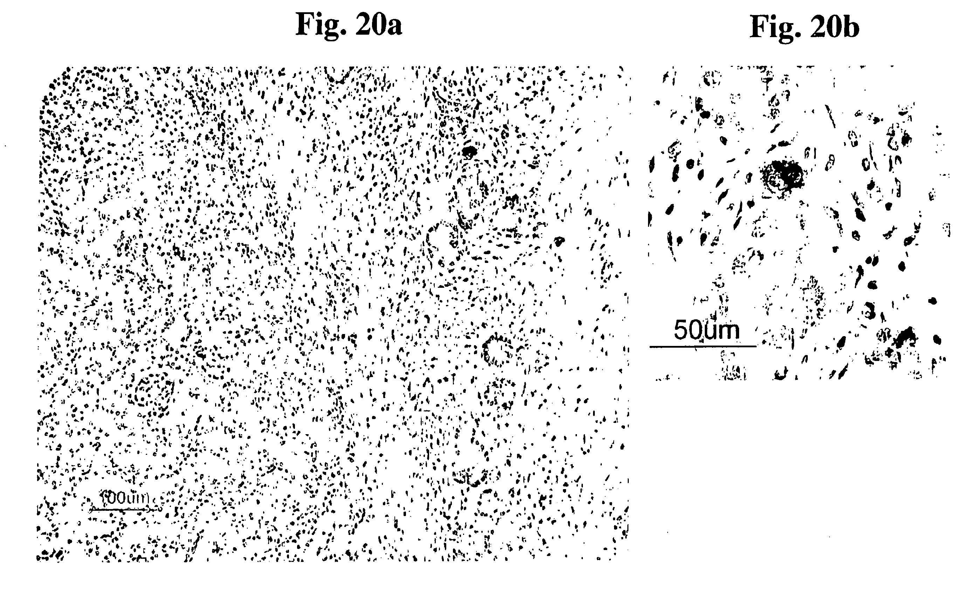

FIGs. 20a-b are photomicrographs depicting functional pancreatic differentiation in a section of an 8-week gestational stage human pancreas-derived graft transplanted under the renal capsule of an NOD/SCID mouse, 6 weeks posttransplantation. The section was immunostained with anti insulin antibody, note foci of insulin-positive beta-cells. Figure 20a is a photomicrograph taken at low magnification and Figure 20b is a photomicrograph taken at high magnification highlighting insulin expression within an islet of Langerhans.

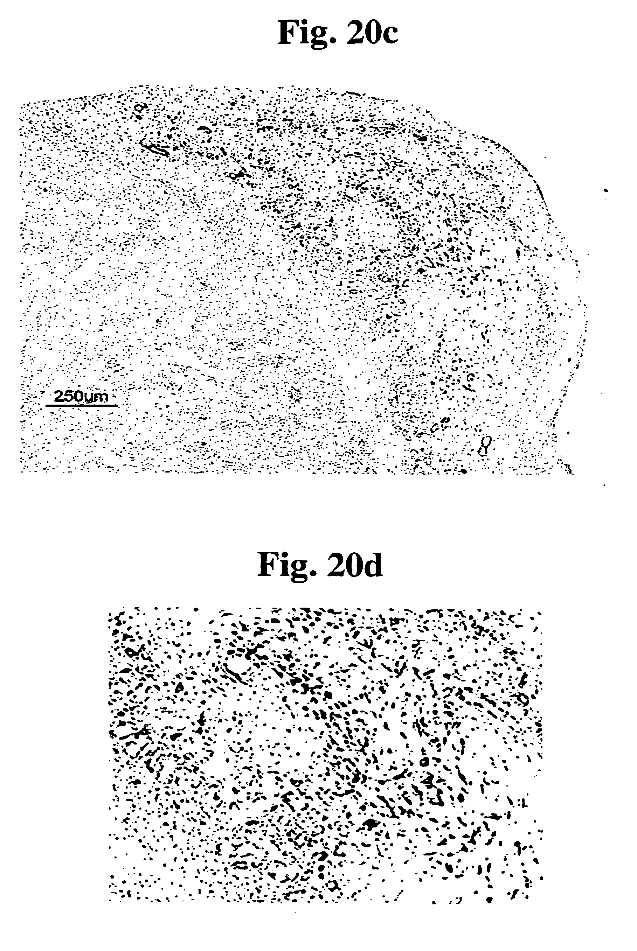

FIGs. 20c-d are photomicrographs depicting pancreatic differentiation in a histology section of an 8-week gestational stage human pancreas-derived graft transplanted under the renal capsule of an NOD/SCID mouse, 6 weeks posttransplantation. The section was immunostained with anti vimentin antibody (non-reactive with mouse tissues), note differentiation of mesenchymal cells of human origin in the graft (Figure 20d). Figures 20c and 20d are photomicrographs of the stained section taken at low and high original magnification, respectively.

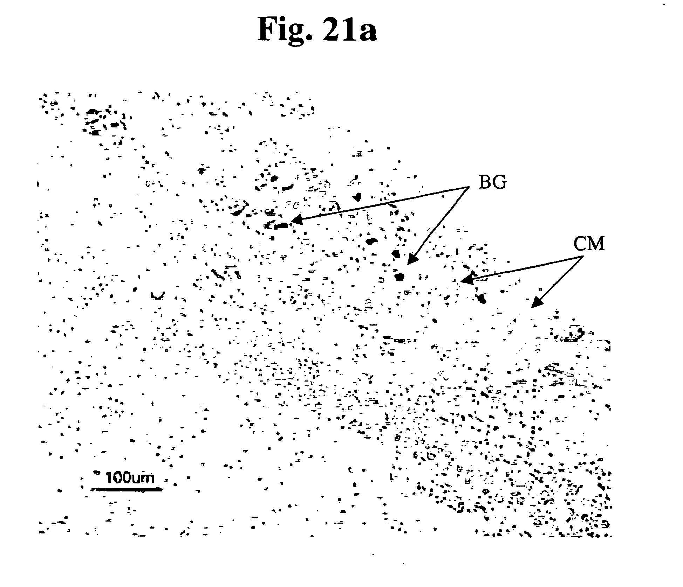

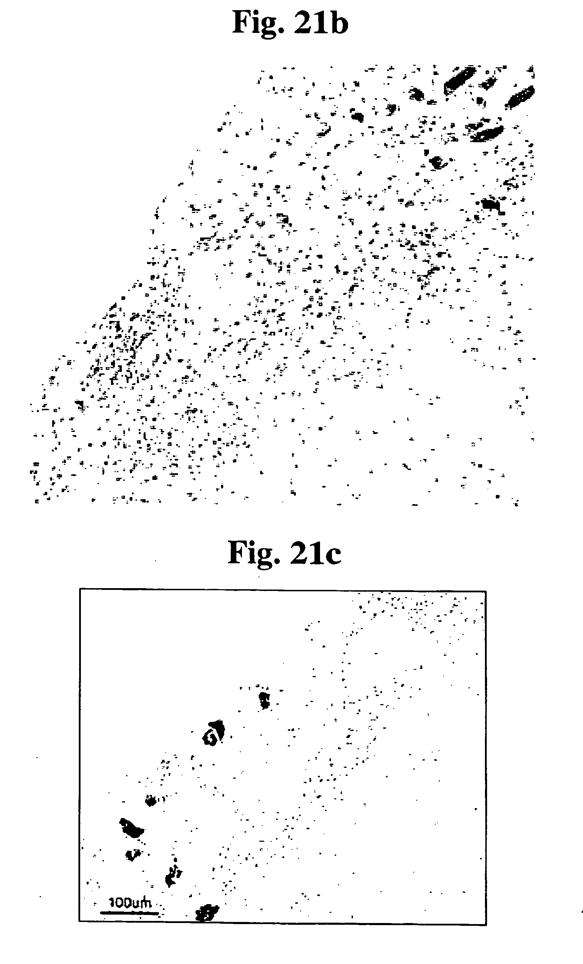

FIGs. 21a-c are photomicrographs depicting cardiac differentiation in histology sections of a 9-week gestational stage human heart-derived graft transplanted under the renal capsule of an NOD/SCID mouse, 6 weeks posttransplantation. Figure 21a depicts an H&E-stained section, note that the transplant contains two distinct types of cardiac-specific cellular components: cardiomyocyiic ("CM") and basal ganglionic ("BG") structures. Figures 21b and 21c depict sections immunostained with anti alpha-sarcomeric actin antibody and anti-neurofilament protein antibody, respectively. Note groups of cardiomyocytic cells identified by alpha-sarcomeric actin positivity, and basal ganglionic cells by neurofilament protein positivity.

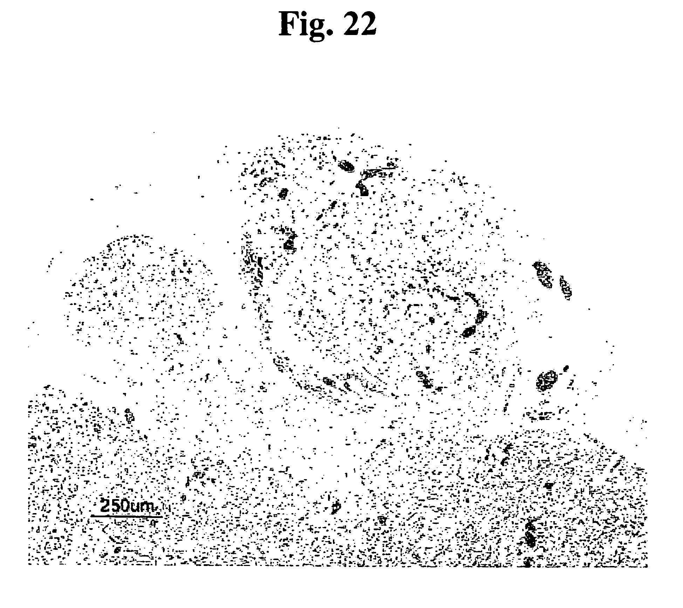

FIG. 22 is a photomicrograph depicting splenic differentiation in an H&E-stained histology section of a 28-day gestational stage porcine spleen-derived graft transplanted under the renal capsule of an NOD/SCID mouse, 6 weeks posttransplantation. Note the well vascularized mesenchymal tissue differentiation. The photomicrograph was taken at ×4 original magnification.

### DESCRIPTION OF THE PREFERRED EMBODIMENTS

The present invention is of methods of treating diseases by transplantation of developing or non syngeneic organs/tissues, and of methods of evaluating the transplantation suitability of grafts. Specifically, the present invention relates to transplantation of 27- to 28-day gestational stage porcine organ or tissue grafts to treat diseases in humans, such as renal, pancreatic, hepatic, cardiac, genetic and/or hematological diseases. Porcine grafts at such gestational stages has the capacity to generate, in the absence of graft-derived teratomas, structurally and functionally differentiated, host vascularized organs/tissues optimally tolerated by alloreactive/xenoreactive human lymphocytes, without or with minimal host immunosuppression. In particular, such grafts, when derived from renal organs/tissues have the capacity to generate host vascularized, urine producing renal organs; when derived from pancreatic organs/tissues have the capacity to generate pancreatic islets comprising insulin-producing beta-cells; when derived from hepatic organs/tissues have the capacity to generate structurally and functionally differentiated hepatic cells and tissues. Such porcine grafts, when derived from lymphoid organs/tissues, have the capacity to differentiate into well differentiated and vascularized lymphoid mesenchymal/stromal tissues. As such, transplantation of such gestational stage porcine grafts can be used to treat human subjects having a disease which is amenable to therapeutic cell, tissue and/or organ transplantation, such as a renal, pancreatic, hepatic, cardiac, genetic and/or hematological disease, without or with minimal immunosuppression of graft recipients.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

Organ or tissue transplantation is the optimal or sole therapy for numerous devastating and lethal diseases, such as renal, pancreatic, hepatic, cardiac, hematological and/or genetic diseases. However, current methods of transplantation are severely hampered by inadequate sources of suitable donor organs/tissues, and by the requirement for permanent and harmful immunosuppressive treatment of graft recipients to prevent graft rejection. Strategies suggested for overcoming these obstacles involve using xenogeneic organ or tissue grafts, which are available in sufficient quantities, and/or developing organ or tissue grafts which have been shown to be better tolerated by mismatched recipients than fully differentiated organ or tissue grafts.

Various approaches for utilizing transplantation of developing and/or non syngeneic organs/tissues to treat human disease have been attempted in the prior art.

For example, transplantation of gestational stage renal organs/tissues has been attempted into non immunosuppressed allogeneic recipients, xenogeneic recipients treated with CTLA4-Ig blockade of costimulation, or immunodeficient xenogeneic recipients reconstituted with human PBMCs. Transplantation of gestational stage pancreatic organs/tissues has been attempted via transplantation of islet cells, pancreases, or cultured gestational stage pancreatic islets, into xenogeneic immunodeficient recipients, or transplantation of porcine fetal islet cell clusters into diabetic human recipients. Another approach has attempted striatal transplantation of allogeneic fetal ventral mesencephalic tissue into human recipients with Parkinson's disease.

However, all prior art approaches employing transplantation of developing, non syngeneic organ or tissue grafts, such as porcine grafts, into a recipient have failed to provide a method of generating in a recipient graft derived organs/tissues which: (i) are optimally structurally/functionally differentiated; (ii) are fully/optimally tolerated by alloreactive/xenoreactive human lymphocytes in a recipient without or with minimal graft recipient immunosuppression (iii) are optimally host vascularized; and (iv) can be generated in the absence of graft-derived teratomas.

In particular, prior art approaches have failed to provide a method of generating in a recipient graft derived porcine renal organs/tissues which display optimal structural/functional differentiation, including urine production, and which can be generated in the absence of graft-derived teratomas and which are optimally tolerated by alloreactive/xenoreactive human lymphocytes in the recipient without or with minimal recipient immunosuppression.

Moreover, prior approaches have failed to provide a method of generating in a recipient graft-derived porcine pancreatic organs/tissues including pancreatic islets and insulin-producing beta-cells, which can be generated in the absence of teratomas, and which will be optimally tolerated by alloreactive/xenoreactive human lymphocytes in the recipient.

Furthermore, prior art approaches have failed to provide a method of generating in a recipient graft-derived porcine structurally and functionally differentiated hepatic cells/tissues, which can be generated in the absence of graft-derived teratomas, and which will be optimally tolerated by alloreactive/xenoreactive human lymphocytes in the recipient.

Additionally, prior art approaches have failed to provide a method of generating in a recipient graft-derived proliferative cardiac cells/tissues which can be generated in the absence of graft-derived teratomas, and which will be optimally tolerated by alloreactive human lymphocytes in the recipient.

As well, prior art approaches have failed to provide a method of generating in a recipient graft-derived well differentiated and vascularized porcine lymphoid tissues which can be generated in the absence of graft-derived teratomas, and which will be optimally tolerated by xenoreactive human lymphocytes.

While reducing the present invention to practice, the existence of specific gestational stages was unexpectedly uncovered during which organs/tissues do not substantially display/express specific lymphocyte coreceptors or ligands thereof, and during which organs/tissues can be transplanted into a recipient so as to generate, in the absence of graft-derived teratomas, cells, organs and tissues which display optimal structural and functional differentiation, such as, in the case of renal grafts, urine production; and which are optimally tolerated by alloreactive/xenoreactive human lymphocytes in the recipient, without or with minimal recipient immunosuppression.

In particular, while reducing the present invention to practice, specific gestational stages were uncovered and defined during which porcine renal grafts can be transplanted into a recipient so as to generate, in the absence of graft-derived teratoma formation, optimally structurally and functionally differentiated urine-producing renal organs and tissues which are optimally tolerated by alloreactive/xenoreactive human lymphocytes in the recipient in the absence of, or with minimal recipient immunosuppression.

Furthermore, while reducing the present invention to practice, specific gestational stages were uncovered and defined duting which porcine hepatic grafts can be transplanted into a recipient so as to generate, in the absence of graft-derived teratoma formation, optimally structurally and functionally differentiated hepatic organs/tissues which will be optimally tolerated by alloreactive/xenoreactive human lymphocytes.

Moreover, while reducing the present invention to practice, specific gestational stages were uncovered and defined during which porcine pancreatic grafts can be transplanted into a recipient so as to generate, in the absence of graft-derived teratoma formation, structurally and functionally differentiated pancreatic organs/tissues including pancreatic islets and insulin producing beta-cells which will be optimally tolerated by alloreactive/xenoreactive human lymphocytes.

Additionally, while reducing the present invention to practice, specific gestational stages were uncovered and defined during which cardiac grafts can be transplanted into a recipient so as to generate, in the absence of graft-derived teratoma formation, differentiated and proliferative cardiac cells/tissues which will be optimally tolerated by alloreactive human lymphocytes.

As well, while reducing the present invention to practice, specific gestational stages were uncovered and defined during which porcine lymphoid grafts can be transplanted into a recipient so as to generate, in the absence of teratoma formation, well-differentiated and vascularized lymphoid mesenchymal/stromal cells/tissues which will be tolerated by xenoreactive human lymphocytes.

Thus, transplantation of porcine organ-/tissue-derived grafts at the aforementioned gestational stages can be used to structurally/functionally replace/repair organs/tissues displaying pathological physiology/morphology in recipients of such grafts, and hence can be used to treat diseases associated with such organs/tissues displaying such pathological physiology/morphology, without any, or with minimal recipient immunosuppression.

Thus, according to one aspect of the present invention there is provided a method of treating a disorder associated with pathological organ or tissue physiology or morphology.

The method is effected by transplanting into a subject in need thereof a therapeutically effective mammalian organ or tissue graft selected: (i) not substantially expressing or presenting a molecule capable of stimulating or enhancing an immune response in the subject; (ii) at a predetermined stage of differentiation sufficiently advanced so as to be capable of generating structurally and functionally differentiated organs/tissues of essentially a single type in the subject, preferably in the absence of graft-derived teratoma formation, but sufficiently early so as to enable the graft to be optimally tolerated by non syngeneic lymphocytes; or (iii) preferably both.

Depending on the transplantation context, in order to facilitate engraftment of the graft, the method may further advantageously comprise treating the subject with an immunosuppressive regimen prior to, concomitantly with, or following transplantation of the graft.

As used herein, "treating" the disorder includes curing, alleviating, or stabilizing the disorder, or inhibiting future onset or development of the disorder.

As used herein, the term "disorder" refers to any disease, or to any pathological or undesired condition, state, or syndrome, or to any physical, morphological or physiological abnormality.

As used herein, the phrase "therapeutically effective graft" refers to a graft having structural and/or functional characteristics such that transplantation thereof into the subject serves to treat the disorder.

Methods of selecting a graft not substantially expressing or presenting the molecule, or at the predetermined stage of differentiation are described further hereinbelow.

Transplanting the graft may be effected in numerous ways, depending on various parameters, such as, for example, the type, stage or severity of the disorder, the physical or physiological parameters specific to the individual subject, and/or the desired therapeutic outcome. For example, depending on the application and purpose, transplanting the graft may be effected using a graft originating from any of various mammalian species and/or organ or tissue type, by implanting the graft into various anatomical locations of the subject, using a graft consisting of a whole or partial organ or tissue, and/or by using a graft consisting of various numbers of discrete organs, tissues, and/or portions thereof.

One of ordinary skill in the art, such as a physician, in particular a transplant surgeon specialized in the disorder, would possess the expertise required for selecting and transplanting a suitable graft so as to treat the disorder according to the teachings of the present invention.

Depending on the application and purpose the method may be effected using a syngeneic, allogeneic or xenogeneic graft derived from essentially any mammalian species.

As used herein, a "syngeneic" graft refers to a graft which is essentially genetically identical with the subject or essentially all lymphocytes of the subject.

Examples of syngeneic grafts include a graft derived from the subject (also referred to in the art as an "autologous graft"), a clone of the subject, or an identical twin of the subject.

As used herein, a "non syngeneic" graft refers to an allogeneic graft or a xenogeneic graft.

As used herein, an "allogeneic graft" refers to a graft derived from a donor non syngeneic with the subject or non syngeneic with a substantial proportion of the lymphocytes present in the subject, where the donor is of the same species as the subject or of the same species as substantially all of the lymphocytes of the subject.

Typically, non clonal/non twin mammals of the same species are allogeneic relative to each other.

As used herein, a "xenogeneic graft" refers to a graft derived from a donor non syngeneic with the subject or non syngeneic with a substantial proportion of the lymphocytes present in the subject, where the donor is of a different species as the subject or of a different species as a substantial proportion of the lymphocytes present in the subject.

Typically, mammals of different species are xenogeneic relative to each other.

As is described and illustrated in the Examples section below, transplanting a animal graft of the present invention into a recipient can be used to generate optimally structurally and functionally differentiated organs and tissues, in the absence of teratoma formation, which are optimally tolerated by alloreactive/xenoreactive human lymphocytes in the recipient without or with minimal recipient immunosuppression.

As used herein, an "optimally tolerated" graft is a graft not rejected or not substantially infiltrated in the subject by T lymphocytes non syngeneic with the graft.

As used herein, a graft which is "rejected" is a graft which causes what is commonly known in the art as hyperacute rejection, acute rejection, or chronic rejection. Ample guidance for ascertaining graft rejection is provided in the literature of the art (for example, refer to: Kirkpatrick CH. and Rowlands DT Jr., 1992. JAMA. 268, 2952; Higgins RM. et al., 1996. Lancet 348, 1208; Suthanthiran M. and Strom TB., 1996. New Engl. J. Med. 331, 365; Midthun DE. et al., 1997. Mayo Clin Proc. 72, 175; Morrison VA. et al., 1994. Am J Med. 97, 14; Hanto DW., 1995. Annu Rev Med. 46, 381; Senderowicz AM. et al., 1997. Ann Intern Med. 126, 882; Vincenti F. et al., 1998. New Engl. J. Med. 338, 161; Dantal J. et al. 1998. Lancet 351, 623). Infiltration of a graft by T lymphocytes of a graft recipient typically correlates with graft rejection.

As used herein, a "minimal immunosuppressive treatment" of the subject refers to an immunosuppressive treatment of the subject restricted to administration of a drug capable of blocking an interaction between a lymphocyte coreceptor and a cognate ligand thereof, or to an immunosuppressive treatment of the subject applied during a single period of 20 days or less.

As described hereinabove, the graft may be derived from various mammalian species.

Depending on the application and purpose, the graft is preferably a porcine graft.

As used herein, the phrase "alloreactive lymphocytes" refers to lymphocytes substantially capable of rejecting an essentially fully differentiated allogeneic graft.

While reducing the present invention to practice, the gestational stages of porcine organs/tissues during which these are at a suitable predetermined stage of differentiation for practicing the method were identified, as described in the Examples section below.

Preferably, the porcine graft is selected at a stage of differentiation corresponding to 20 to 63 days of gestation, more preferably 20 to 56 days of gestation, more preferably 20 to 42 days of gestation, more preferably 20 to 35 days of gestation, more preferably 20 to 28 days of gestation, more preferably 24 to 28 days of gestation, and most preferably 27 to 28 days of gestation.

Alternately, the porcine graft may be advantageously selected at a stage of differentiation corresponding to 22 to 33 days of gestation, 23 to 32 days of gestation, 24 to 31 days of gestation, 25 to 30 days of gestation, 26 to 29 days of gestation, 22 to 63 days of gestation, 22 to 56 days of gestation, 22 to 42 days of gestation, 22 to 35 days of gestation, 22 to 34 days of gestation, 22 to 32 days of gestation, 22 to 31 days of gestation, 22 to 30 days of gestation, 22 to 29 days of gestation, 22 to 28 days of gestation, or 22 to 27 days of gestation.

In order to avoid teratoma formation, the porcine graft is preferably selected at at a stage of differentiation corresponding to at least 22 days of gestation since, as shown in Example 6 of the Examples section which follows, a porcine graft at a stage of differentiation corresponding to a gestational stage as early as 21 days risks causing teratomas when transplanted into a host.

As is described and shown in the Examples section which follows, transplantation into a recipient of porcine grafts at 27 to 28 days of gestation can be used to generate optimally functionally and structurally differentiated graft-derived organs and tissues, in the absence of graft-derived teratoma-formation, which are optimally tolerated by xenoreactive human lymphocytes in the recipient, without or with minimal recipient immunosuppression.

As used herein, the phrase "xenoreactive lymphocytes" refers to lymphocytes substantially capable of rejecting an adult stage xenogeneic graft.

While reducing the method of the present invention to practice, the universal applicability of the method with respect to different cell/organ/tissue types was demonstrated using renal, hepatic, pancreatic, and lymphoid grafts of porcine origin.

As is described and shown in Example I of the Examples section which follows, transplantation into a recipient of a porcine renal graft of the present invention selected at a stage of differentiation corresponding to 27 to 28 days of gestation can be used to generate, in the absence of graft-derived teratomas, optimally functionally and structurally differentiated graft-derived renal organs and tissues which are capable of producing urine, and which are optimally tolerated by xenoreactive human lymphocytes in the recipient, without or with minimal recipient immunosuppression.

As is described and shown in Example 6 of the Examples section which follows, transplantation into a recipient of a porcine hepatic graft of the present invention selected at a stage of differentiation corresponding to 28 days of gestation can be used to generate functionally and structurally differentiated graft-derived hepatic organs/tissues, in the absence of graft-derived teratoma formation, which will be optimally tolerated by xenoreactive human lymphocytes in the recipient, without or with minimal recipient immunosuppression.

As is described and shown in Example 7 of the Examples section which follows, transplantation into a recipient of a porcine pancreatic graft of the present invention selected at a stage of differentiation corresponding to 27-28 days of gestation can be used to generate functionally and structurally differentiated graft-derived pancreatic organs/tissues, including pancreatic islets and insulin-producing beta-cells, in the absence of graft-derived teratoma formation, which will be optimally tolerated by xenoreactive human lymphocytes in the recipient, without or with minimal recipient immunosuppression.

As is described and shown in Example 9 of the Examples section which follows, transplantation into a recipient of a porcine lymphoid organ/tissue graft of the present invention selected at a stage of differentiation corresponding to 28 days of gestation can be used to generate, in the absence of graft-derived teratoma formation, well differentiated and vascularized graft-derived lymphoid mesenchymal/stromal cells/tissues which will be tolerated by xenoreactive human lymphocytes in the recipient.

While reducing the present invention to practice, it was unexpectedly uncovered that the structurally and functionally differentiated organs/tissues generated by the grafts display predominantly subject derived vasculature (for further detail please see the Examples section which follows). Without being bound to a paradigm, the present inventors are of the opinion that porcine grafts at 27 to 28 days of gestation, respectively, or grafts derived from other species at equivalent stages of differentiation, optimally engraft in the subject due to their capacity to generate organs/tissues having such predominantly subject-derived vasculature. In support of this view, it has been suggested in the art that the extent of host derived vasculature in a transplanted graft is correlated with tolerance of such a graft.

The discovery that transplanting porcine organs/tissues at stages of differentiation corresponding to such early gestational stages can be used to generate in a recipient optimally structurally and functionally differentiated graft-derived organs and tissues of graft lineage which are optimally tolerated by alloreactive/xenoreactive human lymphocytes in the recipient, without or with minimal recipient immunosuppression, in the absence of graft derived teratoma formation, was unexpected since: (i) the prior art teaches that transplanting organs/tissues at more advanced stages of differentiation is optimal for such application (for example, refer to Otonkoski T. et al., 1999. Transplantation 68, 1674); and (ii) since the earliest - and hence least immunogenic - gestational stages of grafts during which these would be able to generate optimally differentiated graft-derived organs/tissues in the absence of graft-derived teratomas was unknown.

Grafts from numerous species, other than pig, at optimal stages of differentiation corresponding to the aforementioned porcine optimal gestational stages may also be employed for practicing the method of the present invention. Animals such as the major domesticated or livestock animals, and primates, which have been extensively characterized with respect to correlation of stage of differentiation with gestational stage may be suitable for practicing the method. Such animals include bovines (e.g., cow), equids (e.g., horse), ovids (e.g., goat, sheep), felines (e.g., *Felis domestica*), canines (e.g., *Canis domestica*), rodents (e.g., mouse, rat, rabbit, guinea pig, gerbil, hamster), and primates (e.g., chimpanzee, rhesus monkey, macaque monkey, marmoset).

Various methods may be employed to obtain a graft at a stage of differentiation corresponding to a specific gestational period.

Obtaining such a graft is optimally effected by harvesting the graft from a developing graft donor embryo or fetus at such a stage of gestation.

It will be understood by one ordinarily versed in the art that the gestational stage of an organism is the time period elapsed following fertilization of the oocyte generating the organism.

Alternately, a graft at a stage of differentiation corresponding to a specific gestational stage may be obtained by *in-vitro* culture of cells, organs/tissues being at an earlier stage of differentiation than the graft, such as organ specific precursor cells, so as to generate a graft at a desired stage of differentiation. Such controlled *in-vitro* differentiation of cells, tissues or organs is routinely performed, for example, using culturing of embryonic stem cell lines to generate cultures containing cells/tissues/organs of desired lineages. For example, for generation of various lineages, including endodermal lineages such as liver; ectodermal lineages such as brain, skin and adrenal; and mesodermal lineages such as muscle, cartilage, mullerian duct, and heart, refer, for example, to: Schuldiner M. et al., 2000. Proc Natl Acad Sci USA. 97:11307-11312 and Itskovitz-Eldor J. et al., 2000. Mol Med 6:88; for pancreatic differentiation of embryonic stem cells, refer, for example, to: Lee S.H., et al., 2000. Nature Biotechnol. 18:675; Lumelsky et al., 2001. Science 292:1389-1394; Soria et al., 2000. Diabetes 49:1-6; Schuldiner M. et al., 2000. Proc Natl Acad Sci U S A. 97:11307-11312). For differentiation of pulmonary lineages, refer for example, to Otto WR., 1997. Int J Exp Pathol. 78:291-310.

In order to optimally treat the disorder, transplanting the graft is preferably effected in such a way as to therapeutically replace or repair the organ or tissue displaying pathological physiology or morphology associated with the disorder.

Hence, the graft is preferably selected of an organ or tissue type corresponding to that of the organ or tissue with pathological physiology or morphology. For example, for treatment of a renal, pancreatic, hepatic, or cardiac graft, respectively. For example, for treatment of a hematological and/or genetic disorder, the graft is preferably a lymphoid graft. Alternately, for treatment of a hematological and/or genetic disorder, the lymphoid graft may be derived from any other suitable lymphoid tissue, depending on the application and purpose, such as lymph node, Peyer's patches, thymus or bone marrow.

As is described in the Examples section below, transplantation of a graft selected of an organ or tissue type corresponding to that of the organ or tissue exhibiting pathological physiology or morphology associated with the disorder according to the protocol set forth therein can be used to treat the disorder.

As described hereinabove, transplanting the graft may be effected by transplantation thereof into various anatomical locations. Preferably, the graft is transplanted into an anatomical location where it will be of optimal therapeutic effect.

Depending on the application and purpose, the graft may be transplanted into a homotopic anatomical location (a normal anatomical location for the organ or tissue type of the graft), or into an ectopic anatomical location (an abnormal anatomical location for the organ or tissue type of the graft). Optionally, when transplanting the graft, the organ or tissue displaying pathological physiology or morphology may be removed, for example, so as to enable growth and engraftment of the graft, for example in the context of organ replacement by transplantation of the graft into a homotopic anatomical location.

As used herein, a "homotopic anatomical location" of a graft whose organ or tissue type exists in the form of multiple discrete homologs (e.g., right and left kidneys, different fingers on the same hand, etc.) includes the anatomical location of any such homolog.

Depending on the application and purpose, the graft may be advantageously implanted under the renal capsule, or into the kidney, the testicular fat, the sub cutis, the omentum, the portal vein, the liver, the spleen, the heart cavity, the heart, the pancreas and/or the intra abdominal space.

Preferably, transplanting a renal graft of the present invention is effected by transplanting the graft into the intra abdominal space, or, more preferably in the renal capsule. Preferably transplantation into the renal capsule is effected by subcapsular transplantation. Subcapsular transplantation advantageously enables insertion of a catheter requiring only a short extension to the skin where urine can be drained from the renal graft. Intra abdominal transplantation advantageously enables the developing ureter or the renal pelvis of the renal tissue transplant to be anastomosed to the host's excretory system. As is shown in Example I of the Examples section below, the method may be practiced by transplanting a renal graft of the present invention under the renal capsule of a recipient. Alternately, transplanting the renal graft may be effected by transplanting the graft into the portal vein, the liver, the spleen, the testicular fat, the sub-cutis, or the omentum.

Transplanting a hepatic graft of the present invention may be advantageously effected by transplanting the graft into the portal vein, the liver, the renal capsule, the testicular fat, the sub-cutis, the omentum, the spleen, and/or the intra-abdominal space. Preferably, transplanting a hepatic graft of the present invention is effected by transplanting the graft under the renal capsule or into the spleen. As is shown in Example 6 of the Examples section below, the method may be practiced by transplanting a hepatic graft of the present invention under the renal capsule or into the spleen.

Transplanting a pancreatic graft of the present invention may be advantageously effected by transplanting the graft into the portal vein, the liver, the pancreas, the testicular fat, the sub-cutis, the omentum and/or the intra-abdominal space. Preferably, transplanting a pancreatic graft of the present invention is effected by transplanting the graft under the renal capsule. Preferably, for transplanting a pancreatic graft into the portal vein, the pancreatic graft is a pancreatic islet graft. As is shown in Example 7 of the Examples section below, the method may be practiced by transplanting a pancreatic graft of the present invention under the renal capsule. Guidance for practicing therapeutic transplantation of pancreatic grafts according to the teachings of the present invention is provided in Example 5 of the Examples section below.

Transplanting a cardiac graft of the present invention may be advantageously effected, depending on the application and purpose, by transplanting the graft into the heart cavity, the heart, the myocardium and the intra-abdominal space. As is shown in Example 8 of the Examples section below, the method may be practiced by transplanting a cardiac graft of the present invention under the renal capsule.

Preferably, for treating a cardiac disorder associated with myocardial ischemia, for example due to a cardiac infarct, the cardiac graft is administered to the infarct and/or to the border area of the infarct. As one skilled in the art would be aware, the infarcted area is grossly visible, allowing such specific localization of application of therapeutic grafts to be possible. The precise determination and timing of an effective dose in this particular case may depend, for example, on the size of an infarct, and the time elapsed following onset of myocardial ischemia. Ample guidance is provided in the art for therapeutic implanting a cardiac tissues, such as a cardiac graft of the present invention, into damaged myocardium according to the teachings of the present invention (refer, for example, to: Strauer et al., 2001. Dtsch Med Wochenschr. 126:932; Strauer et al., 2002. Circulation 106:1913; Stamin et al., 2003. Lancet 361:45; Perin et al., 2003. Circulation 107:2294; Assmus et al., 2002. Circulation 106:3009; Britten et al., 2003. Circulation 108:2212; and U.S. Patent No.: 5,733,727, 6,395,016 and 6,592,623).

Transplanting a lymphoid graft of the present invention, such as a splenic graft of the present invention, may be advantageously effected, depending on the application and purpose, by transplanting the graft into the portal vein, the liver, the renal capsule, the sub-cutis, the omentum, the spleen, and the intra-abdominal space. As is shown in Example 9 of the Examples section below, the method may be practiced by transplanting a lymphoid graft of the present invention under the renal capsule.

As described hereinabove, depending on the application and purpose, transplanting the graft may be effected by transplanting a graft consisting of a whole or partial organ. The method may be advantageously effected by transplanting a graft consisting of a partial organ for organ grafts at a stage of differentiation corresponding to that of a 5-week or older gestational stage porcine organ.

As is shown in Example 1 of the Examples section below, transplanting such partial organ grafts at stages of differentiation corresponding to such gestational stages leads to significantly improved engraftment and/or functional and structural differentiation of the graft relative to transplanting a complete organ graft.

As described hereinabove, depending on the application and purpose, transplanting the graft may be effected by transplanting a graft consisting of various numbers of discrete organs, tissues, and/or portions thereof.

For example, transplanting increasing numbers of discrete organ or tissue grafts may be advantageously employed to increase the physiological or physical therapeutic effect of the graft to desired levels. For example, increasing the number of discrete organs/tissues of an endocrine tissue graft (for example, a pancreatic islet graft) can be used to obtain sufficiently high graft derived hormone (for example, insulin) secretion levels so as to achieve a desired effect (for example, increased insulin secretion capacity). In the case of renal grafts, increasing the number of discrete renal organ or tissue derived grafts can be used to obtain sufficient numbers of renal organs so as to achieve, for example, a sufficiently high urine production capacity to alleviate or cure a kidney disorder in the subject.

As described hereinabove, the method of treating the disorder may advantageously comprise treating the subject with an immunosuppressive regimen, prior to, during or following transplantation of the graft.

Various types of immunosuppressive regimens may be used to immunosuppress the subject.

Examples of suitable types of immunoppressive regimens include administration of immunosuppressive drugs, tolerance inducing cell populations, and/or immunosuppressive irradiation.

Ample guidance for selecting and administering suitable immunosuppressive regimens for transplantation is provided in the literature of the art (for example, refer to: Kirkpatrick CH. and Rowlands DT Jr., 1992. JAMA. 268, 2952; Higgins RM. et al., 1996. Lancet 348, 1208; Suthanthiran M. and Strom TB., 1996. New Engl. J. Med. 331, 365; Midthun DE. et al., 1997. Mayo Clin Proc. 72, 175; Morrison VA. et al., 1994. Am J Med. 97, 14; Hanto DW., 1995. Annu Rev Med. 46, 381; Senderowicz AM. et al., 1997. Ann Intern Med. 126, 882; Vincenti F. et al., 1998. New Engl. J. Med. 338, 161; Dantal J. et al. 1998. Lancet 351, 623).

Preferably, the immunosuppressive regimen consists of administering an immunosuppressant drug to the subject.

Examples of suitable immunosuppressive drugs include, but are not limited to, CTLA4-Ig, anti CD40 antibodies, anti CD40 ligand antibodies, anti B7 antibodies, anti CD3 antibodies (for example, anti human CD3 antibody OKT3), methotrexate (MTX), rapamycin, prednisone, methyl prednisolone, azathioprene, cyclosporin A (CsA), tacrolimus, cyclophosphamide and fludarabin, mycophenolate mofetil, daclizumab [a humanized (IgG1 Fc) anti-IL2R alpha chain (CD25) antibody], and anti T cell antibodies conjugated to toxins (for example, cholera A chain, or *Pseudomonas* toxin).

Preferably, the immunosuppressant drug is capable of blocking binding of a lymphocyte coreceptor with a cognate lymphocyte coreceptor ligand thereof.

Examples of suitable drugs capable of blocking binding of a lymphocyte coreceptor with a cognate lymphocyte coreceptor ligand include, but are not limited to, CTL.A4-Ig, anti CD40 antibodies, anti CD40 ligand antibodies, anti B7-1 or -2 antibodies, and anti CD28 antibodies.

Such polypeptide drugs are particularly advantageous since these are, unlike commonly used immunosuppressant drugs like cyclosporin A, essentially non toxic and/or non carcinogenic, and by virtue of passively blocking cell surface receptor interactions, afford reversible and temporary immunosuppression of the subject.

Preferably the drug capable of blocking binding of the lymphocyte coreceptor with the cognate lymphocyte coreceptor ligand thereof is CTLA4-Ig. CTLA4-Ig is a genetically engineered fusion protein of human CTLA4 and the IgG₁ Fc domain. It prevents T-cell activation by binding to human B7, which costimulates T cells through CD28.

Ample guidance for administering immunosuppressant drugs such as CTLA4-Ig so as to facilitate immunosuppression of a transplant recipient is provided in the literature of the art (for example, refer to: Benhamou PY., 2002. Transplantation 73, S40; Najafian N, and Sayegh MH., 2000. Expert Opin Investig Drugs 9, 2147-57).

Preferably, administering the immunosuppressant drug to the subject is effected during a single time period of 1 to 20 days, more preferably 1 to 18 days, more preferably 1 to 16 days, and most preferably 1 to 14 days, as described in Example 1 of the Examples section which follows.

As is described and shown in Example 1 of the Examples section below, transplanting a non syngeneic graft, such as a xenogeneic graft, in conjunction with administration of CTLA4-Ig to a normal immunocompetent subject according to the protocol set forth therein can be used to generate structurally and functionally differentiated organs/tissues optimally tolerated by lymphocytes of the subject.

As such, the method of the present invention, is superior to all prior art methods treating disorders by transplantation of non syngeneic or developing organs/tissues since it may be satisfactorily performed by temporary administration of a blocker of lymphocyte coreceptor-lymphocyte coreceptor ligand interaction, instead of permanent administration of harmful immunosuppressive agents, such as cyclosporin A, the standard method employed in the prior art.

While the method may be practiced to treat the disorder in a subject of essentially any mammalian species, the method is preferably used to treat the disorder in a human subject.

The method can be used to treat essentially any disorder associated with pathological organ or tissue physiology or morphology which is amenable to treatment via transplantation.

Such disorders include renal, pancreatic, cardiac, hepatic, hematological, genetic, pulmonary, brain, gastrointestinal, muscular, endocrine, osseous, neural, metabolic, dermal, cosmetic, ophthalmological, and vascular disorders.

Preferably, the method is used to treat a renal, hepatic, pancreatic, cardiac, genetic and/or hematological disorder.

The method can be used to treat any of various disorders of such types.

Examples of renal disorders which can be treated using a renal graft of the present invention include acute kidney failure, acute nephritic syndrome, analgesic nephropathy, atheroembolic kidney disease, chronic kidney failure, chronic nephritis, congenital nephrotic syndrome, end-stage kidney disease, Goodpasture's syndrome, IgM mesangial proliferative glomerulonephritis, interstitial nephritis, kidney cancer, kidney damage, kidney infection, kidney injury, kidney stones, lupus nephritis, membranoproliferative glomerulonephritis I, membranoproliferative glomerulonephritis II, membranous nephropathy, necrotizing glomerulonephritis, nephroblastoma, nephrocalcinosis, nephrogenic diabetes insipidus, IgA-mediated nephropathy, nephrosis, nephrotic syndrome, polycystic kidney disease, post-streptococcal glomerulonephritis, reflux nephropathy, renal artery embolism, renal artery stenosis, renal papillary necrosis, renal tubular acidosis type I, renal tubular acidosis type II, renal underperfusion and renal vein thrombosis.

Examples of pancreatic disorders which can be treated using a pancreatic graft of the present invention include Type I or Type II diabetes.

Preferably the method is used to treat type I diabetes ("diabetes").

Examples of hepatic disorders which can be treated using a hepatic graft of the present invention include hepatitis C infection (Rosen HR., 2003, hepatic cirrhosis, primary sclerosing cholangitis (Crippin JS., 2002. Can J Gastroenterol. 16:700), hepatocellular carcinoma (Molmenti EP, Klintmalm GB., 2001. J Hepatobiliary Pancreat Surg. 8:427-34), alcoholic liver disease (Podevin P. et al., 2001. J Chir (Paris). 138:147), and hepatitis B (Samuel D., 2000. Acta Gastroenterol Belg. 63:197-9).

In the case of cardiac disorders, disorders which can be treated using a cardiac graft of the present invention include ischemic cardiac insufficiency (Pouzet B. et al., 2001. J Soc Biol. 195:47-9), ventricular arrhythmia (Olivari MT, Windle JR., 2000. J Heart Lung Transplant. 19:S38-42), heart failure (Koerner MM., 2000. Curr Opin Cardiol. 15:178-82), congenital heart defects (Speziali G. et al., 1998. Mayo Clin Proc. 73:923), and cardiac tumors (Michler RE, Goldstein DJ., 1997. Semin Oncol. 24:534-9).

It will be appreciated that since a lymphoid graft of the present invention can be used to generate well differentiated and vascularized graft-derived lymphoid mesenchymal/stromal cells/tissues, that transplantation of such grafts can be used to treat any of various hematological and/or genetic diseases, such as coagulation disorders/coagulation factor deficiencies such as hemophilia (Liu et al., 1994. Transpl Int. 7:201), and lysosomal storage diseases/enzyme deficiencies such as Gaucher disease (Groth CG. et al., Birth Defects Orig Artic Ser. 9:102-5).

It will be further appreciated that, by virtue of enabling generation of well-differentiated and vascularized graft-derived lymphoid stromal tissues, transplantation of a lymphoid graft of the present invention can be used to enable differentiation of host-derived lymphoid tissue, and hence can be used for treating in the host a disease associated with a defect in lymphoid stroma, such as a defect in lymphoid stroma resulting in impaired hematological cellular growth and/or differentiation.

By virtue of enabling generation of well-differentiated and vascularized lymphoid tissues, transplantation of a lymphoid graft of the present invention can be used for treating any of various immunity-associated disorders.

As used herein, the phrase "immunity-associated disorder" refers to any disease associated with an immunodeficiency, a pathogenic immune response, and/or a potentially therapeutic immune response.

It will be appreciated by one of ordinary skill in the art that by virtue of the capacity of lymphoid tissues to support the growth and differentiation of immune effector cells, such as B-cells, T-cells, natural killer (NK) cells, granulocytes, macrophages, as well as hematopoietic stem cells (HSCs), a lymphoid graft of the present invention, such as a splenic graft of the present invention, can provide immune effector functionality, corrective immunoregulation, and support differentiation of various hematopoietic lineages, and hence that transplantation of such a graft can be used to treat essentially any immunity-associated disorder.

Examples of immunodeficiency diseases which can be treated by transplantation of a lymphoid graft of the present invention include acquired immunodeficiency syndrome (AIDS) caused by human immunodeficiency virus (HIV), severe combined immunodeficiencies (SCID), such as adenosine deaminase (ADA) deficiency, and immunodeficiencies resulting from therapeutic myeloreduction/myeloablation, such as in the context of therapy of cancers, such as hematological malignancies. A lymphoid graft of the present invention will enable therapeutic immune reconstitution of an immunodeficient subject in such contexts. The ordinarily skilled artisan will possess the necessary expertise for treating an immunodeficiency disease by transplantation of a lymphoid organ/tissue graft of the present invention according to the teachings of the present invention, and will have ample guidance available for practicing this aspect of the method of the present invention in the literature of the art (refer, for example, to: Fischer A. et al., 1998. Springer Semin Immunopathol. 19:479-92; Kane L. et al., 2001. Arch Dis Child Fetal Neonatal Ed. 85:F110; Horwitz ME., 2000. Pediatr Clin North Am. 47:1371; Friedrich W., 1996. Ann Med. 28:115-9; Parkman R., 1993. Leukemia. 7:1100-2). It will be appreciated by the ordinarily skilled artisan this aspect of the method of the present invention will serve to treat any of various infectious diseases in a subject whose own immune system does not mount adequate protective immune responses. Such infectious diseases include those caused by microbial pathogens, such as viruses, bacteria, mycoplasmas, protozoans, fungi, and the like.

Examples of diseases associated with a potentially therapeutic immune response also include malignancies. The ordinarily skilled artisan will possess the necessary expertise for treating a hematological and/or genetic disease by transplantation of a lymphoid organ/tissue graft of the present invention according to the teachings of the present invention, and will have ample guidance available for practicing this aspect of the method of the present invention in the literature of the art (refer, for example, to: Toungouz M, Goldman M. et al., 2001. Adv Nephrol Necker Hosp. 31:257-72; Parkman R., 1993. Leukemia. 7:1100-2; Porter DL., 2001. J Hematother Stem Cell Res. 10:465-80).

Examples of diseases associated with pathogenic immune responses include autoimmune diseases. By virtue of providing immunoregulatory immune effector cells transplantation of a suitable lymphoid graft of the present invention, such as one which includes suitable suppressor T-cells, can be used to suppress pathogenic immune responses. The ordinarily skilled artisan will possess the necessary expertise for treating a disease associated with a pathological immune response by transplantation of a lymphoid organ/tissue graft of the present invention according to the teachings of the present invention, and will have ample guidance available for practicing this aspect of the method of the present invention in the literature of the art (refer, for example, to: Toungouz M, Goldman M. et al., 2001. Adv Nephrol Necker Hosp. 31:257-72; Moore J, Brooks P., 2001. 23:193-213).

Following transplantation, the growth and/or differentiation of the graft, and the therapeutic effect of the graft may be advantageously monitored.

For example, as described in Example 1 of the Examples section below, the functionality of a renal graft of the present invention may be advantageously monitored following transplantation by analysis of production of fluid by the graft, in particular by analysis of such fluid for urine specific metabolite or by product content. Supra plasma concentrations of urine specific byproducts, such as, for example, urea nitrogen and creatinine are indicative of graft functionality.

As described in Example 5 of the Examples section below, pancreatic islet graft functionality may be advantageously monitored by analyzing serum glucose levels. Normalization of serum glucose levels in the serum of a diabetic subject following transplantation of a pancreatic islet graft is indicative of graft functionality (i.e., physiologically regulated insulin secretion by the graft).

The functionality of a splenic graft of the present invention may be easily monitored following transplantation via numerous assays routinely practiced by the ordinarily skilled artisan, including via analysis of appropriate liver metabolism-specific proteins in the serum of the subject.

Also, the functionality of a cardiac graft of the present invention may also be conveniently monitored following transplantation via numerous methods practiced by the ordinarily skilled artisan, including via echocardiography, electrocardiography, and analysis of cardiac function-specific proteins in the serum of the subject, depending on the application and purpose.

As well, the functionality of a lymphoid graft of the present invention may similarly monitored following transplantation, depending on the application and purpose, via numerous methods routinely practiced by the ordinarily skilled artisan, for example, via harvesting of peripheral blood cells of the subject and analysis thereof with respect to appropriate cell types by fluorescence activated cell sorting (FACS), via appropriate antigen specific lymphocyte stimulation assays, and via assaying of appropriate cytokines, chemokines, antibodies, coagulation factors, lysosomal storage enzymes, and the like in the subject's serum via ELISA, or via assaying of such molecules in the subject's blood cells via FACS.

Following transplantation, the immunological tolerance of the subject to the graft, and/or the growth and differentiation of the graft may be advantageously monitored.

Various methods may be employed to assess the subject's immunological tolerance to the graft.

For example the tolerance may be assessed by monitoring subject specific leukocyte or T cell specific infiltration of the graft, by monitoring the origin of the graft vasculature, and/or by monitoring the histological appearance of organ or tissue specific structures. Such monitoring may be advantageously effected as described in Example 1 of the Examples section below. Infiltration of subject leukocytes, neutrophils, natural killer cells, or T cells into the graft, or lack thereof, are typically indicative of suboptimal or optimal engraftment of the graft in the subject, respectively.

In cases where subject tolerance of the graft is suboptimal, therapeutic adjunct immunosuppressive treatment of the subject may be advantageously performed, as described hereinabove.

While reducing the present invention to practice, organs/tissues at defined stages of differentiation corresponding to a specific gestational stage found not expressing or presenting a molecule capable of stimulating or enhancing an immune response prior to and/or following transplantation thereof into a recipient were unexpectedly revealed to be capable of generating structurally and functionally differentiated organs/tissues optimally tolerated by non syngeneic lymphocytes when transplanted into a subject.

Thus, according to a further aspect of the present invention there is provided a method of evaluating the suitability of a mammalian organ or tissue at a stage of differentiation corresponding to a specific gestational stage for transplantation of a graft of the organ or tissue into a mammalian subject.

The method is preferably effected by evaluating a test transplant taken from the organ or tissue for expression or presentation of the molecule capable of stimulating or enhancing an immune response (hereinafter "the molecule") in the subject prior to and/or following transplantation of the test transplant into a mammalian test recipient.

According to the teachings of the present invention, a test transplant found not substantially expressing or presenting the molecule prior to and/or following transplantation of the test transplant into the test recipient will be optimal for transplantation. In general, the lower the level of expression or presentation of the molecule in the test transplant, the more suitable the organ or tissue graft will be for transplantation. In particular, the lower the level of expression or presentation of the molecule in the test transplant, the more optimally the graft will structurally differentiate, functionally differentiate, and be tolerated by non syngeneic lymphocytes following transplantation into the subject.

It will be appreciated that since test transplants at stages of differentiation corresponding to various gestational stages can be tested for expression of the molecule, the method according to this aspect of the present invention enables identification of an optimal stage of differentiation of the organ or tissue for transplantation of a graft thereof into the subject.

According to the teachings of the present invention, testing the test transplant for the presence of the molecule is preferably effected prior to transplantation of the test transplant into the test recipient, and/or following a posttransplantation period selected from a range of 1 second to 45 days, depending on the type of molecule tested, as described in further detail hereinbelow.

The method according to this aspect of the present invention may be practiced using a test recipient of any of various mammalian species, and/or displaying any of various characteristics, depending on the application and purpose.

According to the teachings of the present invention, the test recipient is preferably a rodent, and/or the subject.

Preferably, the rodent is a mouse.

The use of a mouse as the test recipient is highly advantageous since this species, for numerous reasons, is by far the most convenient, economical, and effective experimental mammal available.

According to further teachings of the present invention, the test recipient bears functional human T lymphocytes.

Preferably, the human T lymphocytes are non syngeneic with the organ or tissue.

As is described and forcefully illustrated in Example 1 of the Examples section below, the method may be effectively practiced by transplanting a human test transplant into a test recipient bearing human T lymphocytes non syngeneic with the organ or tissue.

Thus, the method of evaluating the stage of differentiation of a graft most suitable for transplantation of the present invention is unique and optimal relative to all such prior art methods, and may be conveniently used to identify the stage of differentiation or gestation of essentially any organ or tissue type optimally suitable for therapeutic transplantation of a graft thereof into a human.

Although the method according to this aspect of the present invention may be practiced using a graft derived from essentially any mammalian species, the organ or tissue is preferably a porcine organ or tissue, more preferably a human organ or tissue.

Alternately, the method may be advantageously effected using a porcine organ or tissue at a specific stage of differentiation selected corresponding to 20 to 63 days of gestation, more preferably 20 to 56 days of gestation, more preferably 20 to 42 days of gestation, more preferably 20 to 35 days of gestation, more preferably 20 to 28 days of gestation, more preferably 24 to 28 days of gestation, and most preferably 27 to 28 days of gestation.

The method of evaluating the stage of differentiation of a graft suitable for transplantation of the present invention may be effected by testing the test graft for any of various types of molecules capable of stimulating or enhancing an immune response in the subject.

Examples of such types of molecules include cytokines, chemokines, inflammatory mediators, immune cell receptors, immune cell coreceptors, MHC molecules, antigen-presenting molecules, adhesion molecules, innate immunity mediators, apoptosis mediators, metalloproteinases, immunomodulators, lymphocyte coreceptors and lymphocyte coreceptor ligands.

Preferably, a graft suitable for transplantation does not substantially express or present a lymphocyte coreceptor or lymphocyte coreceptor ligand.

Examples of such lymphocyte coreceptors and lymphocyte coreceptor ligands include CD28, B7-1 (CD80), B7-2 (CD86), CD40, CD40L (CD40 ligand, CD154), CD2, CD58 [lymphocyte function associated antigen-3 (LFA-3)], intercellular adhesion molecule-1 (ICAM-1) and lymphocyte function associated antigen-1 (LFA-1).

Preferably, a graft suitable for transplantation does not substantially express or present B7-1, more preferably CD40 or CD40L, more preferably CD40 and CD40L, and most preferably B7-1, CD40, and CD40L.

As mentioned hereinabove, testing the test transplant for the presence of the molecule is preferably effected prior to transplantation of the test transplant into the test recipient, and/or following a posttransplantation period selected from a range of 1 second to 45 days, depending on the type of molecule tested.

Preferably, testing the test transplant for the presence of CD40 or CD40L is effected both prior to and following transplantation of the test transplant into the test recipient.

Preferably, testing the test transplant for the presence of CD40 following transplantation of the test transplant into the test recipient is effected following a test transplant posttransplantation period selected from a range of 1 second to 45 days, more preferably 11 days to 45 days, more preferably 11 days to 42 days, more preferably 11 days to 31 days, and most preferably 14 days to 28 days.

Preferably, testing the test transplant for the presence of CD40L following transplantation of the test transplant into the test recipient is effected following a test transplant posttransplantation period selected from a range of: 1 second to 45 days; more preferably 11 days to 45 days; more preferably 14 days to 45 days; more preferably 14 days to 31 days; more preferably 17 days to 31 days or 14 days to 28 days; more preferably 25 days to 31 days; more preferably 27 days to 29 days; more preferably 27.5 days to 28.5 days; and most preferably 28 days.

Preferably, testing the test transplant for the presence of B7-1 is effected prior to transplantation of the test transplant.

As is described and shown in Example I of the Examples section below, grafts not substantially expressing B7-1, CD40, and CD40L at the respective optimal test transplant pretransplantation/posttransplantation periods set forth hereinabove are suitable for transplantation. In particular, such grafts can generate structurally and functionally differentiated organs/tissues optimally tolerated by non syngeneic/alloreactive human lymphocytes.

While not being bound by a paradigm, the present inventors are of the opinion that a graft which does not substantially express or present such molecules, which are major antigen presenting cell specific molecules, is optimally tolerated by an allogeneic or xenogeneic subject at least partly as a result of such grafts substantially lacking antigen presenting cells which have been proposed in the art as being critical for activation of graft rejection.

Numerous methods, well known to the ordinarily skilled artisan, may be used to analyze an organ, a tissue or cells, such as the test graft or a portion thereof, for expression or presentation of a specific molecule.

In cases where the molecule is a protein or RNA molecule, expression or presentation of the molecule is preferably evaluated by analysis of cells or tissues for the presence of mRNA encoding the protein molecule, or for the presence of the RNA molecule, respectively.

Analysis of an mRNA or RNA molecule in cells or tissues is preferably effected by RT-PCR analysis. RT-PCR analysis may be advantageously performed as described and illustrated in Example 1 of the Examples section, below. Alternately, analysis of the presence of an mRNA or RNA molecule in cells can be performed by modifications of the RT-PCR protocol described in Example 1 of the Examples section, below (e.g. using a nested PCR phase, competitive RT-PCR, etc.), by Northern blotting, or by microarray analysis.

Alternately, expression or presentation of the protein molecule can be directly detected by directly detecting the protein molecule using various biochemical techniques.

Various methods of detecting expression or presentation of a specific protein in an organ, tissue, or cells are well known to those of ordinary skill in the art. Such methods include immunofluorescence flow cytometry, Western immunoblotting analysis, fluorescence in situ hybridization (FISH), enzyme linked immunosorbent assay (ELISA), microarray hybridization, immunofluorescence confocal microscopy, and the like.

In cases where the molecule is a protein, expression or presentation of both the molecule as well as mRNA encoding the molecule are tested.

In cases where the molecule is a protein, an optimal graft is one which does not substantially express the molecule, more preferably mRNA encoding the molecule, or most preferably both the molecule and mRNA encoding the molecule.

According to the teachings of the present invention, evaluating the suitability of the graft for transplantation may advantageously comprise analyzing grafts for expression or presentation of substantially lower levels than adult stage graft type organs/tissues of the immunity related molecules listed in Table 3 of the Examples section below and on the World Wide Web/Internet (http://www.weizmann.ac.il/ immunology/reisner/immunogenicity.xls).

As is described and illustrated in Example 1 of the Examples section below, grafts expressing substantially lower levels of such immunity related molecules than adult stage graft type organs/tissues are more suitable for transplantation than the latter. A graft which expresses or displays substantially lower levels than adult stage organs/tissues of graft organ tissue type of the greatest possible number of such immunity related molecules may be optimally suitable for transplantation.

Preferably, analysis of expression or presentation of such immunity related molecules in the graft is effected by microarray hybridization analysis of graft derived mRNA, preferably as described and illustrated in Example 1 of the Examples section, below.

Alternately, analysis of expression or presentation of such immunity related molecules in the graft may be effected using any of the analytic techniques described hereinabove for analysis of the graft for expression or presentation of the molecule capable of stimulating or enhancing an immune response in the subject.

Thus, the present invention can be used to optimally treat disorders using transplantation of non syngeneic or developing organ or tissue derived grafts, and to identify organ or tissue grafts optimally suitable for practicing therapeutic transplantation.

The therapeutic transplantation method of the present invention is unique and dramatically superior relative to all such prior art methods since it enables for the first time optimal, generalized treatment of the wide range disorders amenable to therapeutic transplantation in humans by allograft and/or xenograft transplantation without, or with minimal immunosuppression of graft recipients, using either developing xenogeneic or allogeneic grafts which are, respectively, in essentially unlimited supply, or of essentially any allogeneic background.

It is expected that during the life of this patent many relevant medical diagnostic techniques will be developed and the scope of the phrase "method of evaluating the stage of differentiation of a mammalian organ most suitable for transplantation thereof into a mammalian subject" is intended to include all such new technologies *a priori.*

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions, illustrate the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization -A Laboratory Course Manual" CSHL Press (1996); all of which are incorporated by reference as if fully set forth herein. Other general references are provided throughout this document. The procedures therein are believed to be well known in the art and are provided for the convenience of the reader.

The examples marked as reference examples contain a part relating to human embryonic tissus which is not part of the invention.

### Reference EXAMPLE 1

### Transplantation of early gestational stage human or porcine renal organs/tissues generates structurally and functionally differentiated renal organs/tissues tolerated by alloreactive/xenoreactive human lymphocytes

Diseases of organs/tissues for which allogeneic donor organ/tissue transplantation remains the optimal therapeutic option, such as kidney disease, are highly debilitating and associated with significant mortality rates. However, allogeneic donor organ/tissue transplantation is often impossible to implement due to the difficulty of finding a haplotype-matched organ/tissue donor. Moreover, even when a matched donor is found, in order to prevent graft rejection such transplantation requires permanent graft recipient immunosuppression, usually via administration of toxic immunosuppressant drugs such as cyclosporin A. Such immunosuppressive treatments contribute to the drawbacks of allogeneic transplantation, since these are often unsuccessful at preventing graft rejection in the short term, and are usually incapable of indefinitely preventing graft rejection. An alternative to allograft transplantation involves transplantation of xenografts, in particular porcine grafts which are considered the optimal animal alternative to human grafts. However, xenografts generally cannot be used for transplantation due to highly suboptimal tolerance of such grafts by human lymphocytes. Thus, organs/tissues suitable for therapeutic transplantation in humans and tolerated by non syngeneic human lymphocytes, and adequate sources of such organs/tissues, are highly desired. One proposed strategy for providing such organs/tissues involves using grafts at early developmental stages, since it has been demonstrated that the earlier the developmental stage of an organ/tissue, the better it is tolerated when transplanted into a non syngeneic host. However, to date, satisfactory growth and differentiation of developing or non syngeneic organ/tissue grafts, and satisfactory immunological tolerance of such grafts by human lymphocytes in the absence of graft-derived teratomas has not been achieved.

While conceiving the present invention, it was hypothesized that there exists a developmental stage during which organs/tissues are sufficiently differentiated to be committed to organ/tissue specific development in the absence of graft-derived teratomas while being sufficiently undifferentiated so as to be optimally tolerated when transplanted into a non syngeneic host. While reducing the present invention to practice, the existence of specific gestational stages during which human or porcine organs/tissues can be transplanted into a host so as to generate, in the absence of graft-derived teratomas, structurally and functionally differentiated graft-derived organs/tissues which are optimally tolerated by alloreactive/xenoreactive human lymphocytes in the host were unexpectedly uncovered. In particular, the existence of specific gestational stages during which human or porcine renal organs/tissues can be transplanted into a recipient so as to generate, in the absence of graft-derived teratomas, optimally structurally and functionally differentiated renal organs which are optimally tolerated by alloreactive/xenoreactive human lymphocytes in the recipient, were unexpectedly uncovered, as described below and/or as previously described (Dekel B. et al., 2002. Nature Medicine).

### Materials and Methods:

***Preparation of murine transplant hosts:*** Three month old Balb/c mice (Harlan Olac, Shaw's Farm, Blackthorn, Bicester, Oxon., UK) were used as hosts for the transplantation studies. For generation of immunodeficient recipients, Balb/c mice were lethally irradiated by split-dose total body irradiation (TBI; 3.5 Gy followed 3 days later by 9.5 Gy) by a 150-A (60)Co gamma ray source (produced by the Atomic Energy Commission of Canada, Kanata, Ontario) with a focal skin distance of 75 centimeters and a dose rate of 0.7 Gy/minute, as previously described (Lubin I. et al., 1994. Blood 83, 2368; Reisner, Y. and Dagan, S., 1998. Trends Biotechnol. 16, 242-246; Segall, H. et al., 1996. Blood 88, 721-730). Bone marrow cells from NOD/SCID mice (Weizmann Institute Animal Breeding Center, Rehovot, Israel) were flushed from femur and tibia shafts of 4-8 week-old mice, as previously described (Levite M. et al., 1995. Cell Immunol. 162, 138). Recipient Balb/c mice were immune-reconstituted with 3 × 10⁶ bone marrow cells from NOD/SCID mice administered intravenously in 1 milliliter phosphate buffer saline solution one day following the second fraction of total body irradiation (TBI), as previously described (Reisner, Y. and Dagan, S., 1998. Trends Biotechnol. 16, 242-246; Segall, H. et al., 1996. Blood 88, 721-730). The resulting SCID (severe combined immunodeficiency) mouse-like animals have been shown to allow excellent engraftment of functioning human hematopoietic cells or solid tissues (Marcus H. et al., 1995. Blood 86, 398; Segall H. et al., 1996. Blood 88, 88; Reisner Y. and Dagan S., 1998. Trends Biotechnol. 16, 242; Bocher WO. et al., 2001. Eur J Immunol. 31, 2071). Donor NOD/SCID mice were obtained from the Weizmann Institute Animal Breeding Center, Rehovot, Israel, and animal experiments were carried out according to the National Institutes of Health Guide for Care and Use of Experimental Animals and approved by the Weizmann Institute of Science Animal Care Committee.

***Harvesting of developing renal tissue:*** Developing human renal tissues were obtained by curettage with the approval of a Helsinki committee and developing renal tissues were surgically dissected from embryos under a dissection stereoscope as previously described (Rogers S. et al., 1998. Kidney Int. 54, 27). Adult kidney specimens were obtained from normal kidneys removed for stage I clear cell carcinoma. Gestational stage and adult porcine renal tissues were obtained with the assistance of the Lahav Institute for Animal Research, Kibbutz Lahav. Gestational stage porcine renal tissues were isolated from animals using previously described techniques (Rogers S, et al., 1998. Kidney Int. 54, 27). All specimens for gene expression analysis were snap-frozen in liquid nitrogen. Tissues for transplantation were kept in sterile conditions at 4 °C (for approximately 2 hours) in either RPMI 1640 or Dulbecco's modified Eagle's medium (DMEM) supplemented with 10 % fetal calf serum (Biological Industries, Beit HaEmek, Israel).

***Transplantation of developing renal tissue:*** Transplantation of renal tissue under the renal capsule of recipient mice was performed as previously described (Dekel, B. et al., 1997. Transplantation 64, 1541-1550). Whole human (at up to 8 weeks of gestation) or porcine (at up to 4 weeks of gestation) kidney precursors, or whole or 1-2 mm-diameter fragments of renal tissues at later stages of gestation were used in transplantations. Transplantation was performed 7-10 days following reconstitution of irradiated hosts with NOD/SCID bone marrow. For growth assays, renal tissues were transplanted into SCID recipient mice. For transplantation, renal tissues were maintained in sterile conditions at 4 °C for approximately two hours in either RPMI 1640 or Dulbecco's modified Eagle's medium supplemented with 10 % fetal calf serum (FCS; Biological Industries, Beit Haemek, Israel). Transplantation of renal tissues was performed under general anesthesia induced by intraperitoneal injection of 2.5 % Avertin in phosphate buffer saline solution (10 milliliters per kilogram body weight). Both host kidneys were exposed via a bilateral incision, a 1.5 mm incision was made at the caudal end of the renal capsule, and an approximately one cubic millimeter fragment of renal tissue was implanted under each renal capsule. Renal tissues were also transplanted intra-abdominally to control for renal subcapsular space specific immune privilege. In some experiments, renal tissues were implanted and sutured (5-0 suture) onto the testicular fat pad in conjunction with a left nephrectomy. Transplanted mice were treated post-operatively with ciprofloxacin in their drinking water for 7 days.

***Engraftment of mice with human PBMCs:*** One to three days following transplantation of renal tissue, as described above, 10⁸ human PBMCs were injected intraperitoneally in host mice. Control mice did not receive human PBMCs. Generation of human PBMCs, their infusion into recipient mice, and analysis of engraftment of infused cells were performed as previously described (Segall, H. et al., 1996. Blood 88, 721-730). Human PBMCs were generated from buffy coats obtained from normal volunteers, as follows. Blood samples were overlayed on a cushion of Lymphoprep solution (Nycomed, Oslo, Norway) and centrifuged at 2,000 rpm for 20 minutes. The interface layer was collected and washed twice, and the cells were counted and resuspended in phosphate buffer saline solution (pH 7.4) at the desired concentration. For analysis of human lymphocyte engraftment, cells were recovered from peritonea 10 to 14 days following PBMC infusion. Single-cell suspensions were incubated for 30 minutes on ice with labeled anti-human CD3-PE and CD45-PerCP (pan-human leukocyte antigen) antibodies (Becton-Dickinson, Mountain View, CA). After washing, two- or three-color fluorescent analysis of these human antigens was performed using a FACScan analyzer (Becton-Dickinson). Data was collected from lymphocytes selectively gated via standard forward- and side-scatter characteristics.

In certain experiments, dual PBMC infusions from separate human donors were administered to graft recipient mice.

***Analysis of graft infiltration, growth and differentiation:*** Human immune cell infiltration as well as growth and development of renal tissue derived grafts into mature glomeruli and tubuli were monitored following transplantation, as follows. Graft recipients were sacrificed 4-10 weeks posttransplantation, as indicated. Recipient kidneys and their capsules were then removed and fixed in 10 % paraffin. Transplants were sectioned and mounted on slides coated with poly-L-lysine and sections were stained with hematoxylin and eosin (H&E). To determine growth of grafts from time of transplantation to time of harvest, the sizes of the graft pretransplant and at time of harvest (posttransplant) were measured, and posttransplant to pretransplant graft size ratios were calculated. Graft size was determined according to the formula *graft size* = *L × W*, where *L* and *W* represent the long and short axes of the graft, respectively. Assessment of graft development was performed by counting the number of mature glomeruli and tubuli in 10 consecutive microscopic fields (×40 magnification) per transplant in 3 transplants per group. Determination of human T cell infiltration in graft sections was determined as previously described (Naveh MT. et al., 1992. J Clin Invest. 90, 2434). Briefly, graft sections were immunostained with rabbit anti human CD3 antibody (Zymed, San Francisco, CA; pan T-cell), as previously described (Dekel, B. et al., 1999. Int. Immunol. 11, 1673-1683), and the number of human CD3 positive cells was counted in 10 consecutive microscopic fields (×100 magnification) per transplant in 3 transplants per group. Paraffin tissue blocks of transplants were cut 4-6 µm thick, deparaffinized in xylene, rehydrated and placed for 15 min in ethanol containing 3 % H₂O₂ to block endogenous peroxidase. Slides were thoroughly washed with tap water and transferred to PBS. Sections were then treated with 1 % bovine serum albumin to prevent background staining and incubated for 1 hour with anti CD3 antibody at room temperature in a humidified chamber. Slides were rinsed with phosphate buffer saline solution for 3 minutes and incubated with a biotinylated anti rabbit antibody for 30 minutes and then incubated with peroxidase conjugated streptavidin (StrAvigen; Biogenex, San Ramon, CA) for 30 minutes. After rinsing, the peroxidase label was visualized by incubation with for 15 minutes and counterstained with Mayer's hematoxylin using an immunohistochemical staining kit (Biomeda, Foster City, CA), according to the manufacturer's instructions. The reagent 3-amino-9-ethylcarbazol produced a red product that is soluble in alcohol and can be used with an aqueous mounting medium (Kaiser's glycerol gelatin). A negative control for staining of T lymphocytes was performed by following all of the aforementioned steps but omitting addition of primary antibody. Staining was found to be uniformly negative in transplants from control mice not infused with human PBMCs.

***Analysis of host vessel vascularization:*** Five micrometer thick paraffin sections were immunostained with a rat antibody specific for mouse PECAM-1 (Pharmingen, San Diego, California) using a Histostain Plus kit (Zymed, San Francisco, California), according to the manufacturer's instructions. Vessel counts were performed in similar regions within renal grafts per high-power field (5 consecutive fields per transplant in 5 transplants per group).

***Urine collection and analysis:*** Fluid produced by early gestational stage renal tissue graft derived cysts was collected and analyzed for urinary marker content, as follows. Developing human and porcine transplants were surgically exposed in anesthetized mice via midline or left flank incision and a plastic microcatheter was inserted into an identifiable area of fluid concentration. At the site of insertion, the graft walls were sutured around the catheter using a 5-0 nylon suture, and fluid from the graft was collected via the microcatheter into small volume PCR tubes sutured onto the skin of the mice. The drained fluid was subsequently analyzed for urea nitrogen and creatinine concentrations.

***RT-PCR analysis of costimulatory molecule expression in grafts:*** Among the multiple co-stimulatory pathways identified, increasing evidence suggests that interaction of the T cell costimulatory receptors CD28 and CD40 ligand (CD40L, CD154) with their respective ligands B7-1/-2 and CD40 expressed on antigen presenting cells are critical for T cell responses to alloantigens (Sayegh MH. and Turka LA., 1998. N Engl J Med. 338, 1813). Experiments were thus performed to test whether alloreactive human immune cells did not reject allogeneic 7- to 8-week gestational stage human renal tissue derived grafts as a result of such tissues downregulating expression of the co-stimulatory molecules B7-1, B7-2, CD40, CD40L, and HLA-DR, as follows. Messenger RNA from 8-, 14-, and 22-week gestational stage renal tissue derived grafts was analyzed via RT-PCR at the following time points: (i) prior to transplantation; (ii) immediately following transplantation but prior to infusion of alloreactive human PBMCs; and (iii) 2, 4, and 6 weeks following reconstitution of mice with human PBMCs, as follows. Grafts were carefully dissected from the subcapsular implantation site and total RNA was isolated from the dissected grafts as previously described (Dekel, B. et al., 1999. Int. Immunol. 11, 1673-1683). Briefly, the renal graft tissues were homogenized with a glass-Teflon tissue homogenizer in Tri-reagent (Molecular Research Center Inc., Cincinnati, OH) for isolation of total RNA, according to the manufacturer's instructions. The isolated RNA was air-dried, resuspended in nuclease-free water and quantified by spectrophotometry. Aliquots of 1 microgram of total RNA preparation were reverse-transcribed into cDNA with AMV reverse transcriptase using an Access RT-PCR kit (Promega Corp., Madison, Wisconsin), according to the manufacturer's instructions. Sequences specific to the costimulatory molecules and to the control housekeeping gene β-actin (Pratt, J.R. et al., 2002. Nature Med. 8, 582-587) were subsequently PCR amplified from the synthesized cDNA, as follows. Briefly, reverse transcription cDNA product was diluted 1:50, 1:100, and 1:500 in sterile water and PCR amplification was performed using thermostable Tfl DNA polymerase in a 50 microliter reaction mixture containing 40 micromolar of each dNTP, 0.4 micromolar of each primer (Table 1), 10 millimolar Tris HCl (pH 8.3), and 1.5 millimolar MgCl₂. Each sample was tested at least three times, and compared tissue samples were PCR-amplified in parallel using a single master reagent mix. In order to minimize non specific amplification of non target sequences, the PCR annealing temperature was set high (64 °C), and, in order to detect PCR signals in the linear phase of product amplification, the PCR reaction was performed with 20-35 thermal cycles. In all experiments the possibility of amplification from contaminating DNA was ruled out via control reactions using reverse transcription reactions in which reverse transcriptase or template cDNA was omitted. PCR reaction products were separated electrophoretically in 1.5 % agarose gels, the gels were stained with ethidium bromide and photographed using a UV transilluminator, as previously described (Sharma VK. et al., 1996. Transplantation 62, 1860).

***Transplantation of xenogeneic early gestational stage renal tissue grafts in normal immunocompetent mice in conjunction with mild*, *short course costimulation blockade:*** To test the immunogenicity of early gestational stage xenogeneic renal tissue grafts in a normal, immunocompetent mammal, immunocompetent Balb/c mice were transplanted with 27- to 28-day gestational stage porcine renal tissue grafts, as described above for transplantation of grafts into immune reconstituted mice, and subjected to brief blockade of costimulation by intraperitoneal injection of 250 micrograms CTLA4-Ig (kindly provided by Steffen Jung, Hadassa Medical School, Jerusalem, Israel) every 48 hours for 2 weeks. CTLA4-Ig is a fusion protein comprising the extracellular portion of mouse CTLA-4 fused to the constant region of human IgG which blocks the costimulatory interaction of the T cell costimulatory receptor CD28 with its antigen presenting cell costimulatory ligands B7-1 and -2. Control mice were injected with phosphate buffered saline or control immunoglobulin.

**Table 1. Oligonucleotide primers used for PCR amplification of cDNA prepared from human developing renal tissues.**

| Amplified sequences | Oligonucleotide primers (sense/antisense) | PCR product length (bp) |
|---|---|---|
| B7-1 | 5'-GACCAAGGAAGTGAAGTGGC-3' (SEQ ID NO:1)/ 5'-AGGAGAGGTGAGGCTCTGGAAAAC-3' (SEQ ID NO:2) | 410 |
| B7-2 | 5'-CACTATGGGACTGAGTAACATTC-3' (SEQ ID NO:3)/ 5'-GCACTGACAGTTCAGAATTCATC-3' (SEQ ID NO:4) | 383 |
| CD40 | 5'-CTCTGCAGTGCGTCCTCTGGGG-3' (SEQ ID NO:5)/ 5'-GATGGTATCAGAAACCCCTGTAGC-3' (SEQ ID NO:6) | 410 |
| CD40L | 5'-TATCACCCAGATGATTGGGTCAGC-3' (SEQ ID NO:7)/ 5'-CCAGGGTTACCAAGTTGTTGCTCA-3' (SEQ ID NO:8) | 349 |
| HLA-DR | 5'-ATGAAGGTCTCCGCGGCAGCCC-3'-(SEQ ID NO:9)/ 5'-CTAGCTCATCTCCAAAGAGTTG-3' (SEQ ID NO:10) | 215 |
| β-actin | 5'-ACCATCAAGCTCTGCGTGACTG-3' (SEQ ID NO:11)/ 5'-GCAGGTCAGTTCAGTTCCAGGTC-3' (SEQ ID NO:12) | 310 |

| | | |
|---|---|---|
| *Homology searches for all primer sequences were performed using the NCBI's GenBank database to ensure non-specificity of primers for mouse genes. | | |

***Statistical analysis:*** Comparisons between groups were evaluated by the Student's t-test. Data were expressed as mean ± s.e.m., and were considered statistically significant if *P* values were 0.05 or less.

***Microarray analysis:*** Labeled cRNA preparation and hybridization to a Genechip Human Genome HU95A array (Affymetrix) was performed as recommended by the microarray manufacturer. Analysis of Genechip data was performed as previously described (Zuo, F. et al., 2002. Proc. Natl. Acad. Sci. USA 99, 6292-6297; Kaminski, N. et al., 2000. Proc. Natl. Acad. Sci. USA 97, 1778-1783). For cluster analysis CLUSTER, GENE CLUSTER, and TREEVIEW programs (Eisen, M.B. et al., 1998. Proc. Natl. Acad. Sci. USA. 95, 14863-14868) and the SCOREGENE software package (http://FGUSheba.cs.huji.ac.il/) were used. Fold ratios were calculated for each sample against the geometric mean of all the samples. A detailed description of the scoring methods and the approach used for analysis of microarray data have been published (Kaminski N. and Friedman N., 2002. Am. J. Respir. Cell Mol. Biol. 27, 125-132).

### Experimental Results:

***Gestational age determines growth and differentiation:*** An initial experiment was carried out to determine the viability of transplants of adult human renal tissue in immunodeficient mice. At 8 weeks after transplantation all adult transplants (5/5) were found to be sclerotic and non-viable. To assess the influence of developmental stage on growth and differentiation potential, 7- to 14-week gestational stage human renal tissues were transplanted in immunodeficient mice (Table 2). Overall, more than 80 % of the human renal grafts from all donor ages survived, and all recovered grafts had increased in size, with no evidence of neoplasia in any of the recovered grafts.

**Table 2. Transplantation of human or porcine gestational stage renal organs/tissues into recipients bearing non syngeneic human leukocytes.**

| Graft origin | Gestational stage of graft | No. of grafts | Graft type | Graft* growth | Graft renal differentiation** | Graft non-renal differentiation | Necrosis |
|---|---|---|---|---|---|---|---|
| Human | 14-week | 3 | whole | 3 / 3 | none | none | 3 / 3 |
| | 14-week | 8 | fragments | 7 / 8 | 7 / 7 | none | none |
| | 10-week | 2 | whole | 2 / 2 | none | none | 2 / 2 |
| | 10-week | 6 | fragments | 6 / 6 | 6 / 6 | none | none |
| | 8-week | 5 | whole | 5 / 5 | 5 / 5 | none | none |
| | 7-week | 3 | whole | 3 / 3 | 3 / 3 | none | none |
| Porcine | 8-week | 7 | whole | 5 / 7 | none | none | 5 / 5 |
| | 8-week | 6 | fragments | 6 / 6 | 6 / 6 | none | none |
| | 6-week | 5 | whole | 4 / 5 | none | none | 4 / 4 |
| | 6-week | 6 | fragments | 6 / 6 | 6 / 6 | none | none |
| | 27- to 28-day | 12 | whole | 12 / 12 | 12 / 12 | none | none |
| | 24- to 25-day | 9 | whole | 8 / 9 | 5 / 8 | 3 / 8 | none |
| | 20- to 21-day | 9 | whole | 6 / 9 | 3 / 6 | 3 / 6 | none |

| | | | | | | | |
|---|---|---|---|---|---|---|---|
| Transplant growth and differentiation were assessed at 8 weeks after transplantation. Differentiation was categorized as renal (only nephrons), non-renal (differentiated derivatives other than renal) and necrosis (in addition to nephrons, appearance of necrotic areas mostly in center of transplant). | | | | | | | |

Results were distinctly different when 7- and 8-week gestational stage human renal grafts were compared with later gestational stage human renal grafts. At 8 weeks after transplantation, 7- and 8-week gestational stage renal tissue derived grafts (n = 8) had undergone remarkable growth (transplant size ratio was 20.1 ± 2.7). Complete nephrogenesis [5.5 ± 0.8 glomeruli and 19.3 ± 2.7 tubuli per field (x40 magnification)] was observed in transplants derived from these grafts, which originally contained mainly metarenal mesenchymal stem cells and ureteric buds, but no glomeruli. The gross morphology and histological appearances of such an 8-week gestational stage human renal tissue derived graft, 8 weeks after transplantation are shown in Figures 1a-b, respectively. Beyond this gestational time point, transplantation of developing whole fetal kidneys resulted in central graft necrosis and viability was reduced. Therefore small pieces of human fetal renal tissue were grafted into immunodeficient hosts, as previously described (Dekel, B. et al., 1997. Transplantation 64, 1550-1558; Dekel, B. et al., 2000. Transplantation 69, 1470-1478). Under identical conditions, sections of transplants derived from 10- and 14-week gestational stage tissues (n = 14) had significantly lower transplant size ratios (14.8 ± 2.2 and 12.3 ± 1.8, respectively, *P* < 0.01) as well as glomerular and tubular counts (4.2 ± 0.8 and 15.3 ± 2.7; 3.5 ± 0.8 and 11.2 ± 2.2 per HPF, respectively; *P* < 0.05). Therefore, in contrast to transplantation of more mature human fetal renal fragments, grafting of earlier gestational stage renal tissues led to optimal growth and nephrogenesis.

The same approach described above was used to assess the growth and potential to differentiate of gestational stage porcine renal tissues (Table 2). In this case, transplants of 6- and 8-week gestational stage porcine renal tissues exhibited central fibrosis and necrosis and graft deterioration, whereas sectioned grafts had a transplant size ratio of 10.5 ± 2.2 and 8.2 ± 1.2, respectively, at 8 weeks after transplantation. For characterization of early gestational stage porcine renal tissues, 20-21-, 24-25-, and 27-28-day gestational stage transplants (combined data of 30 transplants) were analyzed. The porcine 27-28-day gestational stage transplants all exhibited significant growth with a transplant size ratio at 8 weeks posttransplantation of 28.3 ± 4.1 and full differentiation into mature glomeruli and tubuli (7.0 ± 1.0 glomeruli and 35.5 ± 5.1 tubuli per high power microscopic field). The gross morphology and histological appearances of such a 4-week gestational stage porcine renal tissue derived graft are shown in Figures 1c-d, respectively. In contrast, six of nine 20-21-day gestational stage porcine transplants failed to develop or had evolved into growths containing few glomeruli and tubuli, but containing other differentiated derivatives, such as blood vessels (Figure le), and cartilage and bone (Figures le-f). Furthermore, 24-25-day gestational stage porcine renal tissues were inferior to 27-28-day gestational stage transplants for nephrogenesis, as non-renal cell types and disorganized cell clusters were found in three of nine transplants (Figures 1h-j). These findings complement recent *in-vitro* data (Oliver, J.A. et al., 2002. Am. J. Physiol. Renal Physiol. 283, F799-809), which both indicate that early in gestation the developing kidney contains pluripotent progenitor cells, or embryonic renal stem cells, with the ability to generate many cell types.

***Host-derived vascularization specific to early gestational stage renal tissue derived grafts is associated with graft immune tolerance:*** The ability of transplants to grow as tissues in non syngeneic hosts depends primarily on their ability to sustain angiogenesis in such hosts (Gritsch, H.A. et al., 1994. Transplantation 57, 906-917). In the case of xenotransplantation, the immunological barrier is conditioned to a large extent by the manner in which the transplant derives its blood supply (Cascalho, M. and Platt, J.L., 2001. Immunity 14, 437-446). To determine the ability of recipient mice to support angiogenesis of avascular early gestational stage human or porcine renal tissue derived grafts by ingrowth of recipient vessels, expression of mouse PECAM (CD31), a marker of sprouting endothelial cells, was analyzed on the developing transplants immunohistochemically. Counts of immunoreactive vessels reflecting the combined total number of capillary and larger vessels of host origin were performed per high power microscopic field (Vermeulen, P.B. et al., 1996. Eur. J. Cancer 32A, 2474-2484). At 4 weeks after transplantation, 23.5 ± 4.0 and 21.3 ± 3.2 vessels of host origin per high power microscopic field supplying the developing human and porcine transplants were found, respectively. Among these human and porcine tissue grafts, all external vessels stained positive for mouse CD31 (Figures 2a and b, respectively). In addition, medium and small size capillaries of host origin were detected in both glomeruli (Figures 2c and 2d) and parenchyme (Figures 2e and f) of the gestational stage human and porcine transplants. In transplants originating from mature, vascularized 16-week gestational stage human and 8-week gestational stage porcine renal tissues, there was a significantly reduced mouse CD31 positive vessel count (10.2 ± 1.8 and 11.5 ± 2.2, respectively, *P* < 0.001) composed of mainly external larger vessels, but not endothelial cells in glomeruli and small capillaries (Figures 2g and h). Control sections of vascularized human and porcine renal tissues displayed no CD31 positive host derived vessels (Figures 2i-j, respectively). Thus, recipient mice have a significantly larger contribution to vasculogenesis of early gestational stage human and porcine renal tissue derived grafts including the formation of the microcirculation thereof.

***Early gestational stage human and porcine renal grafts differentiate into functional renal organs producing dilute urine:*** Early gestational stage renal tissue derived grafts were found to form large fluid filled cysts. Figures 3a and b, respectively depict the appearance of such cysts in 8-week gestational stage human and 4-week gestational stage porcine renal tissue derived grafts, respectively. Such cysts were observed to form in particular in abdominal grafts where they were not growth limited by the renal subcapsular space. To assess whether the fluid in these cysts represented by-products of renal function, the cyst fluid from 8-week gestational stage human (n = 2) and 4-week gestational stage porcine (n = 4) renal tissue derived grafts was collected analyzed for urea nitrogen and creatinine content, 6-8 weeks after transplantation. As the transplants cannot use the host's excretory system for urine drainage, fluid was drained by insertion of a permanent microcatheter into the developing renal grafts. Average levels (mg/dl) of urea nitrogen and creatinine were found to be higher in cyst fluid compared with those found in the sera of transplanted mice (518 ± 169 versus 45 ± 8 and 7.2 ± 1.9 versus 0.46 ± 0.048, respectively; *P* < 0.001), indicating that the human and porcine transplants had produced urine. These results are in accordance with the demonstration that murine kidney precursors can develop into functional nephrons (Rogers, S.A. et al., 1998. Kidney Int. 54, 27-37; Rogers, S.A. et al., 2001. Am. J. Physiol. Regul. Integr. Comp. Physiol. 280, R132-136; Rogers, S.A. and Hammerman, M.R., 2001. Am. J. Physiol. Regul. Integr. Comp. Physiol. 280, R1865-1869; Rogers, S.A. and Hammerman, M.R., 2001. Am. J. Physiol. Regul. Integr. Comp. Physiol. 281, R661-665). Levels of urea nitrogen and creatinine in the cyst fluid were significantly lower compared with native bladder urine (518 ± 169 versus 4,279 ± 402 and 7.2 ± 1.9 versus 54 ± 6, respectively; *P* < 0.001). The dilute urine in the cyst fluid is compatible with the reduced capacity of early gestational stage kidneys to concentrate urine.

***Early gestational stage human or porcine renal tissue derived grafts are less susceptible to alloreactive*/*xenoreactive human lymphocytes than later developmental stage grafts:*** The issue of whether early gestational stage renal tissue derived grafts have an immunological advantage over later gestational stage tissue derived grafts was addressed, as follows. Preliminary experiments to establish baseline experimental conditions demonstrated that the minimal number of infused PBMCs capable of inducing complete rejection of adult human renal tissue derived grafts engrafted into recipient mice was 10⁸ cells (data not shown). Four weeks after transplantation of adult human kidney fragments in immunodeficient recipients together with 100 x 10⁶ alloreactive human PBMCs, massive lymphocytic infiltration, tissue destruction and rejection were observed, as previously described (Dekel, B. et al., 1997. Transplantation 64, 1541-1550; Dekel, B. et al., 1999. Int. Immunol. 11, 1673-1683). At 4 weeks after transplantation, grafts originating from 14-week gestational stage renal tissue grafts displayed an average of 39.8 ± 7.8 donor human T lymphocytes per high power microscopic field. Despite the presence of T cells in these grafts, early rejection similar to that of adult transplants did not occur, and growth of 14-, 10-, 8-, and 7-week gestational stage transplants took place during the first month (Dekel, B. et al., 1997. Transplantation 64, 1550-1558; Dekel, B. et al., 2000. Transplantation 69, 1470-1478), as shown in Figures 4a-d, respectively. Nevertheless, analysis of T cell infiltration in 14-week gestational stage renal tissue derived grafts at later time points (6-8 weeks post-transplant) revealed the harmful effects of the infiltrating cells as graft deterioration, in the form of tubule and glomerulus destruction, became apparent (Figures 4e-f, respectively), and transplant growth was significantly halted compared with transplants from animals that were not subjected to infusion of human PBMCs (Figure 4a). Similar results were obtained for 10-week gestational stage human renal grafts (Figure 4b). In contrast, upon infusion of 100 × 10⁶ human cells into the host's peritoneum, 8- or 7-week gestational stage renal tissue derived grafts exhibited preserved glomeruli and tubuli without infiltration of.donor human T cells, (Figures 4g-h, respectively) and grew similarly to transplants of control mice (Figures 4c-d, respectively), and hence displayed no apparent signs of destruction or rejection. Moreover, when the experimental protocol was altered so that two inocula of 100 × 10⁶ alloreactive human PBMCs from different donors were infused 6 weeks apart, immune cells did not reject the 8-week gestational stage human renal graft, but 14-week gestational stage human renal grafts transplanted in conjunction with PBMCs of the second donor were rejected (Figures 5a-b, respectively). Thus, the immunogenicity of the differentiated tissue, which developed for 6 weeks following implantation of 8-week gestational stage renal tissues, was still greatly reduced compared with 14-week gestational stage renal tissues.

Analysis of T cell infiltration was also performed in transplants of porcine renal tissue in hosts subjected to intraperitoneal infusion of 100 × 10⁶ human PBMCs. Preliminary experiments to establish baseline experimental conditions demonstrated that the minimal number of infused PBMCs capable of inducing complete rejection of porcine adult renal tissue derived grafts engrafted into recipient mice was 10⁸ (data not shown). Within 3 weeks, five of six adult porcine renal tissue derived grafts were infiltrated and histologic damage and destruction were apparent (data not shown). Infiltration of lymphocytes analyzed by H&E staining, and destruction of renal parenchyme tissue in the presence of human T cell infiltrate in the adult stage renal tissue derived graft at 4 weeks posttransplant are shown in Figures 6a-b and Figure 6c, respectively.

In 8-week gestational stage porcine renal tissue derived grafts, human T cell infiltration was readily detectable in all six grafts with an average of 40.5 ± 6.75 lymphocytes per high power microscopic field, 4 weeks after transplantation. Transplants of 8- and 6-week gestational stage renal tissues displayed signs of rejection, as evidenced by their low posttransplant to pretransplant size (Figures 7a-b, respectively) relative to that of 3- and 4-week gestational stage renal tissue derived grafts by 6 or 8 weeks posttransplant (Figures 7c-d, respectively). Analysis at later time points indicated that five of six 8-week gestational stage renal tissue derived grafts displayed signs of rejection in the form of tissue damage concomitant with T cell infiltration, as demonstrated by immunohistochemical staining with anti human CD3 antibody, and by H&E histochemical staining (Figures 8a-b, and 8c, respectively) of graft sections. These results, and similar findings from other experiments with 6-week gestational stage porcine renal tissue derived grafts, showed that the xenogeneic human PBMCs induced rejection of the developing porcine renal transplants at these stages of organogenesis. In sharp contrast, 28- and 21-day gestational stage porcine renal tissue derived grafts were not rejected (Figures 7c-d, respectively), nor did such grafts display T cell infiltration, as demonstrated for a 28-day gestational stage porcine renal tissue derived graft in Figures 9a-b. Furthermore, transplants of 28-day gestational stage renal tissue grafts in hosts subjected to a second infusion of 100 × 10⁶ xenogeneic human PBMCs, 4 weeks after transplantation, were not rejected, whereas 8-week gestational stage renal tissue grafts transplanted simultaneously with these cells were eventually rejected (Figures 10a-b, respectively).

Co-stimulatory molecules on donor antigen-presenting cells are crucial in the alloimmune response (Boussiotis, V.A. et al., 1996. J. Exp. Med. 184, 365-376; Schwartz, R.H., 1996. J. Exp. Med. 184, 1-8; Sayegh, M.H. and Turka, L.A., 1998. N. Engl. J. Med. 338, 1813-1821; Li, Y. et al., 1999. Nature Med. 5, 1298-1302). The mRNA expression of the costimulatory molecules B7-1, B7-2, CD40, CD40L, and HLA-DR before and after transplantation of developing human renal tissue in the absence and presence of alloreactive human lymphocytes were therefore analyzed. The results obtained demonstrated differential expression of co-stimulatory molecules in both normal adult and transplanted human renal tissues at different gestational stages, with a distinct deficiency (especially CD40 and B7-1) in 8-week relative to 14- and 22-week gestational stage renal tissue derived transplants (Figures 11a-c, respectively). Expression of CD40 or CD40L mRNA in 8-week gestational stage renal tissue derived grafts was not detected by PCR analysis for up to 6 weeks posttransplant (Figure 11a). In contrast such expression was detected in 14- and 22-week gestational stage renal tissue derived grafts by 4 weeks posttransplant (Figures 11a-c, respectively). In addition, B7-1 expression following transplantation and PBMC infusion was found to be significantly lower in 8-week gestational stage renal tissue derived grafts (Figure 11a) compared to 14- and 22-week gestational stage renal tissue derived grafts (Figures 11a-c, respectively). This pattern of co-stimulatory molecule gene expression is consistent with the *in-vivo* data demonstrating complete absence of immune responses by human allogeneic effectors against transplants of human renal tissue from 7- or 8-week human fetuses and thereby provides a mechanism underlying the allogeneic immune tolerance to such tissues achieved.

***Advantage of early gestational stage renal tissue derived grafts in immunocompetent mice:*** The optimally low immunogenicity of the early gestational stage porcine renal tissues was further demonstrated by transplanting adult or 27- to 28-day gestational stage porcine renal tissues into immunocompetent Balb/c mice. Evaluation of adult (n = 10) and 27- to 28-day gestational stage renal tissue (n = 10) grafts after 2 weeks showed rejection of both tissues. Following short course treatment with CTLA4-Ig, an immunoglobulin fusion protein that directly affects T-cell recognition of B7 on antigen-presenting cells (Linsley, P.S. et al., 1991. J. Exp. Med. 174, 561-569), at 2 to 4 weeks post-transplant, all adult grafts (8/8) had a disturbed morphology, necrotic tissue and a high degree of lymphocyte infiltration. In contrast, at the same time point, infusion of CTLA4-Ig resulted in growth and differentiation of 6 of 10 of the early gestational stage transplants, which was not seen in the untreated animals, indicating the immune advantage of the early gestational stage renal tissue derived transplants over developed adult kidney tissue derived transplants in fully immunocompetent hosts.

***Decreased expression of multiple immunity related genes in early gestational stage renal tissue:*** To investigate inherent immunogenic properties of the early gestational stage renal tissue which might account for its decreased immunogenicity relative to more mature tissue, global gene expression in early gestational stage and adult gestational stage human renal tissues were analyzed by microarray analysis. Furthermore 231 genes having direct immunity related roles were analyzed (the complete list of genes can be found on the World Wide Web/Internet at http://www.weizmann.ac.il/immunology/reisner/immunogenicity.xls. These included genes encoding HLA molecules, cytokines, chemokines, chemokine receptors, apoptosis mediators, adhesion molecules, metalloproteinases, molecules of innate immunity and other immunomodulators. Hierarchical clustering (Eisen, M.B. et al., 1998. Proc. Natl. Acad. Sci. USA. 95, 14863-14868) of all genes on the basis of similarity in gene expression among the experimental groups revealed two main clusters, separating the adult from fetal tissues. Moreover, the immunity related genes were grouped according to gestational stage with a cluster of genes within the earliest gestational stage renal tissue and a cluster of genes within the adult kidney tissue on opposing sides of a hierarchical clustering dendrogram (Figure 12a). The patterns of "immune" gene expression are presented using PLOTTOPGENE program (Zuo, F. et al., 2002. Proc. Natl. Acad. Sci. U. S. A. 99, 6292-6297) (Figure 12b). Such analysis unexpectedly uncovered that 68 genes were significantly changed between adult and fetal tissues (P < 0.05, total number of misclassifications (TNoM) = 0). Expression profiles of these genes demonstrated those increased in the adult tissues (n = 57 genes; Figure 12c, top) and those decreased (n = 11 genes; Figure 12c, bottom). Examples of the most significantly changed immunity related genes include those encoding molecules participating in both the acquired and the innate immune response (Table 3).

***Discussion:*** The presently described results show that 7- to 8-week gestational stage human renal tissue derived grafts, and 20- to 28-day gestational stage porcine renal tissue derived grafts transplanted into immunodeficient mice survive, grow and undergo complete nephrogenesis, forming a functional organ able to produce urine. Earlier gestational stage cells fail to mature exclusively into differentiated renal structures and form non renal differentiated derivatives and disorganized cell clusters. Furthermore, optimal renal organogenesis was achieved by transplantation of whole-organ early gestational stage grafts. At these early gestational stages both human and porcine renal tissues contain renal mesenchymal stem cells and ureteric bud branches, but no glomeruli, emphasizing their remarkable potential to differentiate after transplantation. A key observation of the above described results is that growth and development of such early developmental stage renal tissues is facilitated by host derived vasculature.

**Table 3. Immunity related genes differentially expressed in early gestational versus adult stage human renal tissue.**

| Gene category | Differentially expressed gene |
|---|---|
| HLA | MHC class I, C |
| | MHC class I, A |
| | MHC class I, E |
| | MHC class II, DPβ1 |
| Chemokines/adhesion | RANTES |
| | monocyte chemotactic protein-1 (MCP-1) |
| | monocyte chemotactic protein-2 (MCP-2) |
| | E-selectin |
| Cytokines | osteopontin |
| | interleukin-15 (IL-15) |
| | prointerleukin-1β |
| | interleukin-1 (IL-1) receptor |
| Innate immunity | complement component Clr |
| | complement component 2 |
| | complement control protein factor |
| | mannose receptor-1 |
| Apoptosis | TNF receptor-1 associated protein (TRADD) |
| | TNF-related apoptosis inducing ligand (TRAIL) |
| | caspase-like apoptosis regulatory protein-2 |
| | apoptotic cysteine Mch4 |

It has been known for over four decades that embryonic tissues are less immunogenic compared to their adult counterparts (Medawar, P.B., 1953. Symp. Soc. Exp. Biol. 7, 320-323). Thus, the presently described definition of the earliest time point in human or porcine renal gestation during which normal differentiation and subsequent renal function are possible may also pinpoint the ideal time for harvesting the tissue least prone to immune rejection by alloimmune/xenoimmune responses. Accordingly, graft acceptance may reflect the progressive development of a complex array of cell surface molecules and soluble factors that determine immune recognition in the gestational organ. In the presently described results, microarray analysis established that development of immunological maturity in the human kidney is a rather late event in gestation since early gestational stage renal tissues are restricted in expression of multiple immunity related genes. Thus, 13 of the 57 genes were unexpectedly found to be significantly upregulated in adult versus gestational stage renal tissues belong to the HLA class I and class II systems. In addition, molecules that mediate trafficking of leukocytes, such as the chemokines RANTES and MCP-1 (Nelson, P.J. and Krensky, A.M., 2001. Immunity 14, 377-386), the adhesion molecule E-selectin (Tedder, T.F. et al., 1995. FASEB J. 9, 866-873), pro-inflammatory cytokines such as osteopontin (O'Regan, A.W. et al., 2000. Immunol. Today 21, 475-478; Ashkar, S. et al., 2000. Science 287, 860-863; Xie, Y. et al., 2001. Kidney Int. 60, 1645-1657) and complement genes known to be associated with innate immunity (Pratt, J.R. et al., 2002. Nature Med. 8, 582-587), were also unexpectedly found to be associated with the reduced immunogenicity of early gestational stage renal tissues relative to more mature tissues.

The immunogenicity of the developing renal tissues was evaluated using two different immunological models. In the first model, grafts are implanted in immunodeficient reconstituted with human PBMCs. The significant level of human specific immunity generated in this model following infusion of human PBMCs has been well documented (Segall, H. et al., 1996. Blood 88, 721-730; Reisner, Y. and Dagan, S., 1998. Trends Biotechnol. 16, 242-246). In this model, both primary and secondary infusions of human PBMCs, obtained from separate donors and hence representing two independent T cell repertoires, were not capable of rejecting grafts derived from early gestational stage human or porcine renal tissues. Whereas global gene analysis indicated that immune tolerance of such early gestational stage tissue derived grafts is likely associated with downregulation of multiple immune pathways, the reduction in CD40 and B7-1 expression observed in such grafts implies a possible absence or immaturity of donor hematopoietic antigen-presenting cells. In addition, the reduced immunogenicity could also be associated with the observed depletion of donor endothelial cells, shown recently to perform as antigen-presenting cells and/or as targets for T cell mediated cytotoxicity in direct allorecognition (Kreisel, D. et al., 2002. Nature Med. 8, 233-239). Allogeneic tissue engineered human skin, devoid of donor endothelial cells, and thereby limited in its antigen-presenting capabilities, has been shown to perform similarly to the early gestational stage renal tissues in a humanized model of skin rejection (Briscoe, D.M. et al., 1999. Transplantation 67, 1590-1599).

In the second model, renal tissues were transplanted into normal immunocompetent hosts. Rejection in such hosts can be triggered by donor antigen presenting cells transferred in the implant, or, alternatively, by cross priming against host antigen presenting cells loaded with donor antigens in a fashion similar to the normal process for the presentation of bacterial or viral antigens (Sayegh, M.H. and Turka, L.A., 1998. N. Engl. J. Med. 338, 1813-1821; Benichou, G., 1999. J. Immunol. 162, 352-358). Because the early gestational stage renal tissues possibly lack mature antigen presenting cells, in addition to a relative reduction in homing receptors and specific cytokines or chemokines, the hypothesis that blockade of cross priming may be sufficient to alleviate the observed rejection of these implants was tested. Results indicated that immune rejection of early gestational stage renal tissue grafts could be obviated by short course co-stimulatory blockade with CTLA4-Ig, a protocol that failed to prevent rejection of the developed adult renal tissue derived grafts. Such results highlighted the reduced immunogenicity of early gestational stage tissues relative to later gestational stage tissues. These results may be extrapolated and used for designing immunosuppressive regimens for transplantation of both allogeneic and xenogeneic developing renal organs/tissues in human subjects.

Finally, since the early gestational stage renal tissues generate functional renal organs producing urine separately from the host, such grafts can be used in combination with urinary anastomosis with the host urinary system to treat kidney diseases, for example to correct biochemical aberrations in a uremic individual. Increasing the number of transplants and/or administering specific human growth factors can be used to support functional replacement.

***Conclusion:*** The above described results unexpectedly and convincingly demonstrated that 7- to 8-week gestational stage human organ/tissue derived grafts, or 20- to 28-day gestational stage porcine organ/tissue derived grafts are capable of generating, in the absence of graft-derived teratomas, structurally and functionally differentiated, host vascularized organs/tissues which are optimally tolerated by alloreactive/xenoreactive human lymphocytes. In particular, these results unexpectedly and convincingly demonstrated that human or porcine renal tissue derived grafts at the aforementioned respective gestational stages have all such capacities, including that of generating urine producing renal organs.

As such, the above described general organ/tissue transplantation method is overwhelmingly superior to all such prior art methods since it overcomes the critical limitations of the latter; namely: (i) use of organ/tissue grafts unsatisfactorily tolerated by allogeneic/xenogeneic human lymphocytes; (ii) incapacity of organ/tissue grafts to generate structurally and functionally differentiated organs/tissues, in particular incapacity of renal grafts to differentiate into urine producing renal organs; and/or (iii) use of organ/tissue grafts not available in sufficient quantities.

### Reference EXAMPLE 2

### Treatment of human renal disease by transplantation of early gestational stage human or porcine renal organs/tissues without or with minimal immunosuppression of graft recipients

As shown in Example 1 of the Examples section above, 7- to 8-week gestational stage human organ/tissue derived grafts, or 20- to 28-day gestational stage porcine organ/tissue derived grafts transplanted into a host are capable of generating structurally and functionally differentiated organs/tissues optimally tolerated by alloreactive/xenoreactive human lymphocytes. In particular, it was shown therein that human or porcine renal transplants at the aforementioned respective gestational stages, exhibit all such capacities, including that of generating urine producing renal organs. Thus, while conceiving the present invention, it was hypothesized that transplantation of human or porcine organ/tissue derived grafts at the aforementioned respective gestational stages, could be used to treat diseases of such organs/tissues in humans. In particular, it was hypothesized that transplantation of the aforementioned renal grafts could be used to treat renal disease in humans, as described below.

### Materials and Methods:

A suitable quantity of 7- to 8-week gestational stage human renal tissue or of 20- to 28-day gestational stage porcine renal tissue is harvested as described in Example 1, above, and a therapeutically effective number of grafts are implanted into a suitable anatomical location for kidney transplantation in a human subject having a kidney disease treatable by kidney transplantation. Optionally, short course costimulation blockade treatment is administered to the subject in the form of CTLA4-Ig administration, as described in Example 1 above. Growth and differentiation of graft(s) into functional renal organ(s) is monitored until production of urine is detected, at which time urinary anastomosis is performed between the graft and the subject's urinary system so as to allow drainage of graft produced urine via the urinary system of the subject. Alternately, or in conjunction with urinary anastomosis, drainage of graft produced urine is effected via a catheter, as described above in Example 1 of the Examples section, above.

### Reference EXAMPLE 3

### Transplantation of early gestational stage human and animal pancreatic grafts into a host generates pancreatic organs/tissues displaying 10-fold growth

As described in Example 1 of the Examples section above, early gestational stage human or porcine organs/tissues transplanted into a host are capable of generating structurally and functionally differentiated, host-integrated organs/tissues optimally tolerated by alloreactive/xenoreactive human lymphocytes. Thus, while conceiving the present invention, it was hypothesized that transplanting early gestational stage human or animal pancreatic organs/tissues into a host will generate pancreatic organs/tissues displaying significant development, as follows.

### Materials and Methods:

***Donor pancreatic tissues:*** Human 12- to 16-week gestational stage pancreatic tissues were obtained following curettage, with warm ischemia time of less than 30 minutes. After dissection, the pancreatic tissues were kept at 4 °C in UW solution for less than 45 minutes in sterile conditions. The study protocol was approved by the hospital (Kaplan Medical Center, Rehovot, Israel) Helsinki committee.

Animal pancreatic tissues at 12- to 14-day gestational stage were microdissected from mouse embryos under the light microscope. Tissues were kept at 4 °C in PMRI 1640 medium solution prior to transplantation.

***Transplantation procedure:*** Transplantation of human and animal pancreatic organs/tissues at early stages of gestational development was performed as described in Example 1 of the Examples section above with modifications. For transplantation under the renal capsule, host kidney is exposed through a left lateral incision, a 1.5 millimeter incision is made at the caudal end of the renal capsule, and donor pancreatic tissues are grafted under the renal capsule in [1-2] × [1-2] millimeter fragments.

***Analysis of transplant development:*** Growth and development of transplanted pancreatic organs/tissues was analyzed as described in Example 1 of the Examples section above.

### Experimental Results:

Four 12- to 16-week gestational stage human pancreatic organ/tissue derived grafts were transplanted under the renal capsule in 4 SCID and 4 normal mice. Each fragment size at transplantation was 1-2 millimeters in diameter. In all immunocompetent mice rejection was detected beginning at 5 days after transplantation as determined via histological analysis indicating graft necrosis and tissue destruction. In all immunodeficient mice, graft acceptance was observed, as determined by growth of the graft and the absence of signs of rejection upon histological and macroscopic examination. In a 12-week gestational stage human pancreatic tissue derived graft harvested at 8 weeks posttransplantation, graft size had increased 10 fold (2 × 2 millimeters pre-transplantation to 8 × 5 millimeters at harvesting; Figure 13).

Mouse 14-, 13-, and 12-day gestational stage pancreatic organs/tissues were transplanted under the renal capsule in immunodeficient syngeneic (Balb/c) mice. In a 12-day gestational stage tissue derived graft harvested 2 weeks after transplantation, graft size had increased 10 fold (1 × 1 millimeter pretransplantation to 5 × 3 millimeters posttransplantation).

***Conclusion:*** Early gestational stage human or animal pancreatic organs/tissues transplanted into hosts generate pancreatic organs/tissues displaying significant development.

### EXAMPLE 4

### Generation of diabetic mice

***Methods and Materials:*** Diabetes is induced in mouse hosts by streptozotocin treatment, as previously described (Soria et al., 2000. Diabetes 49, 1-6). Briefly, diabetes is induced in mouse hosts via a single intraperitoneal injection of 200 mg streptozotocin (Sigma) freshly dissolved in citrate buffer (pH = 4.5) per kilogram body weight. Onset of diabetes is then confirmed and monitored by the presence of weight loss, polyuria, and blood glucose levels of less than 500 mg/dl. Blood for glucose tests is obtained by tail snipping and measured between 9 and 11 A.M. under non-fasting conditions and analyzed with a portable glucose meter. Two weeks following injection of streptozotocin, diabetic recipients are engrafted with donor pancreatic tissues, and glucose levels are monitored as described above in order to ascertain restoration of glycemic control.

### Reference EXAMPLE 5

### Treatment of diabetes by transplantation of early gestational stage human or porcine pancreatic tissue into diabetic human recipients without or with minimal immunosuppression of recipients

Diabetes is a disease of tremendous medical and economic impact, however treatment of this disease by daily injection of insulin, the standard prior art therapy, does not satisfactorily prevent or alleviate its debilitating or lethal consequences. An attempted approach to overcome this limitation has been treatment of diabetes by transplantation of adult cadaveric donor pancreatic islets. However, this strategy cannot be routinely practiced due to the insufficient numbers of immunologically matching allogeneic donor pancreases from which to isolate the sufficient numbers of islets required. As shown in Example 1 of the Examples section above, 7- to 8-week gestational stage human organ/tissue derived grafts, or 20- to 28-day gestational stage porcine organ/tissue derived grafts transplanted into hosts generate structurally and functionally differentiated, organs/tissues of graft type optimally tolerated by alloreactive/xenoreactive human lymphocytes. As shown in Example 3 of the Examples section above, transplantation of early gestational stage human or animal pancreatic organs/tissues into a host generates pancreatic organs/tissues displaying significant development. Thus, while conceiving the present invention, it was hypothesized that transplantation of human or porcine pancreatic organs/tissues at the aforementioned respective gestational stages could be used to treat diabetes in humans, as described below.

### Materials and Methods:

A suitable quantity of pancreatic islets from human 7- to 8-week gestational stage pancreatic tissue or of 20- to 28-day gestational stage porcine pancreatic tissue is isolated and transplanted into a diabetic human recipient according to state-of-the-art techniques, as previously described [National Institutes of Diabetes and Digestive and Kidney Diseases (NIDDK; http://www.niddk.nih.gov)]. Briefly, ultrasound is used to guide placement of a small catheter through the upper abdomen and into the liver of the subject. The pancreatic islets are then injected through the catheter into the liver. The recipient receives a local anesthetic, however if the recipient cannot tolerate local anesthesia, general anesthesia is used, and the transplant is performed through a small incision. Typically, for a 70 kilogram recipient, about one million pancreatic islets are administered. It takes some time for the administered cells to attach to new blood vessels and begin releasing insulin, and hence following transplantation, the blood glucose levels of the recipient are closely monitored and exogenous insulin is administered as needed until glycemic control is achieved. Optionally, to prevent graft rejection, the recipient is temporarily immunosuppressed by short course blockade of costimulation in the form of CTLA4-Ig administration, as described in Example 1 above.

### Reference EXAMPLE 6

### Transplantation of 7-week gestational stage human or 28-day gestational stage porcine liver grafts generates, in the absence of graft-derived teratomas, structurally and functionally differentiated hepatic organ/tissues which will be optimally tolerated by alloreactive/xenoreactive human lymphocytes: basis for optimal treatment of liver diseases

Allogeneic donor liver organ/tissue transplantation remains the optimal therapeutic option in case of liver failure. However, therapeutic transplantation of liver organ/tissue grafts derived from an allogeneic donor is often impossible to implement due to haplotype-matching barriers. Moreover, even when a matched donor is found, in order to prevent graft rejection such transplantation requires permanent graft recipient immunosuppression, usually via administration of toxic immunosuppressant drugs such as cyclosporin A. Such immunosuppressive treatments contribute to the drawbacks of allogeneic transplantation, since these are often unsuccessful at preventing graft rejection in the short term, and are usually incapable of indefinitely preventing graft rejection. An alternative to allograft transplantation involves transplantation of xenografts, in particular porcine grafts, which are considered the optimal animal alternative to human grafts. However, xenografts generally cannot be used for transplantation due to highly suboptimal tolerance of such grafts by human lymphocytes. Thus, hepatic organs/tissues suitable for therapeutic transplantation in humans and tolerated by non syngeneic human lymphocytes, and adequate sources of such organs/tissues, are highly desired. One potent strategy for providing hepatic organs/tissues for transplantation involves using grafts of such organs/tissues at early developmental stages, since it has been demonstrated that the earlier the developmental stage of an organ/tissue, the better it is tolerated when transplanted into a non syngeneic host. However, to date, satisfactory growth and differentiation of developing, non syngeneic hepatic organ/tissue grafts, and satisfactory immunological tolerance of such grafts by human lymphocytes in the absence of graft-derived teratomas has not been achieved.

While conceiving the present invention, it was hypothesized that there exists a developmental stage during which hepatic organs/tissues are sufficiently differentiated to be committed to liver specific development in the absence of graft-derived teratomas while being sufficiently undifferentiated so as to be optimally tolerated when transplanted into a non syngeneic host. While reducing the present invention to practice, the existence of specific gestational stages during which human and porcine hepatic organs/tissues can be transplanted into a host so as to generate, in the absence of graft-derived teratomas, structurally and functionally differentiated hepatic organs/tissues which will be optimally tolerated by alloreactive/xenoreactive human lymphocytes were unexpectedly uncovered, as described below.

### Materials and Methods:

***Harvesting of human gestational stage hepatic organs*/*tissues:*** Human gestational stage hepatic organs/tissues for transplantation were obtained by extraction of organ/tissue fragments following voluntary abortions performed mechanically by aspiration at a gestational stage of 7 weeks, after obtaining informed consent. The warm ischemia time of the harvested samples was kept at under 30 minutes, and following dissection, the organ precursors were kept at 40 degrees centigrade in UW solution or PBS for less than 45 minutes under sterile conditions. The study protocol was approved by the hospital (Kaplan Medical Center, Rehovot, Israel) Helsinki committee.

***Harvesting of porcine gestational stage hepatic organs*/*tissues:*** Porcine gestational stage hepatic organs/tissues for transplantation were obtained with the assistance of the Lahav Institute for animal research, Kibbutz Lahav. Developing tissues were harvested at a gestational stage of 28 days from pregnant sows operated on under general anesthesia. The study protocol was approved by the local institute's Ethics Committee. Tissues for transplantation were extracted under a light microscope and were kept in sterile conditions at 40 degrees centigrade for about two hours in RPMI 1640 (Biological Industries, Bet HaEmek, Israel) before transplantation.

***Transplantation procedures:*** Transplantations were performed in NOD/SCID recipients under general anesthesia induced by intraperitoneal injection of 2.5 % Avertin in PBS (10 ml/kg). For transplantation under the renal capsule, the host kidney was exposed through a left lateral incision. A 1.5-mm incision was made at the caudal end of the renal capsule, and 1-2 mm-diameter fragments of gestational stage liver grafts were implanted under the renal capsule. For intra-spleen transplantation, gestational stage liver tissue was minced to 1 mm fragments in sterile PBS. The host spleen was exposed through a left lateral incision and a suspension of 1 mm-diameter fragments of gestational stage liver was injected into the lower pole of the spleen. Hemostasis was achieved by suture ligation below the injection site.

***Histological analysis:*** Tissues were fixed by overnight incubation in 4 percent paraformaldehyde in PBS, the fixed tissues were processed through graded alcohols, through xylenes, and paraffin-embedded. Four micron-thick sections of embedded tissues were cut and mounted on positively charged glass slides. The slide-mounted tissue sections were deparaffinized in xylene following rehydration in graded alcohols. Endogenous peroxidase was quenched in 0.6 percent hydrogen peroxide in 70 percent methanol for 20 minutes. Antigen retrieval by microwave boiling or protease pretreatment was applied when needed. For immunostaining, slides were incubated in a humidified chamber for 60 minutes with primary antibody, following application of DAKO Envision TM+ system, horseradish peroxidase (HRP). Diaminobenzidine (DAB) or aminoethylcarbasole (AEC) reagents were used as chromogens. The slides were hematoxylin counterstained and mounted.

Anti porcine albumin antibody was obtained from Bethyl Laboratories; anti Ki67 antibody was used as a marker of cell proliferation, and periodic acid-Schiff (PAS) dye was used as a marker of glycogen synthesis.

Further details regarding the experimental protocols described in this Example may be provided under Materials and Methods in Example 1 of this section, above.

### Experimental Results:

***Porcine 28-day, but not 21-day, gestational stage hepatic xenografts engraft and display functional and structural hepatic differentiation:*** Grafts derived from 21-day gestational stage porcine liver transplanted into NOD/SCID mouse recipients bearing xenoreactive human PBMCs showed clear teratoma development (Figure 14a) with extensive cartilage differentiation (Figure 14b) when examined 7 weeks posttransplantation, clearly indicating for the first time that the optimal gestational stage for transplantation of such tissues to obtain suitable hepatic differentiation in the absence of teratomas is greater than day 21 of gestation. In contrast, grafts derived from 28-day gestational stage porcine liver transplanted into spleens of such mice which were examined 6 weeks posttransplantation displayed liver specific structural and functional differentiation, 6 weeks posttransplantation. Graft tissue sections stained with H&E, periodic acid-Schiff, or anti porcine albumin antibody (Figures 15a,15b, and 15c, respectively) all displayed marked differentiation of hepatic lobular structures. Liver functionality was demonstrated by glycogen synthesis/storage, and by albumin synthesis (Figures 15b and 15c, respectively). Furthermore, staining of sections with antibody specific for the proliferation marker Ki67 demonstrated the clear proliferative capacity of the graft-derived hepatocytes. Similarly, such grafts transplanted under the renal capsule of such recipients analyzed 6 weeks posttransplantation also showed hepatic function, as evidenced by glycogen synthesis/storage, and albumin synthesis, as determined by staining of graft tissue sections using periodic acid-Schiff, and anti porcine albumin antibody (Figures 16a and 16b, respectively).

***Human 7-week gestational stage hepatic allografts engraft and display functional and structural hepatic differentiation:*** Grafts derived from 7-week gestational stage human liver transplanted under the renal capsule of NOD/SCID mice displayed liver specific structural and functional differentiation, 6 weeks posttransplantation. Graft tissue sections stained with H&E or periodic acid-Schiff displayed marked differentiation of bile ducts, and glycogen synthesis/storage (Figures 17a, and 17b, respectively).

***Conclusion:*** The above described results convincingly demonstrate that 7-week gestational stage human liver derived grafts, or 28-day, but not 21-day, gestational stage porcine liver derived grafts are capable of generating, in the absence of graft-derived teratomas, structurally and functionally differentiated hepatic organs/tissues which will be optimally tolerated by allogeneic/xenogeneic human lymphocytes. As such, the above described results demonstrate that 7-week gestational stage human liver derived grafts, or 28-day porcine liver derived grafts, can be used for optimally performing therapeutic transplantation of allogeneic and xenogeneic hepatic tissues/organs, respectively, relative to all prior art methods.

### reference EXAMPLE 7

### Transplantation of 8-week gestational stage human, or 27- to 28-day gestational stage porcine, pancreatic grafts generates, in the absence of graft-derived teratomas, insulin-producing pancreatic organs/tissues which will be optimally tolerated by alloreactive/xenoreactive human lymphocytes: basis for optimal treatment of diabetes

Allogeneic donor pancreatic organ/tissue transplantation remains the optimal therapeutic option in case of pancreatic failure. However, therapeutic transplantation of pancreatic organ/tissue grafts derived from an allogeneic donor is often impossible to implement due to haplotype-matching barriers. Moreover, even when a matched donor is found, in order to prevent graft rejection such transplantation requires permanent graft recipient immunosuppression, usually via administration of toxic immunosuppressant drugs such as cyclosporin A. Such immunosuppressive treatments contribute to the drawbacks of allogeneic transplantation, since these are often unsuccessful at preventing graft rejection in the short term, and are usually incapable of indefinitely preventing graft rejection. An alternative to allograft transplantation involves transplantation of xenografts, in particular porcine grafts, which are considered the optimal animal alternative to human grafts. However, xenografts generally cannot be used for transplantation due to highly suboptimal tolerance of such grafts by human lymphocytes. Thus, pancreatic organs/tissues suitable for therapeutic transplantation in humans and tolerated by non syngeneic human lymphocytes, and adequate sources of such organs/tissues, are highly desired. One potent strategy for providing pancreatic organs/tissues for transplantation involves using grafts of such organs/tissues at early developmental stages, since it has been demonstrated that the earlier the developmental stage of an organ/tissue, the better it is tolerated when transplanted into a non syngeneic host. However, to date, generation of pancreatic graft-derived tissues/organs displaying satisfactory growth and differentiation in the absence of graft-derived teratomas, and satisfactory immunological tolerance by alloreactive/xenoreactive human lymphocytes, without or with minimal immunosuppression, has not been achieved.

While conceiving the present invention, it was hypothesized that there exists a developmental stage during which pancreatic organs/tissues are sufficiently differentiated to be committed to pancreas specific development in the absence of graft-derived teratomas while being sufficiently undifferentiated so as to be optimally tolerated when transplanted into a non syngeneic host. While reducing the present invention to practice, the existence of specific gestational stages during which human or porcine pancreatic organs/tissues can be transplanted into a host so as to generate, in the absence of graft-derived teratomas, structurally and functionally differentiated insulin-producing organs/tissues which will be optimally tolerated by alloreactive/xenoreactive human lymphocytes were unexpectedly uncovered, as described below.

### Materials and Methods:

***Harvesting of human gestational stage pancreatic organs*/*tissues:*** Human gestational stage pancreatic organs/tissues for transplantation were obtained by extraction of organ/tissue fragments following voluntary abortions performed mechanically by aspiration at a gestational stage of 8 weeks, after obtaining informed consent. The warm ischemia time of the harvested samples was kept at under 30 minutes, and following dissection, the organ precursors were kept at 40 degrees centigrade in UW solution or PBS for less than 45 minutes under sterile conditions. The study protocol was approved by the hospital (Kaplan Medical Center, Rehovot, Israel) Helsinki committee.

***Harvesting of porcine gestational stage pancreatic organs*/*tissues:*** Porcine gestational stage pancreatic organs/tissues for transplantation were obtained with the assistance of the Lahav Institute for animal research, Kibbutz Lahav. Developing tissues were harvested at a gestational stage of 27-28 days from pregnant sows operated on under general anesthesia. The study protocol was approved by the local institute's Ethics Committee. Tissues for transplantation were extracted under a light microscope and were kept in sterile conditions at 40 degrees centigrade for about two hours in RPMI 1640 (Biological Industries, Bet HaEmek, Israel) before transplantation.

***Transplantation procedure:*** Transplantations were performed in Balb/c × NOD/SCID chimeras or NOD/SCID mice under general anesthesia induced by intraperitoneal injection of 2.5 % Avertin in PBS (10 ml/kg). For transplantation under the renal capsule, the host kidney was exposed through a left lateral incision. A 1.5-mm incision was made at the caudal end of the renal capsule, and 1-2 mm-diameter fragments of gestational stage pancreatic tissue were implanted under the renal capsule.

***Histological analysis:*** Anti cytokeratin antibody clone MNF 116 (non cross-reactive with mouse tissues) was used for immunostaining porcine epithelium; and anti insulin antibody and anti human vimentin antibody clone V9 (non cross-reactive with mouse tissues; used for staining human mesenchymal cells) were obtained from DAKO. Tissues were fixed by overnight incubation in 4 percent paraformaldehyde in PBS, the fixed tissues were processed through graded alcohols, through xylenes, and paraffin-embedded. Four micron-thick sections of embedded tissues were cut and mounted on positively charged glass slides. The slide-mounted tissue sections were deparaffinized in xylene following rehydration in graded alcohols. Endogenous peroxidase was quenched in 0.6 percent hydrogen peroxide in 70 percent methanol for 20 minutes. Antigen retrieval by microwave boiling or protease pretreatment was applied when needed. For immunostaining, slides were incubated in a humidified chamber for 60 minutes with primary antibody, following application of DAKO Envision TM+ system, horseradish peroxidase (HRP). Diaminobenzidine (DAB) or aminoethylcarbasole (AEC) reagents were used as chromogens. The slides were hematoxylin counterstained and mounted.

### Experimental Results:

***Transplantation of porcine 27- to 28-day gestational stage pancreatic xenografts en-graft and display functional and structural pancreatic differentiation:*** Grafts derived from 27- to 28-day gestational stage porcine pancreas transplanted under the renal capsule of NOD/SCID mice clearly displayed pancreas specific structural and functional differentiation, 6 weeks posttransplantation. Grafts derived from 28-day gestational stage porcine liver transplanted into spleens of such mice which were examined 5 weeks posttransplantation displayed significant pancreatic growth, as can be seen from a whole graft photograph (Figure 18a). Pancreatic structural differentiation was clearly evident 6 weeks posttransplantation by a graft derived from 27-day gestational stage porcine pancreatic tissue as determined via H&E-stained graft tissue sections which showed differentiation of pancreatic lobular structures with ductal and acinar pancreatic structures (Figures 18b-c). Pancreatic functional differentiation was also evident at 6 weeks posttransplantation in tissue sections of a graft derived from 27-day gestational stage tissue in the form of insulin and pancreatic peptide synthesis (Figures 19a and 19b, respectively). As can further be seen in Figure 19c, immunostaining of a graft derived from 28-day gestational stage porcine pancreatic tissue with anti cytokeratin antibody clearly showed differentiation of graft derived pancreatic ductal epithelia.

***Human 8-week gestational stage pancreatic allografts engraft and display functional and structural pancreatic differentiation:*** Grafts derived from 8-week gestational stage human pancreatic tissue transplanted under the renal capsule of NOD/SCID mice bearing alloreactive human lymphocytes clearly displayed pancreas specific structural and functional differentiation, 6 weeks posttransplantation. Pancreatic functionality of the graft was convincingly demonstrated by differentiation of insulin-positive beta-cells within pancreatic islets (Figures 20a-b). Furthermore, grafts derived from 8-week gestational stage human pancreatic tissue transplanted under the renal capsule of Balb/c x NOD/SCID chimeras bearing alloreactive human PBMCs also clearly displayed pancreas specific structural and functional differentiation, as shown via differentiation of vimentin positive human mesenchymal cells (Figures 20c-d).

***Conclusion:*** The above described results convincingly demonstrate that 8-week gestational stage human, or 27- to 28-day gestational stage porcine, pancreatic tissue derived grafts are capable of generating, in the absence of graft-derived teratomas, structurally and functionally differentiated insulin-producing pancreatic organs/tissues which will be optimally tolerated by alloreactive/xenoreactive human lymphocytes. As such, the above described results demonstrate that 8-week gestational stage human pancreatic tissue derived grafts, or 27- to 28-day porcine pancreatic tissue derived grafts, can be used for optimally performing therapeutic transplantation of allogeneic/xenogeneic pancreatic tissues/organs, respectively, relative to all prior art methods.

### EXAMPLE 8

### Transplantation of 9-week gestational stage human cardiac tissue-derived grafts generates, in the absence of graft-derived teratomas, cells/tissues displaying a proliferative cardiac phenotype which will be optimally tolerated by alloreactive human lymphocytes: basis for optimal treatment of cardiac diseases

Allogeneic donor cardiac organ/tissue transplantation remains the optimal therapeutic option in case of heart failure. However, therapeutic transplantation of cardiac organ/tissue grafts derived from an allogeneic donor is often impossible to implement due to haplotype-matching barriers. Moreover, even when a matched donor is found, in order to prevent graft rejection such transplantation requires permanent graft recipient immunosuppression, usually via administration of toxic immunosuppressant drugs such as cyclosporin A. Such immunosuppressive treatments contribute to the drawbacks of allogeneic transplantation, since these are often unsuccessful at preventing graft rejection in the short term, and are usually incapable of indefinitely preventing graft rejection. Thus, cardiac organs/tissues suitable for therapeutic transplantation in humans and tolerated by non syngeneic human lymphocytes, and adequate sources of such organs/tissues, are highly desired. One potent strategy for providing cardiac organs/tissues for transplantation involves using grafts of such organs/tissues at early developmental stages, since it has been demonstrated that the earlier the developmental stage of an organ/tissue, the better it is tolerated when transplanted into a non syngeneic host. However, to date, satisfactory growth and differentiation of developing, non syngeneic cardiac organ/tissue grafts, and satisfactory immunological tolerance of such grafts by human lymphocytes in the absence of graft-derived teratomas has not been achieved.

While conceiving the present invention, it was hypothesized that there exists a developmental stage during which cardiac organs/tissues are sufficiently differentiated to be committed to heart specific development in the absence graft-derived teratoma formation while being sufficiently undifferentiated so as to be optimally tolerated when transplanted into a non syngeneic host. While reducing the present invention to practice, the existence of specific gestational stages during which human cardiac organs/tissues can be transplanted into a host so as to generate, in the absence of graft-derived teratomas, organs/tissues displaying a proliferative cardiac phenotype which will be optimally tolerated by alloreactive human lymphocytes were unexpectedly uncovered, as described below.

### Materials and Methods:

***Harvesting of human gestational stage cardiac organs*/*tissues:*** Human gestational stage cardiac organs/tissues for transplantation were obtained by extraction of organ/tissue fragments following voluntary abortions performed mechanically by aspiration at a gestational stage of 9 weeks, after obtaining informed consent. The warm ischemia time of the harvested samples was kept at under 30 minutes, and following dissection, the organ precursors were kept at 40 degrees centigrade in UW solution or PBS for less than 45 minutes under sterile conditions. The study protocol was approved by the hospital (Kaplan Medical Center, Rehovot, Israel) Helsinki committee.

***Transplantation procedures:*** Transplantations were performed in NOD/SCID recipients under general anesthesia induced by intraperitoneal injection of 2.5 % Avertin in PBS (10 ml/kg). For transplantation under the renal capsule, the host kidney was exposed through a left lateral incision. A 1.5-mm incision was made at the caudal end of the renal capsule, and 1-2 mm-diameter fragments of the gestational stage heart grafts were implanted under the renal capsule.

***Histological analysis:*** Anti alpha-sarcomeric actin antibody and anti-neurofilament protein antibody, respectively, were used to stain for cardiomyocytic cells and basal ganglionic cells. Tissues were fixed by overnight incubation in 4 percent paraformaldehyde in PBS, the fixed tissues were processed through graded alcohols, through xylenes, and paraffin-embedded. Four micron-thick sections of embedded tissues were cut and mounted on positively charged glass slides. The slide-mounted tissue sections were deparaffinized in xylene following rehydration in graded alcohols. Endogenous peroxidase was quenched in 0.6 percent hydrogen peroxide in 70 percent methanol for 20 minutes. Antigen retrieval by microwave boiling or protease pretreatment was applied when needed. For immunostaining, slides were incubated in a humidified chamber for 60 minutes with primary antibody, following application of DAKO Envision TM+ system, horseradish peroxidase (HRP). Diaminobenzidine (DAB) or aminoethylcarbasole (AEC) reagents were used as chromogens. The slides were hematoxylin counterstained and mounted.

### Experimental Results:

***Human 9-week gestational stage cardiac tissue allografts engraft and display a proliferative cardiac phenotype:*** Grafts derived from 9-week gestational stage human cardiac tissue transplanted under the renal capsule of mice bearing alloreactive human PBMCs clearly displayed a proliferative cardiac phenotype 6 weeks posttransplantation. Cardiac differentiation of the grafts in the form of differentiation of cardiomyocytic cells and basal ganglionic cells was clearly shown in graft tissue sections stained with anti alpha-sarcomeric actin antibody or anti-neurofilament protein antibody (Figures 21a and 21b, respectively).

***Conclusion:*** The above described results convincingly demonstrate that 9-week gestational stage human cardiac tissue derived grafts are capable of generating, in the absence of graft-derived teratomas, graft-derived cells/tissues displaying a proliferative cardiac phenotype which will be optimally tolerated by allogeneic human lymphocytes. As such, the above described results demonstrate that 9-week gestational stage human cardiac organ/tissue derived grafts can be used for optimally performing therapeutic transplantation of allogeneic cardiac organs/tissues, respectively, relative to all prior art methods.

### EXAMPLE 9

### Transplantation of 28-day gestational stage porcine lymphoid organ/tissue-derived grafts generates, in the absence of graft-derived teratomas, well differentiated and vascularized lymphoid mesenchymal/stromal tissues which will be optimally tolerated by xenoreactive human lymphocytes: basis for optimal treatment of genetic and/or hematological diseases

Transplantation of lymphoid organs/tissues capable of generating lymphoid stroma of xenogeneic origin, in particular of porcine origin, which are considered the optimal animal alternative to human grafts, is a potentially optimal therapeutic option for hematological and/or genetic diseases, including those associated with a coagulation disorder/clotting factor deficiency/malfunction such as hemophilia, those associated with an enzyme deficiency/malfunction, such as Gaucher disease, and/or those associated with a lymphoid stroma defect. However, xenografts generally cannot be used for transplantation due to highly suboptimal tolerance of such grafts by human lymphocytes. Thus, lymphoid organs/tissues suitable for therapeutic transplantation in humans and tolerated by xenogeneic human lymphocytes, and adequate sources of such organs/tissues, are highly desired. One potent strategy for providing such lymphoid organs/tissues for transplantation involves using grafts of such organs/tissues at early developmental stages, since it has been demonstrated that the earlier the developmental stage of an organ/tissue, the better it is tolerated when transplanted into a non syngeneic host. However, to date, satisfactory growth and differentiation of developing, xenogeneic lymphoid organ/tissue grafts, and satisfactory immunological tolerance of such grafts by xenoreactive human lymphocytes in the absence of graft-derived teratomas has not been achieved.

While conceiving the present invention, it was hypothesized that there exists a developmental stage during which lymphoid organs/tissues are sufficiently differentiated to be committed to lymphoid organ/tissue specific development in the absence of graft-derived teratomas while being sufficiently undifferentiated so as to be optimally tolerated when transplanted into a non syngeneic host. While reducing the present invention to practice, the existence of specific gestational stages during which porcine lymphoid organs/tissues can be transplanted into a host so as to generate, in the absence of graft-derived teratomas, well-differentiated and vascularized lymphoid mesenchymal/stromal tissues which will be optimally tolerated by xenoreactive human lymphocytes were unexpectedly uncovered, as described below.

### Materials and Methods:

***Harvesting of porcine gestational stage splenic organs*/*tissues:*** Porcine gestational stage splenic organs/tissues for transplantation were obtained with the assistance of the Lahav Institute for animal research, Kibbutz Lahav. Developing tissues were harvested at a gestational stage of 28 days from pregnant sows operated on under general anesthesia. The study protocol was approved by the local institute's Ethics Committee. Tissues for transplantation were extracted under a light microscope and were kept in sterile conditions at 40 degrees centigrade for about two hours in RPMI 1640 (Biological Industries, Bet HaEmek, Israel) before transplantation.

***Transplantation procedures:*** Splenic tissue graft transplantations were performed in NOD/SCID mice under general anesthesia induced by intraperitoneal injection of 2.5 % Avertin in PBS (10 ml/kg). For transplantation under the renal capsule, the host kidney was exposed through a left lateral incision. A 1.5-mm incision was made at the caudal end of the renal capsule, and 1-2 mm-diameter fragments of gestational stage splenic tissue grafts were implanted under the renal capsule.

***Histological analysis:*** H&E staining was used to identify splenic differentiation. Tissues were fixed by overnight incubation in 4 percent paraformaldehyde in PBS, the fixed tissues were processed through graded alcohols, through xylenes, and paraffin-embedded. Four micron-thick sections of embedded tissues were cut and mounted on positively charged glass slides. The slide-mounted tissue sections were deparaffinized in xylene following rehydration in graded alcohols. Endogenous peroxidase was quenched in 0.6 percent hydrogen peroxide in 70 percent methanol for 20 minutes.

### Experimental Results:

***Porcine 28-day gestational stage splenic xenografts engraft and display splenic differentiation:*** Analysis, 6 weeks posttransplantation, of H&E-stained sections of grafts derived from 28-day gestational stage porcine splenic tissue transplanted under the renal capsule of mice clearly showed generation of well-differentiated and vascularized lymphoid mesenchymal/stromal tissues which will be optimally tolerated by xenogeneic human lymphocytes (Figure 22).

***Conclusion:*** The above described results convincingly demonstrate that 28-day, gestational stage porcine lymphoid organ/tissue grafts are capable of generating, in the absence of graft-derived teratomas, graft-derived well-differentiated and vascularized lymphoid mesenchymal/stromal tissues which will be optimally tolerated by xenoreactive human lymphocytes. As such, the above described results demonstrate that 28-day porcine lymphoid organ/tissue grafts, can be used for optimally performing therapeutic transplantation of xenogeneic lymphoid tissues relative to all prior art methods, for example for treatment of hematological and/or genetic diseases, including those associated with a coagulation disorder/clotting factor deficiency/malfunction such as hemophilia, those associated with an enzyme deficiency/malfunction, such as Gaucher disease, and/or those associated with a lymphoid stroma defect

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subrombination.

## Claims

1. A gestational stage porcine pancreatic whole or partial organ graft for use in the treatment of a pancreatic disorder associated with a pathological organ or tissue physiology or morphology, the pancreatic whole or partial organ graft selected not expressing or presenting at least one molecule capable of stimulating or enhancing an immune response in a subject, wherein said at least one molecule is a lymphocyte coreceptor or lymphocyte coreceptor ligand, and wherein said porcine whole or partial organ graft is selected at a specific stage of differentiation corresponding to 20 to 42 days of gestation.

2. The pancreatic whole or partial organ graft according to claim 1 for use in the treatment of a pancreatic disorder associated with a pathological organ or tissue physiology or morphology of claim 1, wherein said subject is a human.

3. The pancreatic whole or partial organ graft according to claim 1 for use in the treatment of a pancreatic disorder associated with a pathological organ or tissue physiology or morphology of claim 1, further comprising the use of an immunosuppressant drug, thereby promoting engraftment of said whole or partial organ graft in said subject.

4. The pancreatic whole or partial organ graft according to claim 1 for use in the treatment of a pancreatic disorder associated with a pathological organ or tissue physiology or morphology of claim 3, wherein said immunosuppressant drug is capable of blocking binding of a lymphocyte coreceptor with a ligand of said lymphocyte coreceptor.

5. The pancreatic whole or partial organ graft according to claim 1 for use in the treatment of a pancreatic disorder associated with a pathological organ or tissue physiology or morphology of claim 4, wherein said immunosuppressant drug is CTLA4-Ig.

6. The pancreatic whole or partial organ graft according to claim 1 for use in the treatment of a pancreatic disorder associated with a pathological organ or tissue physiology or morphology of claim 1, wherein said lymphocyte coreceptor or lymphocyte coreceptor ligand is selected from the group consisting of B7-1, CD40, and CD40L.

7. The pancreatic whole or partial organ graft according to claim 1 for use in the treatment of a pancreatic disorder associated with a pathological organ or tissue physiology or morphology of claim 1, wherein said selecting said whole or partial organ graft is effected via RT-PCR analysis of said whole or partial organ.

8. The pancreatic whole or partial organ graft according to claim 1 for use in the treatment of a pancreatic disorder associated with a pathological organ or tissue physiology or morphology of claim 1, wherein said pancreatic disorder is diabetes.

9. The pancreatic whole or partial organ graft according to claim 1 for use in the treatment of a pancreatic disorder associated with a pathological organ or tissue physiology or morphology of claim 1, wherein said whole or partial organ graft is a pancreatic islet graft.

10. The pancreatic whole or partial organ graft according to claim 1 for use in the treatment of a pancreatic disorder associated with a pathological organ or tissue physiology or morphology of claim 1, wherein said porcine whole or partial organ graft is selected at a specific stage of differentiation corresponding to 42 days of gestation.

11. The pancreatic whole or partial organ graft according to claim 1 for use in the treatment of a pancreatic disorder associated with a pathological organ or tissue physiology or morphology of claim 1, wherein said graft is implanted in the sub-cutis.

12. The pancreatic whole or partial organ graft according to claim 1 for use in the treatment of a pancreatic disorder associated with a pathological organ or tissue physiology or morphology of claim 1, wherein said graft is unplanted in an anatomical location of said subject selected from the group consisting of the portal vein, the liver, the pancreas, the renal capsule, the testicular fat, the sub-cutis and the intra-abdominal space.

13. The pancreatic whole or partial organ graft according to claim 1 for use in the treatment of a pancreatic disorder associated with a pathological organ or tissue physiology or morphology of claim 1, wherein when transplanting said graft, the organ or tissue displaying pathological physiology or morphology is removed.

14. The pancreatic whole or partial organ graft according to claim 1 for use in the treatment of a pancreatic disorder associated with a pathological organ or tissue physiology or morphology of claim 1, wherein said pancreatic whole or partial organ graft is a pancreatic islet graft.

## Patentansprüche

1. Ganzes oder teilweises Bauchspeicheldrüsentransplantat vom Schwein im Gestationsstadium zum Einsatz bei der Behandlung einer mit einer pathologischen Organ- oder Gewebephysiologie oder -morphologie verbundenen Bauchspeicheldrüsenstörung, wobei das gewählte ganze oder teilweise Bauchspeicheldrüsenimplantat mindestens ein Molekül, das eine Immunreaktion bei einem Probanden hervorrufen oder verstärken kann, nicht exprimiert oder aufweist, wobei das mindestens eine Molekül ein Lymphozytenkorezeptor oder -korezeptorligand ist, und wobei das ganze oder teilweise Organimplantat vom Schwein in einem bestimmten Differenzierungsstadium ausgewählt wird, das dem 20. bis 42, Gestationstag entspricht.

2. Ganzes oder teilweises Organimplantat nach Anspruch 1 zum Einsatz bei der Behandlung einer mit einer pathologischen Organ- oder Gewebephysiologie oder - morphologie verbundenen Bauchspelcheldrüsenstörung, wobei der Proband ein Mensch ist.

3. Ganzes oder teilweises Organimplantat nach Anspruch 1 zum Einsatz bei der Behandlung einer mit einer pathologischen Organ- oder Gewebephysiologie oder - morphologie verbundenen Bauchspeicheldrüsenstörung, ferner umfassend den Einsatz eines Immunosuppressivum, wodurch die Einpflanzung des ganzen oder teilweisen Organimplantats im Probanden gefördert wird.

4. Ganzes oder teilweises Organimplantat nach Anspruch 1 zum Einsatz bei der Behandlung einer mit einer pathologischen Organ- oder Gewebephysiologie oder - morphologie verbundenen Bauchspeicheldrüsenstörung nach Anspruch 3, wobei das Immunosuppressivum die Bindung eines Lymphozytenkorezeptors mit einem Liganden des Lymphozytenkorezeptors verhindern kann.

5. Ganzes oder teilweises Organimplantat nach Anspruch 1 zum Einsatz bei der Behandlung einer mit einer pathologischen Organ- oder Gewebephysiologie oder - morphologie verbundenen Bauchspeicheldrüsenstörung nach Anspruch 4, wobei es sich beim Immunosuppressivum um CTLA4-Ig handelt.

6. Ganzes oder teilweises Organimplantat nach Anspruch 1 zum Einsatz bei der Behandlung einer mit einer pathologischen Organ- oder Gewebephysiologie oder - morphologie verbundenen Bauchspeicheldrüsenstörung, wobei der Lymphozytenkorezeptor oder Lymphozytenkorezeptorenligand aus der Gruppe von B7-1, CD40 und CD40L gewählt ist.

7. Ganzes oder teilweises Organimplantat nach Anspruch 1 zum Einsatz bei der Behandlung einer mit einer pathologischen Organ- oder Gewebephysiologie oder - morphologie verbundenen Bauchspeicheldrüsenstörung, wobei die Auswahl des ganzen oder teilweisen Organimplantats mittels einer RT-PCR-Analyse des Organs bzw. Organteils erfolgt.

8. Ganzes oder teilweises Organimplantat nach Anspruch 1 zum Einsatz bei der Behandlung einer mit einer pathologischen Organ- oder Gewebephysiologie oder - morphologie verbundenen Bauchspeicheldrüsenstörung, wobei es sich bei der Bauchspeicheldrüsenstörung um Diabetes handelt.

9. Ganzes oder teilweises Organimplantat nach Anspruch 1 zum Einsatz bei der Behandlung einer mit einer pathologischen Organ- oder Gewebephysiologie oder - morphologie verbundenen Bauchspeicheldrüsenstörung, wobei es sich beim ganzen oder teilweisen Organimplantat um ein Bauchspeicheldrüsen-Inselimplantat handelt.

10. Ganzes oder teilweises Organimplantat nach Anspruch 1 zum Einsatz bei der Behandlung einer mit einer pathologischen Organ- oder Gewebephysiologie oder - morphologie verbundenen Bauchspeicheldrüsenstörung, wobei das ganze oder teilweise Organimplantat vom Schwein in einem bestimmten Differenzierungsstadium ausgewählt wird, das dem 42. Gestationstag entspricht.

11. Ganzes oder teilweises Organimplantat nach Anspruch 1 zum Einsatz bei der Behandlung einer mit einer pathologischen Organ- oder Gewebephysiologie oder - morphologie verbundenen Bauchspeicheldrüsenstörung, wobei das Implantat in die Subkutis implantiert wird.

12. Ganzes oder teilweises Organimplantat nach Anspruch 1 zum Einsatz bei der Behandlung einer mit einer pathologischen Organ- oder Gewebephysiologie oder - morphologie verbundenen Bauchspeicheldrüsenstörung, wobei das Implantat an einer anatomischen Stelle des Probanden implantiert wird, die aus der Gruppe von Pfortader, Leber, Bauchspeicheldrüse, Nierenkapsel, Hodenfett, Subkutis und Bauchraum gewählt ist.

13. Ganzes oder teilweises Organimplantat nach Anspruch 1 zum Einsatz bei der Behandlung einer mit einer pathologischen Organ- oder Gewebephysiologie oder - morphologie verbundenen Bauchspeicheldrüsenstörung, wobei das pathologische Physiologie oder Morphologie aufweisende Organ bzw. Gewebe beim Transplantieren des Implantats entfernt wird.

14. Ganzes oder teilweises Organimplantat nach Anspruch 1 zum Einsatz bei der Behandlung einer mit einer pathologischen Organ- oder Gewebephysiologie oder - morphologie verbundenen Bauchspeicheldrüsenstörung, wobei es sich beim ganzen oder teilweisen Organimplantat um ein Bauchspeicheldrüsen-Inselimplantat handelt.

## Revendications

1. Greffe d'organe pancréatique porcin entier ou partiel au stade gestationnel destinée à être utilisée dans le traitement d'un trouble pancréatique associé à une physiologie ou morphologie pathologique d'un organe ou d'un tissu, la greffe d'organe pancréatique entier ou partiel sélectionnée n'exprimant ou ne présentant pas au moins une molécule capable de stimuler ou d'améliorer une réponse immunitaire chez un sujet, où ladite au moins une molécule est un corécepteur de lymphocytes ou un ligand d'un corécepteur de lymphocytes, et où ladite greffe d'organe porcin entier ou partiel est sélectionnée à un stade de différenciation spécifique correspondant à 20 à 42 jours de gestation.

2. Greffe d'organe pancréatique entier ou partiel selon la revendication 1 destinée à être utilisée dans le traitement d'un trouble pancréatique associé à une physiologie ou morphologie pathologique d'un organe ou d'un tissu selon la revendication 1, où ledit sujet est un humain.

3. Greffe d'organe pancréatique entier ou partiel selon la revendication 1 destinée à être utilisée dans le traitement d'un trouble pancréatique associé à une physiologie ou morphologie pathologique d'un organe ou d'un tissu selon la revendication 1, qui comprend en outre l'utilisation d'un médicament immunosuppresseur, ce qui favorise ainsi la prise de greffe de ladite greffe d'organe entier ou partiel chez ledit sujet.

4. Greffe d'organe pancréatique entier ou partiel selon la revendication 1 destinée à être utilisée dans le traitement d'un trouble pancréatique associé à une physiologie ou morphologie pathologique d'un organe ou d'un tissu selon la revendication 3, où ledit médicament immunosuppresseur est capable de bloquer la liaison d'un corécepteur de lymphocytes à un ligand dudit corécepteur de lymphocytes.

5. Greffe d'organe pancréatique entier ou partiel selon la revendication 1 destinée à être utilisée dans le traitement d'un trouble pancréatique associé à une physiologie ou morphologie pathologique d'un organe ou d'un tissu selon la revendication 4, où ledit médicament immunosuppresseur est une Ig anti-CTLA4.

6. Greffe d'organe pancréatique entier ou partiel selon la revendication 1 destinée à être utilisée dans le traitement d'un trouble pancréatique associé à une physiologie ou morphologie pathologique d'un organe ou d'un tissu selon la revendication 1, où ledit corécepteur de lymphocytes ou ligand d'un corécepteur de lymphocytes est sélectionné dans le groupe consistant en B7-1, CD40, et CD40L.

7. Greffe d'organe pancréatique entier ou partiel selon la revendication 1 destinée à être utilisée dans le traitement d'un trouble pancréatique associé à une physiologie ou morphologie pathologique d'un organe ou d'un tissu selon la revendication 1, où ladite sélection de ladite greffe d'organe entier ou partiel est réalisée via une analyse de RT-PCR dudit organe entier ou partiel.

8. Greffe d'organe pancréatique entier ou partiel selon la revendication 1 destinée à être utilisée dans le traitement d'un trouble pancréatique associé à une physiologie ou morphologie pathologique d'un organe ou d'un tissu selon la revendication 1, où ledit trouble pancréatique est le diabète.

9. Greffe d'organe pancréatique entier ou partiel selon la revendication 1 destinée à être utilisée dans le traitement d'un trouble pancréatique associé à une physiologie ou morphologie pathologique d'un organe ou d'un tissu selon la revendication 1, où ladite greffe d'organe entier ou partiel est une greffe d'îlots pancréatiques.

10. Greffe d'organe pancréatique entier ou partiel selon la revendication 1 destinée à être utilisée dans le traitement d'un trouble pancréatique associé à une physiologie ou morphologie pathologique d'un organe ou d'un tissu selon la revendication 1, où ladite greffe d'organe porcin entier ou partiel est sélectionnée à un stade de différenciation spécifique correspondant à 42 jours de gestation.

11. Greffe d'organe pancréatique entier ou partiel selon la revendication 1 destinée à être utilisée dans le traitement d'un trouble pancréatique associé à une physiologie ou morphologie pathologique d'un organe ou d'un tissu selon la revendication 1, où ladite greffe est implantée dans le tissu sous-cutané.

12. Greffe d'organe pancréatique entier ou partiel selon la revendication 1 destinée à être utilisée dans le traitement d'un trouble pancréatique associé à une physiologie ou morphologie pathologique d'un organe ou d'un tissu selon la revendication 1, où ladite greffe est implantée dans un emplacement anatomique dudit sujet sélectionné dans le groupe consistant en la veine porte, le foie, le pancréas, la capsule rénale, le tissu adipeux testiculaire, le tissu sous-cutané et l'espace intra-abdominal.

13. Greffe d'organe pancréatique entier ou partiel selon la revendication 1 destinée à être utilisée dans le traitement d'un trouble pancréatique associé à une physiologie ou morphologie pathologique d'un organe ou d'un tissu selon la revendication 1, où pendant la transplantation de ladite greffe, l'organe ou le tissu présentant une physiologie ou morphologie pathologique est retiré.

14. Greffe d'organe pancréatique entier ou partiel selon la revendication 1 destinée à être utilisée dans le traitement d'un trouble pancréatique associé à une physiologie ou morphologie pathologique d'un organe ou d'un tissu selon la revendication 1, où ladite greffe d'organe pancréatique entier ou partiel est une greffe d'îlots pancréatiques.


### SEQUENCE LISTING

<110> Reisner, Yair Dekel, Benjamin
<120> METHODS OF TREATING DISEASE BY TRANSPLANTATION OF DEVELOPING ALLOGENEIC OR
   XENOGENEIC ORGANS OR TISSUES
<130> 27542
<160> 12
<170> PatentIn version 3.1
<210> 1
   <211> 20
   <212> DNA
   <213> Artificial sequence
<220>
   <223> Single strand DNA primer
<400> 1
   gaccaaggaa gtaaagtggc 20
<210> 2
   <211> 24
   <212> DNA
   <213> Artificial sequence
<220>
   <223> Single strand DNA primer
<400> 2
   aggagaggtg aggctctgga aaac 24
<210> 3
   <211> 23
   <212> DNA
   <213> Artificial sequence
<220>
   <223> Single strand DNA primer
<400> 3
   cactatggga ctgagtaaca ttc 23
<210> 4
   <211> 23
   <212> DNA
   <213> Artificial sequence
<220>
   <223> Single strand DNA primer
<400> 4
   gcactgacag ttcagaattc atc 23
<210> 5
   <211> 22
   <212> DNA
   <213> Artificial sequence
<220>
   <223> Single strand DNA primer
<400> 5
   ctctgcagtg cgtcctctgg gg 22
<210> 6
   <211> 24
   <212> DNA
   <213> Artificial sequence
<220>
   <223> Single strand DNA primer
<400> 6
   gatggtatca gaaacccctg tagc 24
<210> 7
   <211> 24
   <212> DNA
   <213> Artificial sequence
<220>
   <223> single strand DNA primer
<400> 7
   tatcacccag atgattgggt cagc 24
<210> 8
   <211> 24
   <212> DNA
   <213> Artificial sequence
<220>
   <223> Single strand DNA primer
<400> 8
   ccagggttac caacttgttg ctca 24
<210> 9
   <211> 22
   <212> DNA
   <213> Artificial sequence
<220>
   <223> Single strand DNA primer
<400> 9
   atgaaggtct ccgcggcagc cc 22
<210> 10
   <211> 22
   <212> DNA
   <213> Artificial sequence
<220>
   <223> Single strand DNA primer
<400> 10
   ctagctcatc tccaaagagt tg 22
<210> 11
   <211> 22
   <212> DNA
   <213> Artificial sequence
<220>
   <223> Single strand DNA primer
<400> 11
   accatcaagc tctgcgtgac tg 22
<210> 12
   <211> 23
   <212> DNA
   <213> Artificial sequence
<220>
   <223> Single strand DNA primer
<400> 12
   gcaggtcagt tcagttccag gtc 23